# EUROPEAN PATENT APPLICATION

(11) **EP 4 818 204 A1**
(43) Date of publication of application: **07.10.2026**
(21) Application number: 24896721.8
(22) Date of filing: 29.11.2024
(51) Int. Cl.: C07K 16/22, C12N 15/13, A61K 39/395, A61P 35/00, A61P 9/04

(54) **ANTI-GDF15 ANTIBODY, AND METHOD AND USE THEREFOR**

(30) Priority: 30.11.2023 WO PCT/CN2023/135524; 05.07.2024 WO PCT/CN2024/103906
(71) Applicant: Nanjing Leads Biolabs Co., Ltd., Jiangbei New Area Nanjing Jiangsu 210031 (CN)
(72) Inventor: WANG, Baohui, Nanjing, Jiangsu 210019 (CN); DING, Mi, Nanjing, Jiangsu 210019 (CN); HUANG, Jing, Nanjing, Jiangsu 210019 (CN); HUANG, Xiao, Nanjing, Jiangsu 210019 (CN); LING, Hong, Nanjing, Jiangsu 210019 (CN)
(74) Representative: Bianchetti & Minoja with Trevisan & Cuonzo IPS SRL
(86) International application number: PCT/CN2024/135678
(87) International publication number: WO 2025/113639

(57) **Abstract**

A GDF15 binding molecule, in particular an antibody specifically recognizing GDF15 and a fragment thereof. A nucleic acid or host cell containing the antibody or fragment thereof, a drug containing the antibody or fragment thereof, and a therapeutic and diagnostic method or use applying the antibody and fragment.

## Description

### TECHNICAL FIELD

The present disclosure belongs to the field of antibodies, and in particular relates to a GDF15-binding molecule, particularly an antibody and a fragment thereof that specifically recognize GDF15. In addition, the present disclosure further relates to nucleic acids or host cells comprising such antibodies or fragments thereof, medicaments comprising such antibodies or fragments thereof, and therapeutic and diagnostic methods or uses employing these antibodies and fragments.

### BACKGROUND

Growth differentiation factor 15 (GDF15) is a member of the transforming growth factor β (TGFβ) superfamily. GDF15 is secreted as a 40 kDa propeptide, which releases a 25 kDa dimeric protein as the active substance upon cleavage. GDF15 is a stress-responsive cytokine whose expression can be significantly increased under various stress conditions, such as inflammation, hypoxia, tissue injury, myocardial ischemia, and various malignant tumors. It has been reported that circulating GDF15 levels are elevated in patients with various cancers, including prostate cancer, pancreatic cancer, endometrial carcinoma, colorectal cancer, and the like. Within the tumor microenvironment, GDF15 can be expressed by tumor cells, cancer-associated fibroblasts, and tumor-associated macrophages. In addition, chemotherapy can also induce a significant increase in GDF15 levels in patients (Suriben R et al., Nature Medicine, 2020, 26(8): 1264-1270.).

Cachexia is a condition that causes the loss of lean body mass and occurs during the progression of many diseases. Cachexia is estimated to cause 2 million deaths worldwide each year and is one of the leading causes of human mortality (Ferrer M et al., Cell, 2023, 186(9): 1824-1845.). Several recent studies (Mullican S E et al., Nature Medicine, 2017, 23(10): 1150-1157; Yang L et al., Nature Medicine, 2017, 23(10): 1158-1166; Emmerson P J et al., Nature Medicine, 2017, 23(10): 1215-1219) have found that GDF15 can bind to the glial cell line derived neurotrophic factor (GDNF) -family receptor α-like (GFRAL) protein, a receptor predominantly expressed in the hindbrain, and then form a heterohexamer with a rearranged during transfection (RET) molecule to activate the GFRAL-RET signaling pathway; this transmits anorexic neural signals, thereby reducing body weight, affecting fat and muscle degradation, and inducing cachexia. GDF15/GFRAL antibodies can reverse this response and are potential targets for treating cachexia.

The GDF15 antibody Ponsegromab (CN112912395A) developed by Pfizer and the GFRAL antibody NGM-120 (WO2023/039359A1) developed by NGM have already entered phase 2 clinical trials for the amelioration of tumor cachexia and have exhibited potential to increase the patient's body weight.

However, the specificity, potency, and druggability of current GDF15 antibodies still need to be improved.

### SUMMARY

In one aspect, the present disclosure provides a GDF15 antibody having high binding affinity for GDF15.

In some embodiments, the anti-GDF15 antibody of the present disclosure or an antigen-binding fragment thereof binds to GDF15 (e.g., human GDF15, monkey GDF15 such as cynomolgus monkey GDF15, or murine GDF15 such as mouse GDF15) with higher affinity. In some embodiments, the anti-GDF15 antibody or the antigen-binding fragment thereof of the present disclosure is capable of binding to both human GDF15 and monkey GDF15 such as cynomolgus monkey GDF15, and optionally also binds to murine GDF15 such as mouse GDF15. In some embodiments, the affinity of the antibody is determined by biolayer interferometry or surface plasmon resonance. In some embodiments, the anti-GDF15 antibody of the present disclosure binds to human GDF15 or monkey GDF15 such as cynomolgus monkey GDF15 with an equilibrium dissociation constant (KD) of less than about 10 nM, 5 nM, 4 nM, 3 nM, 2 nM, 1 nM, 0.5 nM, 0.4 nM, 0.3 nM, 0.2 nM, 0.1 nM, 0.09 nM, 0.08 nM, 0.07 nM, 0.06 nM, 0.05 nM, 0.04 nM, 0.03 nM, 0.02 nM, 0.01 nM, or 1 pM, or between any of the above values. In some embodiments, the anti-GDF15 antibody of the present disclosure binds to murine GDF15 such as mouse GDF15 with an equilibrium dissociation constant (KD) of less than about 20 nM, 15 nM, 14 nM, 13 nM, 12 nM, 11 nM, 10 nM, 5 nM, 4 nM, 3 nM, 2 nM, 1 nM, or 0.5 nM, or between any of the above values, for example, between 0.5 nM and 15 nM, e.g., between 1 nM and 15 nM.

In some embodiments, the anti-GDF15 antibody or the antigen-binding fragment thereof of the present disclosure binds to cell-expressed human or cynomolgus monkey or mouse GDF15. In some embodiments, the affinity of the anti-GDF15 antibody for cell-expressed human or cynomolgus monkey or mouse GDF15 is determined by flow cytometry.

In some embodiments, the anti-GDF15 antibody or the antigen-binding fragment thereof of the present disclosure does not cross-react with human TGFβ family proteins, for example, it does not bind to human GDF1, GDF2, GDF3, AMH, BMP4, GDF11, GDF8, GDNF, TGFβ1, GFRα1, Activin A, Activin B, and/or BMP2 proteins.

In some embodiments, the anti-GDF15 antibody or the antigen-binding fragment thereof of the present disclosure inhibits free GDF15 in blood, such as serum, e.g., with superior performance compared to known controls, such as Ponsegromab.

In some embodiments, the anti-GDF15 antibody or the antigen-binding fragment thereof of the present disclosure blocks the activation of a stress signaling pathway by GDF15, for example, blocks the activation of a serum response element (SRE), e.g., in a dose-dependent trend, e.g., exhibiting stronger efficacy compared to controls, such as Ponsegromab.

In some embodiments, the anti-GDF15 antibody or the antigen-binding fragment thereof of the present disclosure inhibits downstream ERK phosphorylation levels of GDF15/GFRAL/RET, e.g., with superior performance compared to known controls, such as Ponsegromab.

In some embodiments, the anti-GDF15 antibody or the antigen-binding fragment thereof of the present disclosure reverses tumor-induced cachexia (e.g., tumor cachexia), e.g., with superior performance compared to known controls, such as Ponsegromab, wherein the tumor is, for example, a solid tumor, such as fibrosarcoma.

In some embodiments, the anti-GDF15 antibody or the antigen-binding fragment thereof of the present disclosure reverses cachexia induced by a chemotherapeutic agent such as cisplatin, e.g., with superior performance compared to known controls, such as Ponsegromab.

In some embodiments, the anti-GDF15 antibody or the antigen-binding fragment thereof of the present disclosure exhibits a good drug exposure level, e.g., superior to those of known controls, such as Ponsegromab.

In some embodiments, the anti-GDF15 antibody or the antigen-binding fragment thereof of the present disclosure has pharmacokinetic characteristics suitable for drug development.

In some embodiments, the anti-GDF15 antibody or the antigen-binding fragment thereof of the present disclosure comprises a heavy chain variable region and a light chain variable region.

In some embodiments, the anti-GDF15 antibody of the present disclosure is a full-length antibody or an antigen-binding fragment thereof.

In some embodiments, the anti-GDF15 antibody of the present disclosure is a VHH antibody or a heavy-chain antibody comprising same.

The present disclosure further relates to a nucleic acid molecule encoding the antibody, and a therapeutic method and use employing the antibody.

### BRIEF DESCRIPTION OF THE DRAWINGS

FIGs. 1A and 1B show the reporter gene activity of humanized anti-GDF15 antibodies.
FIG. 1C shows the reporter gene activity of a humanized anti-GDF15 antibody with YTE mutations in the Fc region.
FIG. 2A shows a PAGE image for the inhibition of ERK1/2 phosphorylation by the humanized anti-GDF15 antibodies as determined by Western blot.
FIG. 2B shows the grayscale value analysis results for the inhibition of ERK1/2 phosphorylation by the humanized anti-GDF15 antibodies as determined by Western blot.
FIG. 3A shows a PAGE image for the inhibition of ERK1/2 phosphorylation by the VHH humanized anti-GDF15 antibody as determined by Western blot.
FIG. 3B shows the grayscale value analysis results for the inhibition of ERK1/2 phosphorylation by the VHH humanized anti-GDF15 antibody as determined by Western blot.
FIG. 4 shows the binding activity of a control antibody, humanized anti-GDF15 antibodies, and an IgG control to GDF2.
FIG. 5 shows the binding activity of a control antibody, humanized anti-GDF15 antibodies, and an IgG control to GDNF.
FIGs. 6A and 6B show the changes in net body weight of mice on day 6 (10 mg/kg) and day 7 (3 mg/kg and 1 mg/kg) after administration.
FIG. 6C shows the average daily food intake after administration.
FIG. 6D shows the ovarian fat weight of mice on day 7 after administration.
FIG. 7 shows the free drug levels in mice 72 h after a single administration.
FIG. 8 shows the changes in net body weight of mice after administration in a cisplatin model.
FIG. 9 shows the serum concentration levels at the endpoint in a cisplatin efficacy model.
FIG. 10 shows the serum concentration-time curves of the anti-GDF15 antibodies in the serum of humanized FcRn mice.

### DETAILED DESCRIPTION

Before the present disclosure is described in detail, it should be understood that the present disclosure is not limited to the particular methods or experimental conditions described in this description, as the methods and conditions may vary. Furthermore, the terms used herein are for the purpose of describing particular embodiments only and are not intended to be limiting.

Before the present disclosure is described in detail, it should be understood that the present disclosure is not limited to the particular methods or experimental conditions described in this description, as the methods and conditions may vary. Furthermore, the terms used herein are for the purpose of describing particular embodiments only and are not intended to be limiting.

### I. Definitions

In order to illustrate this description, the following definitions will be used, and wherever appropriate, terms used in the singular form may also include the plural form, and vice versa. Unless otherwise defined, all technical and scientific terms used herein have the same meaning as commonly understood by those of ordinary skill in the art to which the present disclosure belongs.

The term "about" used in combination with a numerical value is intended to encompass the numerical values within a range from a lower limit less than the specified numerical value by 5% to an upper limit greater than the specified numerical value by 5%.

As used herein, the term "and/or" refers to any one of the options or two or more or all of the options.

As used herein, the term "comprise" or "include" is intended to refer to the inclusion of the elements, integers, or steps, without the exclusion of any other elements, integers, or steps. The term "comprise" or "include" used herein, unless otherwise indicated, also encompasses the situation where the entirety consists of the described elements, integers, or steps. For example, when referring to an antibody variable region "comprising" a specific sequence, it is also intended to encompass an antibody variable region consisting of the specific sequence.

Growth differentiation factor 15 (GDF15) is a distinct member of the TGFβ superfamily, and is also referred to as macrophage-inhibitory cytokine 1 (M1C1) (Bootcov MR, 1997, Proc Natl Acad Sci, 94: 11514-9), placental bone morphogenetic factor (PLAB) (Hromas R, 1997, Biochim Biophys Acta., 1354: 40-4), placental transforming growth factor β (PTGFB) (Lawton LN, 1997, Gene., 203: 17-26), prostate-derived factor (PDF) (Paralkar VM, 1998, J Biol Chem., 273: 13760-7), and non-steroidal anti-inflammatory drug-activated gene (NAG-1) (Baek SJ, 2001, J Biol Chem., 276: 33384-92). Mature GDF15 peptides have low homology to other family members (Katoh M, 2006, Int J Mol Med., 17: 951-5). GDF15 is synthesized as a large precursor protein, which is cleaved at a dibasic cleavage site to release a carboxyl-terminal mature peptide. The full-length human precursor contains 308 amino acids and is cleaved at the RGRRRAR (SEQ ID NO: 185) cleavage site to generate the mature GDF peptide. Naturally occurring GDF15 is a 25 KD homodimer of mature peptides covalently linked by an interchain disulfide bond. In some embodiments, GDF15 is, for example, a human GDF15 or cynomolgus monkey GDF15 or mouse GDF15 protein, e.g., the human GDF15 protein under Accession No. Q99988-1, or the cynomolgus monkey GDF15 protein under Accession No. G7PWZ3, or the mouse GDF15 protein under Accession No. Q9Z0J7. In one embodiment, the human GDF15 protein of the present disclosure comprises or consists of the amino acid sequence set forth in SEQ ID NO: 40 (or the amino acid sequence without a His tag), or an amino acid sequence having at least 85%, 90%, 91%, 92%, 93%, 94%, 95%, 96%, 97%, 98%, or 99% identity thereto. In one embodiment, the cynomolgus monkey GDF15 protein of the present disclosure comprises or consists of the amino acid sequence set forth in SEQ ID NO: 41 (or the amino acid sequence without a His tag), or an amino acid sequence having at least 85%, 90%, 91%, 92%, 93%, 94%, 95%, 96%, 97%, 98%, or 99% identity thereto. In one embodiment, the mouse GDF15 protein of the present disclosure comprises or consists of the amino acid sequence set forth in SEQ ID NO: 42 (or the amino acid sequence without a His tag), or an amino acid sequence having at least 85%, 90%, 91%, 92%, 93%, 94%, 95%, 96%, 97%, 98%, or 99% identity thereto.

The term "anti-GDF15 antibody", "anti-GDF15", "GDF15 antibody", or "GDF15-binding antibody" used herein refers to an antibody capable of binding to a GDF15 protein or a fragment thereof with sufficient affinity. In one embodiment, the anti-GDF15 antibody binds to a non-GDF15 protein to an extent of about 10%, about 20%, about 30%, about 40%, about 50%, about 60%, about 70%, about 80%, about 90%, or more, weaker than the antibody binding to GDF15. In some embodiments, the binding is measured by, e.g., radioimmunoassay (RIA), biolayer interferometry (BLI), bioluminescence interferometry, MSD assay, surface plasmon resonance (SPR), or flow cytometry.

In one embodiment, the binding molecule specifically binding to GDF15, e.g., the anti-GDF15 antibody or the antigen-binding fragment thereof, of the present disclosure has high-affinity binding activity to cells expressing human GDF15 or cynomolgus monkey GDF15. In one embodiment, the binding molecule specifically binding to GDF15, e.g., the anti-GDF15 antibody or the antigen-binding fragment thereof, of the present disclosure has cross-reactivity with human and monkey (e.g., cynomolgus monkey) GDF15 or an extracellular domain thereof; i.e., it is capable of binding to both human and monkey (e.g., cynomolgus monkey) GDF15. In one embodiment, the binding molecule specifically binding to GDF15, e.g., the anti-GDF15 antibody or the antigen-binding fragment thereof, of the present disclosure is also capable of binding to mouse GDF15.

The terms "full antibody", "full-length antibody", "complete antibody", and "intact antibody" are used interchangeably herein to refer to a naturally occurring glycoprotein comprising at least two heavy (H) chains and two light (L) chains interconnected by disulfide bonds. Each heavy chain consists of a heavy chain variable region (abbreviated herein as VH) and a heavy chain constant region. The heavy chain constant region consists of 3 domains: CH1, CH2, and CH3. Each light chain consists of a light chain variable region (abbreviated herein as VL) and a light chain constant region. The light chain constant region consists of one domain, CL. The VH and VL regions can be further divided into hypervariable regions (complementarity determining regions (CDRs)), with relatively conservative regions (framework regions (FRs)) inserted therebetween. Each VH or VL consists of three CDRs and four FRs, arranged from amino-terminus to carboxyl-terminus in the following order: FR1, CDR1, FR2, CDR2, FR3, CDR3, and FR4. The constant regions are not directly involved in the binding of antibodies to antigens, but exhibit a variety of effector functions.

In some embodiments, the antibody heavy chain constant region HC of the present disclosure is a heavy chain constant region of IgG1, IgG2, IgG3, or IgG4, preferably a heavy chain constant region of IgG1.

In some embodiments, the antibody light chain constant region LC of the present disclosure is a lambda or kappa light chain constant region.

The term "antibody fragment" includes a portion of an intact antibody. In a preferred embodiment, the antibody fragment is an antigen-binding fragment.

The term "antigen-binding fragment" is a portion or segment of an intact antibody or a complete antibody that has fewer amino acid residues than the intact antibody or the complete antibody, which can bind to an antigen or compete with the intact antibody (i.e., the intact antibody from which the antigen-binding fragment is derived) for binding to the antigen. The antigen-binding fragment may be prepared by recombinant DNA techniques, or by enzymatic or chemical cleavage of an intact antibody. The antigen-binding fragment includes, but is not limited to, a Fab, a Fab', a F(ab')₂, an Fv, a single-chain Fv, a diabody, and a single-domain antibody (sdAb). The Fab fragment is a monovalent fragment consisting of VL, VH, CL, and CH1 domains and can be obtained, for example, by papain digestion of a complete antibody. In addition, the F(ab')₂, a dimer of the Fab', is a bivalent antibody fragment produced by pepsin digestion of a portion below disulfide bonds in the hinge region of a complete antibody. The F(ab')₂ can be reduced by disrupting the disulfide bonds in the hinge region under a neutral condition, and the F(ab')₂ dimer is thus converted into Fab' monomers. The Fab' monomer is essentially a Fab fragment with a hinge region. The Fv fragment consists of the VL and VH domains of a single arm of an antibody. Furthermore, although the two domains VL and VH of the Fv fragment are encoded by separate genes, they can be linked, using a recombinant method, by a synthetic linker peptide that enables these two domains to be produced as a single protein chain, in which the VL and VH regions are paired to form a single-chain Fv (scFv). The antibody fragment can be obtained by a chemical method, a recombinant DNA method, or a protease digestion method.

The "Fab fragment" and "Fab" are used interchangeably herein to refer to an immunoglobulin fragment consisting of two polypeptide chains and comprising an immunoglobulin heavy chain variable domain VH, a heavy chain constant domain CH1, a light chain variable domain VL, and a light chain constant domain CL, wherein one polypeptide chain comprises, from N-terminus to C-terminus, a VH and one constant region selected from CH1 and CL, and the other polypeptide chain comprises, from N-terminus to C-terminus, a VL and the other constant region selected from CL and CH1, wherein the VH and VL domains are paired to form an antigen-binding site. A Fab chain comprising a heavy chain constant region CH1 is also referred to as a "Fab heavy chain"; correspondingly, a Fab chain comprising a light chain constant region CL is also referred to as a "Fab light chain".

The term "antigen" refers to a molecule that induces an immune response. Such an immune response may involve antibody production or activation of specific immune cells, or both. Those skilled in the art will appreciate that any macromolecules, including substantially all proteins or peptides, can be used as antigens. In addition, an antigen may be derived from recombinant or genomic DNA.

The term "variable region" or "variable domain" refers to a domain of a heavy or light chain of an antibody involved in the binding of the antibody to an antigen. The variable regions of the heavy and light chains of a native antibody generally have similar structures, wherein each domain comprises four conserved framework regions (FRs) and three complementarity determining regions.

The "complementarity determining region" or "CDR region" or "CDR" is a region in an antibody variable domain that is highly variable in sequence and forms a structurally defined loop ("hypervariable loop") and/or comprises antigen-contacting residues ("antigen contact sites"). The CDRs are primarily responsible for binding to antigenic epitopes. The CDRs of the heavy and light chains are generally referred to as CDR1, CDR2, and CDR3, and are numbered sequentially from the N-terminus. The CDRs located in the heavy chain variable domain of the antibody are referred to as HCDR1, HCDR2, and HCDR3, while the CDRs located in the light chain variable domain of the antibody are referred to as LCDR1, LCDR2, and LCDR3. In a given amino acid sequence of a light chain variable region or a heavy chain variable region, the exact amino acid sequence boundary of each CDR may be determined using any one or a combination of many well-known antibody CDR assignment schemes including, e.g., Chothia based on the three-dimensional structure of antibodies and the topology of the CDR loops (Chothia et al., (1989) Nature, 342: 877-883; Al-Lazikani et al., Standard conformations for the canonical structures of immunoglobulins, Journal of Molecular Biology, 273, 927-948 (1997)), Kabat based on antibody sequence variability (Kabat et al., Sequences of Proteins of Immunological Interest, 4th ed., U.S. Department of Health and Human Services, National Institutes of Health (1987)), AbM (University of Bath), Contact (University College London), international ImMunoGeneTics database (IMGT) (imgt.cines.fr/ on the World Wide Web), and North CDR definition based on the affinity propagation clustering using a large number of crystal structures.

The following are the regional ranges of the CDRs defined using the Kabat, AbM, Chothia, Contact, and IMGT schemes.

| CDR | Kabat scheme | AbM scheme | Chothia scheme | Contact scheme | IMGT scheme |
|---|---|---|---|---|---|
| LCDR1 (Kabat and Chothia numbering systems) | L24-L34 | L24-L34 | L26-L32 | L30-L36 | L27-L32 |
| LCDR2 (Kabat and Chothia numbering systems) | L50-L56 | L50-L56 | L50-L52 | L46-L55 | L50-L51 |
| LCDR3 (Kabat and Chothia numbering systems) | L89-L97 | L89-L97 | L91-L96 | L89-L96 | L89-L97 |
| HCDR1 (Kabat numbering system) | H31-H35B | H26-H35B | H26-H32...34 | H30-H35B | H26-H35B |
| HCDR1 (Chothia numbering system) | H31-H35 | H26-H35 | H26-H32 | H30-H35 | H26-H33 |
| HCDR2 (Kabat and Chothia numbering systems) | H50-H65 | H50-H58 | H52-H56 | H47-H58 | H51-H56 |
| HCDR3 (Kabat and Chothia numbering systems) | H95-H102 | H95-H102 | H96-H101 | H93-H101 | H93-H102 |

Unless otherwise stated, the term "CDR" or "CDR sequence" used in the present disclosure encompasses CDR sequences determined by any one of the schemes described above. CDRs may also be determined based on having the same Kabat numbering positions as a reference CDR sequence (e.g., any one of the exemplary CDRs of the present disclosure). Unless otherwise stated, in the present disclosure, residue positions of an antibody variable region (including heavy chain variable region residues and light chain variable region residues) are determined according to the Kabat numbering system (Kabat et al., Sequences of Proteins of Immunological Interest, 5th ed., Public Health Service, National Institutes of Health, Bethesda, Md. (1991)).

In one embodiment, the HCDRs and LCDRs in the antibody of the present disclosure are determined according to the Kabat scheme.

In some embodiments, the HCDRs in the VHH antibody or the heavy-chain antibody comprising same of the present disclosure are determined according to IMGT.

The term "Fc domain", "Fc region", or "Fc fragment" is used herein to define a C-terminal region of an immunoglobulin heavy chain, which comprises at least a portion of the constant region. The term includes Fc regions of native sequences and variant Fc regions. A native immunoglobulin "Fc domain" comprises two or three constant domains, i.e., a CH2 domain, a CH3 domain, and optionally a CH4 domain. For example, in native antibodies, an immunoglobulin Fc domain comprises the second and the third constant domains (CH2 domain and CH3 domain) derived from two heavy chains of IgG, IgA, and IgD antibodies; or comprises the second, the third, and the fourth constant domains (CH2 domain, CH3 domain, and CH4 domain) derived from two heavy chains of IgM and IgE antibodies. Unless otherwise stated herein, amino acid residues in the Fc region or the heavy chain constant region are numbered according to the EU numbering system (also referred to as the EU index) as described in, for example, Edelman, G. M. et al., Proc. Natl. Acad. USA, 63, 78-85 (1969) (https://pubmed.ncbi.nlm.nih.gov/5257969/), see also http://www.imgt.org/IMGTScientificChart/Numbering/Hu_IGHGnber.html. Herein, the term "Fc domain", "Fc region", or "Fc fragment" does not comprise a heavy chain variable region VH and a light chain variable region VL as well as a heavy chain constant region CH1 (without hinge region) and a light chain constant region CL of an immunoglobulin, but in some cases, it may comprise a hinge region at the N-terminus of the heavy chain constant region, such as EPKSS or EPKSC. In some embodiments, the heavy chain constant region Fc suitable for use in the present disclosure is from an antibody heavy chain constant region, e.g., a constant region of human IgG1, IgG2, IgG3, or IgG4, preferably from a constant region of IgG1. In some embodiments, the Fc region comprises or consists of the amino acid sequence set forth in SEQ ID NO: 46 or an amino acid sequence having at least 85%, 90%, 91%, 92%, 93%, 94%, 95%, 96%, 97%, 98%, or 99% identity thereto.

Examples of "effector functions" of immunoglobulins include: C1q binding and complement-dependent cytotoxicity (CDC), Fc receptor binding, antibody-dependent cell-mediated cytotoxicity (ADCC), antibody-dependent cellular phagocytosis (ADCP), cytokine secretion, immune complex-mediated antigen uptake in antigen-presenting cells, down-regulation of cell surface receptors (e.g., B-cell receptors), and B-cell activation.

The term "chimeric antibody" refers to an antibody molecule in which: (a) a constant region or a portion thereof is modified, substituted, or exchanged such that an antigen-binding site is linked to a constant region of a different or modified class, effector function, and/or species, or an entirely different molecule (e.g., an enzyme, a toxin, a hormone, a growth factor, or a drug) conferring new properties to the chimeric antibody, etc.; or (b) a variable region or a portion thereof is modified, substituted, or exchanged with a variable region having a different or modified antigen specificity. For example, a mouse antibody can be modified by substituting its constant region with a constant region from a human immunoglobulin. Due to the substitution with a human constant region, the chimeric antibody may retain its specificity for recognizing antigens, while having reduced immunogenicity in humans as compared to the original mouse antibody.

The "humanized antibody" is an antibody that retains the antigen-specific reactivity of a non-human antibody (such as a murine antibody) and has lower immunogenicity when administered to humans as a therapeutic agent. This can be achieved, for example, by retaining non-human antigen-binding sites and substituting the remainder of the antibodies with their human counterparts (i.e., the portions of the variable regions not involved in binding are substituted with the corresponding parts of human antibodies).

As used herein, the term "anti", "binding", or "specifically binding to" means that the binding action is selective for the target or antigen and may be distinguished from undesired or non-specific interactions. The ability of a binding site to bind to a particular target or antigen may be determined by flow cytometry, enzyme-linked immunosorbent assay (ELISA), or conventional binding assays known in the art, such as radioimmunoassay (RIA), biolayer interferometry, MSD assay, or surface plasmon resonance (SPR).

The "affinity" or "binding affinity" refers to the inherent binding affinity that reflects the interaction between members of a binding pair. The affinity of molecule X for its partner Y may be generally represented by the dissociation constant (K_{D}), which is a ratio of the dissociation rate constant (K_{dis}) to the association rate constant (Kₒₙ). The affinity can be measured by common methods known in the art. One specific method for measuring affinity is the ForteBio kinetic binding assay.

The "percent identity (%)" of an amino acid sequence refers to the percentage of amino acid residues in a candidate sequence that are identical to those in a specific amino acid sequence shown in this description when aligning the candidate sequence with the specific amino acid sequence shown in this description, with gaps introduced if necessary to achieve maximum percent sequence identity and without considering any conservative substitutions as part of sequence identity. In some embodiments, the present disclosure considers variants of the antibody molecule of the present disclosure that have a considerable degree of identity to the antibody molecule and the sequence thereof specifically disclosed herein. For example, the identity is at least 80%, 85%, 90%, 95%, 97%, 98%, 99%, or higher. The variants may comprise conservative modifications, or be conservatively modified variants.

For polypeptide sequences, the "conservative modifications" include substitutions of, deletions of, or additions to a polypeptide sequence but do not substantially change the desired functional activity of the polypeptide sequence. For example, conservative substitutions often result in the substitution of an amino acid with a chemically similar amino acid. Conservative substitution tables providing functionally similar amino acids are well known in the art. 8 groups comprising amino acids that can be conservatively substituted with each other are listed as follows: 1) alanine (A) and glycine (G); 2) aspartic acid (D) and glutamic acid (E); 3) asparagine (N) and glutamine (Q); 4) arginine (R) and lysine (K); 5) isoleucine (I), leucine (L), methionine (M), and valine (V); 6) phenylalanine (F), tyrosine (Y), and tryptophan (W); 7) serine (S) and threonine (T); and 8) cysteine (C) and methionine (M). In some embodiments, the term "conservative sequence modification" is used to refer to an amino acid modification that does not significantly affect or change the binding characteristics for an antigen of interest of the antibody molecule or the binding protein molecule of the present disclosure comprising the amino acid sequence. For example, conservatively modified variants retain at least 80%, 85%, 90%, 95%, 98%, 99%, or higher, such as 100%-110% or higher, binding affinity for an antigen of interest relative to the parent antibody or binding protein.

The term "host cell" refers to a cell into which an exogenous polynucleotide has been introduced, including the progeny of such cells. The host cell includes "transformants" and "transformed cells", which include primary transformed cells and progeny derived therefrom. The host cell is any type of cell system that may be used to produce the antibody molecule of the present disclosure, including eukaryotic cells, e.g., mammalian cells, insect cells, and yeast cells, and prokaryotic cells, e.g., *Escherichia coli* cells. The host cell includes cultured cells, as well as cells within a transgenic animal, a transgenic plant, or a cultured plant tissue or animal tissue.

The term "vector" as used herein refers to a nucleic acid molecule capable of proliferating another nucleic acid to which it is linked. The term includes vectors that serve as self-replicating nucleic acid structures as well as vectors integrated into the genome of a host cell into which they have been introduced. The term "expression vector" refers to a vector comprising a recombinant polynucleotide, which comprises an expression control sequence operably linked to the nucleotide sequence to be expressed. Expression vectors contain sufficient cis-acting elements for expression, and other elements for expression may be provided by a host cell or in an *in vitro* expression system. Expression vectors include all those known in the art, including cosmids, plasmids (e.g., naked or contained in liposomes), and viruses (e.g., lentiviruses, retroviruses, adenoviruses, and adeno-associated viruses) into which recombinant polynucleotides are incorporated.

The terms "individual" and "subject" are used interchangeably and refer to a mammal. The mammal includes, but is not limited to, domesticated animals (e.g., cows, sheep, cats, dogs, and horses), primates (e.g., humans and non-human primates such as monkeys), rabbits, and rodents (e.g., mice and rats). In particular, the individual is a human.

The term "treatment" (or "treat" or "treating") refers to slowing, interrupting, arresting, alleviating, stopping, reducing, or reversing the onset of symptoms, complications, or biochemical indications of a disease, or relieving symptoms, or preventing or inhibiting the further progression of a disease, symptom, or disorder.

The term "prevention" (or "prevent" or "preventing") includes the inhibition of the development or progression of a disease or disorder, or symptoms of a specific disease or disorder. In some embodiments, a subject with a family history of cancer is a candidate for a preventive regimen. Generally, in the context of cancer, the term "prevention" refers to the administration of a drug prior to the occurrence of signs or symptoms of cancer, particularly in subjects at risk of cancer.

The term "therapeutic agent" described herein encompasses any substance that is effective in preventing or treating a tumor, e.g., cancer, including a chemotherapeutic agent, a cytokine, a cytotoxic agent, an additional antibody (e.g., an immune checkpoint molecule antibody), a small molecule drug, or an immunomodulatory agent (e.g., an immunosuppressant or an agonist).

The term "cytotoxic agent" used in the present disclosure refers to a substance that inhibits or prevents cell functions and/or causes cell death or cell destruction.

The "chemotherapeutic agent" includes chemical compounds useful in the treatment of cancers or immune system diseases.

The term "small molecule drug" refers to a low-molecular-weight organic compound capable of regulating biological processes. The "small molecule" is defined as a molecule with a molecular weight of less than 10 kD, usually less than 2 kD, and preferably less than 1 kD. The small molecule includes, but is not limited to, inorganic molecules, organic molecules, organic molecules containing inorganic components, molecules containing radioactive atoms, synthetic molecules, peptide mimetics, and antibody mimetics. As therapeutic agents, small molecules are more cell-permeable, less susceptible to degradation, and less likely to induce an immune response compared to macromolecules.

The term "immunomodulatory agent" used herein refers to a natural or synthetic active agent or drug that suppresses or modulates an immune response. The immune response may be a humoral response or a cellular response. The immunomodulatory agent includes an immunosuppressant or an agonist. In some embodiments, the immunomodulatory agent of the present disclosure includes an immune checkpoint inhibitor or an immune checkpoint agonist.

The term "effective amount" refers to an amount or dose of the antibody, fragment, composition, or combination of the present disclosure which generates expected effects in a patient in need of treatment or prevention after being administered to the patient in a single dose or multiple doses.

The "therapeutically effective amount" refers to an amount effective to achieve a desired therapeutic result at a necessary dose for a necessary period of time. The therapeutically effective amount is also such an amount that any toxic or adverse effect of the antibody, antibody fragment, composition, or combination is outweighed by the therapeutically beneficial effect. The "therapeutically effective amount" preferably inhibits a measurable parameter or improves a measurable parameter by at least about 40%, and even more preferably by at least about 50%, 55%, 60%, 65%, 70%, 75%, 80%, 85%, 90%, or even 100%, relative to untreated subjects.

The "prophylactically effective amount" refers to an amount effective to achieve a desired prophylactic result at a necessary dose for a necessary period of time. Generally, since a prophylactic dose is administered to a subject before or at an earlier stage of a disease, the prophylactically effective amount will be less than the therapeutically effective amount.

The term "tumor" refers to all neoplastic cell growth and proliferation, whether malignant or benign, and all pre-cancerous and cancerous cells and tissues. The terms "cancer", "carcinoma", and "tumor" are not mutually exclusive when referred to herein. The term "tumor" encompasses both solid tumors and hematological tumors. The term "anti-tumor effect" or "tumor inhibitory effect" refers to a biological effect that can be demonstrated by a variety of means, including but not limited to, for example, decrease in tumor volume, decrease in the number of tumor cells, decrease in tumor cell proliferation, or decrease in tumor cell viability.

The term "pharmaceutical supplementary material" refers to a diluent, an adjuvant (e.g., Freund's adjuvant (complete and incomplete)), an excipient, a carrier, or a stabilizer, etc., that is administered together with the active substance.

The term "pharmaceutical composition" refers to a composition that is present in a form allowing the biological activity of the active ingredient contained therein to be effective, and does not contain additional ingredients having unacceptable toxicity to a subject to which the composition is administered.

The term "pharmaceutical combination or combination product" refers to a non-fixed combination product or a fixed combination product, including but not limited to a kit. The term "non-fixed combination" means that the active ingredients (e.g., (i) the binding molecule of the present disclosure, and (ii) an additional therapeutic agent) are administered to a patient as separate entities either simultaneously without specific time limitation or sequentially at identical or different time intervals, wherein such administration provides two or more active agents at prophylactically or therapeutically effective levels in the patient. The term "fixed combination" means that two or more active agents are administered to a patient simultaneously in the form of a single entity. The dose and/or time intervals of two or more active agents are preferably selected such that the combined use of the ingredients can result in a therapeutic effect on the disease or disorder greater than that achieved by the use of any one of the ingredients alone. The ingredients may each take a separate formulation form, and their formulation forms may be the same or different.

The term "combination therapy" refers to the administration of two or more therapeutic agents or treatment modalities (e.g., radiotherapy or surgery) to treat the diseases as described herein. Such administration includes co-administration of these therapeutic agents in a substantially simultaneous manner, for example, in a single capsule with a fixed ratio of active ingredients. Alternatively, such administration includes co-administration of the active ingredients in a variety of or separate containers (e.g., tablets, capsules, powders, and liquids). The powder and/or liquid may be reconstituted or diluted to a desired dose before administration. In addition, such administration further includes using each type of therapeutic agent at approximately the same time or in a sequential manner at different times. In any case, the therapeutic regimen will provide the beneficial effect of the pharmaceutical combination in the treatment of the disorders or symptoms described herein.

The "subject/patient/individual sample" refers to a collection of cells or fluids obtained from a patient or a subject. The source of tissue or cell samples can be solid tissues, e.g., from fresh, frozen, and/or preserved organ or tissue samples or biopsy samples or puncture samples; blood or any blood component; body fluids such as tears, vitreous humors, cerebrospinal fluids, amniotic fluids, peritoneal fluids, or interstitial fluids; and cells from a subject at any time during pregnancy or development. In some embodiments, the tissue sample is a tumor tissue. The tissue sample may comprise compounds that are not naturally intermixed with the tissue in nature, such as preservatives, anticoagulants, buffers, fixatives, nutrients, and antibiotics.

### II. Antibody of the Present Disclosure

One specific aspect of the present disclosure relates to an anti-GDF15 antibody or an antigen-binding fragment thereof.

In some embodiments, the anti-GDF15 antibody of the present disclosure is a full-length antibody or an antigen-binding fragment thereof.

In some embodiments, the anti-GDF15 antibody or the antigen-binding fragment thereof of the present disclosure comprises 3 complementarity determining regions from a heavy chain variable region (HCDRs): HCDR1, HCDR2, and HCDR3.

In some embodiments, the anti-GDF15 antibody or the antigen-binding fragment thereof of the present disclosure comprises 3 complementarity determining regions from a light chain variable region (LCDRs): LCDR1, LCDR2, and LCDR3.

In some embodiments, the anti-GDF15 antibody or the antigen-binding fragment thereof of the present disclosure comprises 3 complementarity determining regions from a heavy chain variable region (HCDRs) and 3 complementarity determining regions from a light chain variable region (LCDRs).

In some aspects, the anti-GDF15 antibody or the antigen-binding fragment thereof of the present disclosure comprises a heavy chain variable region (VH). In some aspects, the anti-GDF15 antibody or the antigen-binding fragment thereof of the present disclosure comprises a light chain variable region (VL). In some aspects, the anti-GDF15 antibody or the antigen-binding fragment thereof of the present disclosure comprises a heavy chain variable region (VH) and a light chain variable region (VL). In some embodiments, the heavy chain variable region comprises 3 complementarity determining regions (CDRs) from a heavy chain variable region: HCDR1, HCDR2, and HCDR3. In some embodiments, the light chain variable region comprises 3 complementarity determining regions (CDRs) from a light chain variable region: LCDR1, LCDR2, and LCDR3.

In some embodiments, the anti-GDF15 antibody or the antigen-binding fragment thereof of the present disclosure further comprises an antibody heavy chain constant region HC. In some embodiments, the anti-GDF15 antibody or the antigen-binding fragment thereof of the present disclosure further comprises an antibody light chain constant region LC. In some embodiments, the anti-GDF15 antibody or the antigen-binding fragment thereof of the present disclosure further comprises a heavy chain constant region HC and a light chain constant region LC.

In some embodiments, the heavy chain variable region VH of the anti-GDF15 antibody of the present disclosure
(i) comprises or consists of an amino acid sequence having at least 90%, 91%, 92%, 93%, 94%, 95%, 96%, 97%, 98%, or 99% identity to the amino acid sequence of SEQ ID NO: 7, 14, 23, or 25; or
(ii) comprises or consists of the amino acid sequence of SEQ ID NO: 7, 14, 23, or 25; or
(iii) comprises or consists of an amino acid sequence having one or more (preferably no more than 10, and more preferably no more than 5, 4, 3, 2, or 1) amino acid modifications (preferably amino acid substitutions, and more preferably conservative amino acid substitutions) compared to the amino acid sequence of SEQ ID NO: 7, 14, 23, or 25, wherein preferably, the amino acid modifications do not occur in the CDRs.

In some embodiments, the light chain variable region VL of the anti-GDF15 antibody of the present disclosure
(i) comprises or consists of an amino acid sequence having at least 90%, 91%, 92%, 93%, 94%, 95%, 96%, 97%, 98%, or 99% identity to the amino acid sequence of SEQ ID NO: 8, 15, 24, or 26; or
(ii) comprises or consists of the amino acid sequence of SEQ ID NO: 8, 15, 24, or 26; or
(iii) comprises or consists of an amino acid sequence having one or more (preferably no more than 10, and more preferably no more than 5, 4, 3, 2, or 1) amino acid modifications (preferably amino acid substitutions, and more preferably conservative amino acid substitutions) compared to the amino acid sequence of SEQ ID NO: 8, 15, 24, or 26, wherein preferably, the amino acid modifications do not occur in the CDRs.

In some embodiments, the 3 complementarity determining regions from the heavy chain variable region (HCDRs) of the anti-GDF15 antibody of the present disclosure, HCDR1, HCDR2, and HCDR3, are
(i) three complementarity determining regions HCDR1, HCDR2, and HCDR3 contained in the VH set forth in SEQ ID NO: 7, 14, 23, or 25; or
(ii) three HCDRs collectively comprising at least one and no more than 5, 4, 3, 2, or 1 amino acid modification (preferably amino acid substitution, and preferably conservative substitution) relative to the sequences of (i);
preferably, the HCDRs are determined according to Kabat.

In some embodiments, the 3 complementarity determining regions from the light chain variable region (LCDRs) of the anti-GDF15 antibody of the present disclosure, LCDR1, LCDR2, and LCDR3, are
(i) three complementarity determining regions LCDR1, LCDR2, and LCDR3 contained in the VL set forth in SEQ ID NO: 8, 15, 24, or 26; or
(ii) three LCDRs collectively comprising at least one and no more than 5, 4, 3, 2, or 1 amino acid modification (preferably amino acid substitution, and preferably conservative substitution) relative to the sequences of (i);
preferably, the LCDRs are determined according to Kabat.

In some embodiments, the HCDR1 comprises or consists of the amino acid sequence set forth in SEQ ID NO: 1, or an amino acid sequence having 1, 2, or 3 amino acid modifications (preferably amino acid substitutions, and preferably conservative substitutions) compared to the amino acid sequence.

In some embodiments, the HCDR2 comprises or consists of the amino acid sequence set forth in SEQ ID NO: 2, 9, or 20, or an amino acid sequence having 1, 2, or 3 amino acid modifications (preferably amino acid substitutions, and preferably conservative substitutions) compared to the amino acid sequence.

In some embodiments, the HCDR3 comprises or consists of the amino acid sequence set forth in SEQ ID NO: 3 or 10, or an amino acid sequence having 1, 2, or 3 amino acid modifications (preferably amino acid substitutions, and preferably conservative substitutions) compared to the amino acid sequence.

In some embodiments, the LCDR1 comprises or consists of the amino acid sequence set forth in SEQ ID NO: 4, 11, or 21, or an amino acid sequence having 1, 2, or 3 amino acid modifications (preferably amino acid substitutions, and preferably conservative substitutions) compared to the amino acid sequence.

In some embodiments, the LCDR2 comprises or consists of the amino acid sequence set forth in SEQ ID NO: 5, 12, or 22, or an amino acid sequence having 1, 2, or 3 amino acid modifications (preferably amino acid substitutions, and preferably conservative substitutions) compared to the amino acid sequence.

In some embodiments, the LCDR3 comprises or consists of the amino acid sequence set forth in SEQ ID NO: 6 or 13, or an amino acid sequence having 1, 2, or 3 amino acid modifications (preferably amino acid substitutions, and preferably conservative substitutions) compared to the amino acid sequence.

In some embodiments, in the anti-GDF15 antibody of the present disclosure,
the HCDR1 comprises or consists of the amino acid sequence of SEQ ID NO: 1; the HCDR2 comprises or consists of the amino acid sequence of SEQ ID NO: 2; the HCDR3 comprises or consists of the amino acid sequence of SEQ ID NO: 3; the LCDR1 comprises or consists of the amino acid sequence of SEQ ID NO: 4; the LCDR2 comprises or consists of the amino acid sequence of SEQ ID NO: 5; and/or the LCDR3 comprises or consists of the amino acid sequence of SEQ ID NO: 6;
the HCDR1 comprises or consists of the amino acid sequence of SEQ ID NO: 1; the HCDR2 comprises or consists of the amino acid sequence of SEQ ID NO: 9; the HCDR3 comprises or consists of the amino acid sequence of SEQ ID NO: 10; the LCDR1 comprises or consists of the amino acid sequence of SEQ ID NO: 11; the LCDR2 comprises or consists of the amino acid sequence of SEQ ID NO: 12; and/or the LCDR3 comprises or consists of the amino acid sequence of SEQ ID NO: 13; or
the HCDR1 comprises or consists of the amino acid sequence of SEQ ID NO: 1; the HCDR2 comprises or consists of the amino acid sequence of SEQ ID NO: 20; the HCDR3 comprises or consists of the amino acid sequence of SEQ ID NO: 3; the LCDR1 comprises or consists of the amino acid sequence of SEQ ID NO: 21; the LCDR2 comprises or consists of the amino acid sequence of SEQ ID NO: 22; and/or the LCDR3 comprises or consists of the amino acid sequence of SEQ ID NO: 6.

In some specific embodiments of the present disclosure, the anti-GDF15 antibody or the antigen-binding fragment thereof of the present disclosure comprises a VH and a VL, wherein
the VH comprises or consists of the amino acid sequence set forth in SEQ ID NO: 7 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 8 or an amino acid sequence having at least 90%, 91%, 92%, 93%, 94%, 95%, 96%, 97%, 98%, or 99% identity thereto;
the VH comprises or consists of the amino acid sequence set forth in SEQ ID NO: 14 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 15 or an amino acid sequence having at least 90%, 91%, 92%, 93%, 94%, 95%, 96%, 97%, 98%, or 99% identity thereto;
the VH comprises or consists of the amino acid sequence set forth in SEQ ID NO: 23 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 24 or an amino acid sequence having at least 90%, 91%, 92%, 93%, 94%, 95%, 96%, 97%, 98%, or 99% identity thereto; or
the VH comprises or consists of the amino acid sequence set forth in SEQ ID NO: 25 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 26 or an amino acid sequence having at least 90%, 91%, 92%, 93%, 94%, 95%, 96%, 97%, 98%, or 99% identity thereto.

In some specific embodiments of the present disclosure, the anti-GDF15 antibody or the antigen-binding fragment thereof of the present disclosure comprises
(i). three complementarity determining regions HCDR1, HCDR2, and HCDR3 contained in the VH set forth in SEQ ID NO: 7, and three complementarity determining regions LCDR1, LCDR2, and LCDR3 contained in the VL set forth in SEQ ID NO: 8;
(ii). three complementarity determining regions HCDR1, HCDR2, and HCDR3 contained in the VH set forth in SEQ ID NO: 14, and three complementarity determining regions LCDR1, LCDR2, and LCDR3 contained in the VL set forth in SEQ ID NO: 15;
(iii).three complementarity determining regions HCDR1, HCDR2, and HCDR3 contained in the VH set forth in SEQ ID NO: 23, and three complementarity determining regions LCDR1, LCDR2, and LCDR3 contained in the VL set forth in SEQ ID NO: 24; or
(iv). three complementarity determining regions HCDR1, HCDR2, and HCDR3 contained in the VH set forth in SEQ ID NO: 25, and three complementarity determining regions LCDR1, LCDR2, and LCDR3 contained in the VL set forth in SEQ ID NO: 26.

In some specific embodiments of the present disclosure, the anti-GDF15 antibody or the antigen-binding fragment thereof of the present disclosure comprises:
the HCDR1 set forth in SEQ ID NO: 1, the HCDR2 set forth in SEQ ID NO: 2, the HCDR3 set forth in SEQ ID NO: 3, the LCDR1 set forth in SEQ ID NO: 4, the LCDR2 set forth in SEQ ID NO: 5, and the LCDR3 set forth in SEQ ID NO: 6;
the HCDR1 set forth in SEQ ID NO: 1, the HCDR2 set forth in SEQ ID NO: 9, the HCDR3 set forth in SEQ ID NO: 10, the LCDR1 set forth in SEQ ID NO: 11, the LCDR2 set forth in SEQ ID NO: 12, and the LCDR3 set forth in SEQ ID NO: 13; or
the HCDR1 set forth in SEQ ID NO: 1, the HCDR2 set forth in SEQ ID NO: 20, the HCDR3 set forth in SEQ ID NO: 3, the LCDR1 set forth in SEQ ID NO: 21, the LCDR2 set forth in SEQ ID NO: 22, and the LCDR3 set forth in SEQ ID NO: 6.

In some specific embodiments of the present disclosure, the anti-GDF15 antibody or the antigen-binding fragment thereof of the present disclosure comprises a VH and a VL, wherein
the VH comprises or consists of the amino acid sequence set forth in SEQ ID NO: 7, and the VL comprises or consists of the amino acid sequence set forth in SEQ ID NO: 8;
the VH comprises or consists of the amino acid sequence set forth in SEQ ID NO: 14, and the VL comprises or consists of the amino acid sequence set forth in SEQ ID NO: 15;
the VH comprises or consists of the amino acid sequence set forth in SEQ ID NO: 23, and the VL comprises or consists of the amino acid sequence set forth in SEQ ID NO: 24; or
the VH comprises or consists of the amino acid sequence set forth in SEQ ID NO: 25, and the VL comprises or consists of the amino acid sequence set forth in SEQ ID NO: 26.

In one embodiment of the present disclosure, the amino acid modification described herein includes amino acid substitutions, insertions, or deletions. In a preferred embodiment, the amino acid modification described in the present disclosure occurs in a region outside the CDRs (e.g., in an FR). More preferably, the amino acid modification described in the present disclosure occurs in a region outside the heavy chain variable regions and/or outside the light chain variable regions. Preferably, the amino acid modification described herein is an amino acid substitution, preferably a conservative substitution.

In some embodiments, the anti-GDF15 antibody or the antigen-binding fragment thereof of the present disclosure has one or more of the following properties:
(i) exhibiting the same or similar binding affinity and/or specificity for GDF15 as the antibody of the present disclosure;
(ii) inhibiting (e.g., competitively inhibiting) the binding of the antibody of the present disclosure to GDF15;
(iii) binding to the same or an overlapping epitope as the antibody of the present disclosure;
(iv) competing with the antibody of the present disclosure for binding to GDF15; and
(v) having one or more biological properties of the antibody of the present disclosure.

In some embodiments, the anti-GDF15 antibody or the antigen-binding fragment thereof of the present disclosure is an antibody in the form of IgG1, IgG2, IgG3, or IgG4, or an antigen-binding fragment thereof, e.g., an antibody in the form of IgG1 or an antigen-binding fragment thereof. In some embodiments, the heavy chain constant region of the anti-GDF15 antibody of the present disclosure is a constant region of IgG1, IgG2, IgG3, or IgG4, e.g., a constant region of IgG1. The "constant region" includes a native constant region, an allotype, or a native variant, e.g., D356E and L358M.

In some embodiments, the IgG1 constant region of the present disclosure comprises an Fc region. As understood by those skilled in the art, according to the expected use of the antibody molecule of the present disclosure, the antibody molecule of the present disclosure may further comprise one or more modifications in the Fc region. In one embodiment, the Fc region is modified in a property of its effector function (e.g., complement activation function of the Fc region). In one embodiment, the effector function has been reduced or eliminated relative to a wild-type isotype Fc region. In one embodiment, the effector function is reduced or eliminated by using a method selected from: using an Fc isotype that naturally has a reduced or an eliminated effector function, and performing Fc region modification. In one preferred embodiment, the Fc region has a reduced effector function mediated by the Fc region, such as a reduced or an eliminated ADCC or ADCP or CDC effector function, for example, comprising a mutation(s) for achieving the above function.

In some embodiments, the antibody molecule of the present disclosure may further comprise, in the Fc region, a modification(s) that alters the binding affinity to one or more Fc receptors. In one embodiment, the Fc receptor is an Fcγ receptor, in particular a human Fcγ receptor. In some embodiments, the Fc region comprises a mutation(s) that reduces binding to the Fcγ receptor. For example, in some embodiments, the Fc region for use in the present disclosure has one or more of L234A/L235A mutations and a G237A mutation, which reduce binding to the Fcγ receptor. In some embodiments, the Fc region for use in the present disclosure has L234A/L235A mutations and a G237A mutation, which reduce binding to the Fcγ receptor.

In yet another preferred embodiment, the Fc region may have a mutation(s) that leads to an increased serum half-life, e.g., a mutation(s) that improves the binding of the Fc fragment to FcRn. In some embodiments, the Fc region of the present disclosure has a mutation(s) that extends the half-life, e.g., M252Y/S254T/T256E mutations (YTE mutations). In some embodiments, the Fc region of the present disclosure comprises M252Y/S254T/T256E mutations.

In some embodiments, the antibody molecule of the present disclosure has, in the Fc region, a mutation(s) that may reduce the binding to the Fcγ receptor and/or extend the half-life and/or increase the affinity for FcRn, e.g., L234A/L235A mutations and/or a G237A mutation and/or M252Y/S254T/T256E mutations. In some embodiments, the Fc region of the present disclosure comprises L234A/L235A mutations, a G237A mutation, and M252Y/S254T/T256E mutations.

In some embodiments, the amino acid sequence of the Fc region suitable for use in the antibody molecule of the present disclosure comprises or consists of SEQ ID NO: 43, or an amino acid sequence having at least 90% identity, e.g., 95%, 96%, 97%, 99%, or higher identity, thereto. In some embodiments, the Fc region comprises an amino acid sequence having at least 90% identity, e.g., 95%, 96%, 97%, 99%, or higher identity, to SEQ ID NO: 43, and comprises L234A/L235A mutations and a G237A mutation.

In some embodiments, the amino acid sequence of the Fc region suitable for use in the antibody molecule of the present disclosure comprises or consists of SEQ ID NO: 47, or an amino acid sequence having at least 90% identity, e.g., 95%, 96%, 97%, 99%, or higher identity, thereto. In some embodiments, the Fc region comprises an amino acid sequence having at least 90% identity, e.g., 95%, 96%, 97%, 99%, or higher identity, to SEQ ID NO: 47, and comprises L234A/L235A mutations, a G237A mutation, and M252Y/S254T/T256E mutations.

In some embodiments, the IgG1 heavy chain constant region suitable for use in the antibody molecule of the present disclosure comprises or consists of the amino acid sequence of SEQ ID NO: 44, or an amino acid sequence having at least 90% identity, e.g., 95%, 96%, 97%, 99%, or higher identity, thereto. In some embodiments, the heavy chain constant region comprises an amino acid sequence having at least 90% identity, e.g., 95%, 96%, 97%, 99%, or higher identity, to the amino acid sequence of SEQ ID NO: 44, and comprises L234A/L235A mutations and a G237A mutation.

In some embodiments, the IgG1 heavy chain constant region suitable for use in the antibody molecule of the present disclosure comprises or consists of the amino acid sequence of SEQ ID NO: 48, or an amino acid sequence having at least 90% identity, e.g., 95%, 96%, 97%, 99%, or higher identity, thereto. In some embodiments, the heavy chain constant region comprises an amino acid sequence having at least 90% identity, e.g., 95%, 96%, 97%, 99%, or higher identity, to the amino acid sequence of SEQ ID NO: 48, and comprises L234A/L235A mutations, a G237A mutation, and M252Y/S254T/T256E mutations.

In some embodiments, the light chain constant region of the anti-GDF15 antibody of the present disclosure is a kappa or lambda light chain constant region, e.g., a kappa light chain constant region.

In some embodiments, the light chain constant region comprises or consists of the amino acid sequence of SEQ ID NO: 45, or an amino acid sequence having at least 90% identity, e.g., 95%, 96%, 97%, 99%, or higher identity, thereto.

In some embodiments, the anti-GDF15 antibody or the antigen-binding fragment thereof of the present disclosure comprises an antibody heavy chain. In some embodiments, the anti-GDF15 antibody or the antigen-binding fragment thereof of the present disclosure comprises an antibody light chain. In some embodiments, the anti-GDF15 antibody or the antigen-binding fragment thereof of the present disclosure comprises a heavy chain and a light chain. In some embodiments, the anti-GDF15 antibody or the antigen-binding fragment thereof of the present disclosure comprises two heavy chains and two light chains.

In one embodiment, the heavy chain of the anti-GDF15 antibody of the present disclosure
(i) comprises or consists of an amino acid sequence having at least 90%, 91%, 92%, 93%, 94%, 95%, 96%, 97%, 98%, or 99% identity to the amino acid sequence of SEQ ID NO: 28, 30, 33, 35, or 49; or
(ii) comprises or consists of the amino acid sequence of SEQ ID NO: 28, 30, 33, 35, or 49; or
(iii) comprises or consists of an amino acid sequence having one or more (preferably no more than 10, and more preferably no more than 5, 4, 3, 2, or 1) amino acid modifications (preferably amino acid substitutions, and more preferably conservative amino acid substitutions) compared to the amino acid sequence of SEQ ID NO: 28, 30, 33, 35, or 49, wherein preferably, the amino acid modifications do not occur in the CDRs, and more preferably, the amino acid modifications do not occur in the variable region.

In some embodiments, the light chain of the anti-GDF15 antibody of the present disclosure
(i) comprises or consists of an amino acid sequence having at least 90%, 91%, 92%, 93%, 94%, 95%, 96%, 97%, 98%, or 99% identity to the amino acid sequence of SEQ ID NO: 29, 31, 34, or 36; or
(ii) comprises or consists of the amino acid sequence of SEQ ID NO: 29, 31, 34, or 36; or
(iii) comprises or consists of an amino acid sequence having one or more (preferably no more than 10, and more preferably no more than 5, 4, 3, 2, or 1) amino acid modifications (preferably amino acid substitutions, and more preferably conservative amino acid substitutions) compared to the amino acid sequence of SEQ ID NO: 29, 31, 34, or 36, wherein preferably, the amino acid modifications do not occur in the CDRs, and more preferably, the amino acid modifications do not occur in the variable region.

In some specific embodiments of the present disclosure, the anti-GDF15 antibody or the antigen-binding fragment thereof of the present disclosure comprises a heavy chain and a light chain, wherein
(i). the heavy chain comprises or consists of the amino acid sequence set forth in SEQ ID NO: 28 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 29 or an amino acid sequence having at least 90%, 91%, 92%, 93%, 94%, 95%, 96%, 97%, 98%, or 99% identity thereto;
(ii). the heavy chain comprises or consists of the amino acid sequence set forth in SEQ ID NO: 30 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 31 or an amino acid sequence having at least 90%, 91%, 92%, 93%, 94%, 95%, 96%, 97%, 98%, or 99% identity thereto;
(iii).the heavy chain comprises or consists of the amino acid sequence set forth in SEQ ID NO: 33 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 34 or an amino acid sequence having at least 90%, 91%, 92%, 93%, 94%, 95%, 96%, 97%, 98%, or 99% identity thereto;
(iv). the heavy chain comprises or consists of the amino acid sequence set forth in SEQ ID NO: 35 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 36 or an amino acid sequence having at least 90%, 91%, 92%, 93%, 94%, 95%, 96%, 97%, 98%, or 99% identity thereto; or
(v). the heavy chain comprises or consists of the amino acid sequence set forth in SEQ ID NO: 49 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 34 or an amino acid sequence having at least 90%, 91%, 92%, 93%, 94%, 95%, 96%, 97%, 98%, or 99% identity thereto.

In some specific embodiments of the present disclosure, the anti-GDF15 antibody or the antigen-binding fragment thereof of the present disclosure comprises a heavy chain and a light chain, wherein
(i). the heavy chain comprises or consists of the amino acid sequence set forth in SEQ ID NO: 28, and the light chain comprises or consists of the amino acid sequence set forth in SEQ ID NO: 29;
(ii). the heavy chain comprises or consists of the amino acid sequence set forth in SEQ ID NO: 30, and the light chain comprises or consists of the amino acid sequence set forth in SEQ ID NO: 31;
(iii).the heavy chain comprises or consists of the amino acid sequence set forth in SEQ ID NO: 33, and the light chain comprises or consists of the amino acid sequence set forth in SEQ ID NO: 34;
(iv). the heavy chain comprises or consists of the amino acid sequence set forth in SEQ ID NO: 35, and the light chain comprises or consists of the amino acid sequence set forth in SEQ ID NO: 36; or
(v). the heavy chain comprises or consists of the amino acid sequence set forth in SEQ ID NO: 49, and the light chain comprises or consists of the amino acid sequence set forth in SEQ ID NO: 34.

In some embodiments, the anti-GDF15 antibody is a monoclonal antibody.

In some embodiments, the anti-GDF15 antibody is humanized. The humanization may be achieved by the following method: substituting one or more amino acid residues, particularly in framework region sequences, of the heavy and light chain variable regions of a native antibody of a non-human origin with residues from the corresponding positions in the variable regions of a conventional human antibody. Methods for humanizing antibodies are well known in the art. Typically, the humanization substitutions are made in a manner that preserves the favorable binding properties of the antibody. Assays for determining the biological properties, such as binding affinity, of the humanized antibody to identify and select a suitable humanized residue mutation or mutation combination are well known in the art.

In some embodiments, the humanized antibody of the present disclosure may be obtained by a method comprising the following steps:
1) determining the CDR loop structures of a parent antibody (e.g., an antibody obtained from hybridoma preparation);
2) finding the closest homologous sequence for each V/J region in a human germline sequence database (e.g., the IMGT database);
3) screening for a human germline framework with the best match to the heavy chain and the minimum amount of back-mutations, wherein in one embodiment of the present disclosure, the human germline gene sequence IGHV1-3*01 serves as the humanized framework of the heavy chain variable region; and constructing the CDRs of the chimeric antibody onto the human framework regions;
4) screening for a human germline framework with the best match to the light chain and the minimum amount of back-mutations, wherein in one embodiment of the present disclosure, the human germline gene is IGKV6-21*01; and constructing the CDRs of the chimeric antibody onto the human framework regions;
5) determining amino acid positions in the framework regions that maintain the CDR functions by using sequence and structural features;
6) performing back-mutations (reverting to the input amino acid type) at sequence positions determined as important;
7) optionally, optimizing amino acids at risk sites; and
8) obtaining the humanized antibody, and optionally sequencing the antibody sequence.

In one embodiment, the anti-GDF15 antibody of the present disclosure further encompasses an antibody fragment (e.g., an antigen-binding fragment) thereof, preferably an antibody fragment selected from: a Fab, a Fab', a Fab'-SH, an Fv, a single-chain antibody (e.g., scFv), a (Fab')₂, a single-domain antibody (e.g., VHH), a domain antibody (dAb), a heavy-chain antibody, and a linear antibody. In one embodiment, the anti-GDF15 antibody fragment of the present disclosure is a Fab comprising the VH and VL, and the CH1 and CL described herein.

In one embodiment, the anti-GDF15 antibody of the present disclosure may also be a bispecific antibody or a multispecific antibody that specifically binds to GDF15 and one or more other antigens.

Another specific aspect of the present disclosure relates to an anti-GDF15 antibody, which is an anti-GDF15 VHH antibody or a heavy-chain antibody comprising same.

### Single-domain antibody

In some embodiments, the anti-GDF15 antibody of the present disclosure is a single-domain antibody, particularly a VHH antibody.

The single-domain antibody or the VHH antibody has a molecular weight of about one-tenth of a human IgG molecule, and a physical diameter of only a few nanometers. Due to the small molecular size, a single-domain monoclonal antibody has the following advantages over conventional four-chain antibodies: high stability and solubility, and the ability to recognize hidden antigenic sites. In addition, the single-domain antibody is also cheaper to prepare than conventional four-chain antibodies. In addition to being used as an individual molecule, the single-domain antibody is also a suitable component for the construction of multispecific molecules.

In some embodiments, the anti-GDF15 single-domain antibody of the present disclosure is a VHH antibody comprising or consisting of a heavy chain variable region typically having the following structure: FR1-VHH CDR1-FR2-VHH CDR2-FR3-VHH CDR3-FR4, wherein FR1 to FR4 refer to framework regions 1-4; VHH CDR1 to VHH CDR3 refer to complementarity determining regions 1-3. The CDR sequences in the VHH variable region may be determined according to any of the CDR definition schemes described in the "Definition" section, and preferably, the boundaries of the three CDRs in the VHH sequence may be defined according to IMGT.

In some embodiments, the anti-GDF15 VHH antibody of the present disclosure comprises
(i) three complementarity determining regions (CDRs) contained in a VH set forth in any one of SEQ ID NOs: 19 and 27, or
(ii) three CDRs collectively comprising at least one and no more than 5, 4, 3, 2, or 1 amino acid modification (preferably amino acid substitution, and preferably conservative substitution) relative to the sequences of (i);
preferably, the sequences of the CDRs are defined according to IMGT.

In some embodiments, the anti-GDF15 VHH antibody of the present disclosure comprises or consists of a heavy chain variable region comprising
(i) three complementarity determining regions (CDRs) contained in a VH set forth in any one of SEQ ID NOs: 19 and 27, or
(ii) three CDRs collectively comprising at least one and no more than 5, 4, 3, 2, or 1 amino acid modification (preferably amino acid substitution, and preferably conservative substitution) relative to the sequences of (i);
preferably, the sequences of the CDRs are defined according to IMGT.

In some embodiments, the anti-GDF15 VHH antibody of the present disclosure comprises complementarity determining regions (CDRs) VHH CDR1, VHH CDR2, and VHH CDR3. In some embodiments, the anti-GDF15 VHH of the present disclosure comprises or consists of a heavy chain variable region comprising complementarity determining regions (CDRs) VHH CDR1, VHH CDR2, and VHH CDR3.

In some embodiments, the VHH CDR1 comprises or consists of the amino acid sequence of SEQ ID NO: 16, or the VHH CDR1 comprises an amino acid sequence having one, two, or three modifications (preferably amino acid substitutions, and preferably conservative substitutions) compared to the amino acid sequence of SEQ ID NO: 16.

In some embodiments, the VHH CDR2 comprises or consists of the amino acid sequence of SEQ ID NO: 17, or the VHH CDR2 comprises an amino acid sequence having one, two, or three modifications (preferably amino acid substitutions, and preferably conservative substitutions) compared to the amino acid sequence of SEQ ID NO: 17.

In some embodiments, the VHH CDR3 comprises or consists of the amino acid sequence of SEQ ID NO: 18, or the VHH CDR3 comprises an amino acid sequence having one, two, or three modifications (preferably amino acid substitutions, and preferably conservative substitutions) compared to the amino acid sequence of SEQ ID NO: 18.

In one embodiment, the anti-GDF15 VHH antibody of the present disclosure comprises complementarity determining regions (CDRs) VHH CDR1, VHH CDR2, and VHH CDR3, wherein the VHH CDR1 comprises or consists of the amino acid sequence set forth in SEQ ID NO: 16, the VHH CDR2 comprises or consists of the amino acid sequence set forth in SEQ ID NO: 17, and the VHH CDR3 comprises or consists of the amino acid sequence set forth in SEQ ID NO: 18.

In one embodiment, the anti-GDF15 VHH antibody of the present disclosure comprises or consists of a heavy chain variable region, wherein the heavy chain variable region comprises complementarity determining regions (CDRs) VHH CDR1, VHH CDR2, and VHH CDR3, wherein
the VHH CDR1 comprises or consists of the amino acid sequence set forth in SEQ ID NO: 16, the VHH CDR2 comprises or consists of the amino acid sequence set forth in SEQ ID NO: 17, and the VHH CDR3 comprises or consists of the amino acid sequence set forth in SEQ ID NO: 18.

In some embodiments, the anti-GDF15 VHH antibody of the present disclosure comprises or consists of a heavy chain variable region, wherein the heavy chain variable region
(i) comprises or consists of an amino acid sequence having at least 90%, 91%, 92%, 93%, 94%, 95%, 96%, 97%, 98%, or 99% identity to an amino acid sequence selected from any one of SEQ ID NOs: 19 and 27; or
(ii) comprises or consists of an amino acid sequence selected from any one of SEQ ID NOs: 19 and 27; or
(iii) comprises an amino acid sequence having one or more (preferably no more than 10, and more preferably no more than 5, 4, 3, 2, or 1) amino acid modifications (preferably amino acid substitutions, and more preferably conservative amino acid substitutions) compared to an amino acid sequence selected from any one of SEQ ID NOs: 19 and 27, wherein preferably, the amino acid modifications do not occur in the CDRs.

In some embodiments, the anti-GDF15 VHH antibody of the present disclosure comprises or consists of an amino acid sequence selected from any one of SEQ ID NOs: 19 and 27.

In some embodiments, the VHH antibody of the present disclosure comprises CDR amino acid sequences and/or framework (FR) amino acid sequences derived from a Camelidae heavy-chain antibody produced by immunizing an animal of the Camelidae family (e.g., an alpaca). In some embodiments, a VHH monoclonal antibody of the present disclosure derived from the Camelidae heavy-chain antibody may be engineered, for example, to comprise framework region sequences derived from human amino acid sequences (i.e., human antibodies) or from other non-Camelidae mammalian species. In one embodiment, to further improve the properties (e.g., affinity) of the engineered antibody, camelid-derived amino acid residues at corresponding positions in the parent camelid-derived antibody may be introduced into the engineered antibody by a back-mutation(s) at one or more positions (e.g., framework regions).

In one embodiment, the VHH antibody of the present disclosure is a humanized antibody. The humanization may be achieved by the following method: substituting one or more amino acid residues, particularly in framework region sequences, of a native VHH sequence of a non-human origin (e.g., a VHH sequence derived from an animal of the Camelidae family or an alpaca after immunization) with residues from the corresponding positions in the heavy chain VH of a conventional human antibody. Methods for humanizing VHHs are well known in the art, for example, the method described in Example 3. Generally, the humanization substitutions are made in a manner that preserves the favorable binding properties of the single-domain antibody. Assays for determining the biological properties, such as binding affinity, of the humanized single-domain antibody to identify and select a suitable humanized residue mutation or mutation combination are well known in the art.

In some embodiments, the humanized single-domain antibody of the present disclosure may be obtained by a method comprising the following steps:
determining the CDR loop structures of a parent single-domain antibody (e.g., a camelid-derived VHH antibody obtained from screening in a phage display library);
finding the closest homologous sequence in a human germline sequence database as a template for each V/J region, for example, by aligning with the IMGT human antibody heavy chain variable region germline gene database (http://www.imgt.org/3Dstructure-DB/cgi/DomainGapAlign.cgi), and selecting a heavy chain variable region germline gene with high homology to the VHH antibody as a template;
grafting the CDRs of the VHH antibody into the selected corresponding humanized templates, respectively, to form a variable region sequence in the order of FR1-CDR1-FR2-CDR2-FR3-CR3-FR4, wherein preferably, the framework sequences used for substitution are structurally similar to the framework sequences of the antibody to be humanized, e.g., with at least 80%, 85%, 90%, or 95%, 96%, 97%, 98%, 99% or more sequence identity; and
back-mutating key amino acids in the FRs to amino acids corresponding to the VHH antibody as needed to maintain the original affinity, so as to obtain the humanized anti-GDF15 VHH antibody, and optionally sequencing the VHH antibody.

In some embodiments, the back-mutation site is selected from one or more in the frameworks (FRs).

In some embodiments, the heavy chain variable region germline gene suitable for humanizing the VHH antibody of the present disclosure is selected from IGHV3-23*04.

In some embodiments, the present disclosure further provides a functional variant of the single-domain antibody (particularly the VHH antibody) of the present disclosure. The functional variant may be obtained by well-known methods in the present disclosure, for example, by introducing a mutation(s) into the coding nucleic acid sequences (e.g., CDR sequences and/or FR sequences) of an exemplary single-domain antibody of the present disclosure via random or site-directed mutagenesis, and then screening (e.g., by phage display library screening) for variants that retain the desired properties. Generally, the functional variant retains significant sequence identity to the parent single-domain antibody (or VHH). Preferably, the functional variant retains the desired biological properties of the parent single-domain antibody (or VHH). For example, the variant has comparable biological activity (e.g., at least 50%, 60%, 70%, or 80%, preferably 90% or more) or improved biological activity (e.g., 110%-150% or higher) relative to the biological activity of the parent. The desired biological properties include, for example, but are not limited to, binding affinity for an antigen of interest (e.g., GDF15) (as measured by KD value), activity in blocking the binding of the antigen of interest to a receptor (e.g., as measured by IC₅₀ value), activity in activating T cells in *in-vitro* or *in-vivo* assays (e.g., as measured by release amount of cytokines), and inhibition of tumor growth/survival in *in-vitro* or *in-vivo* assays.

In some embodiments, the present disclosure provides an affinity variant of a VHH polypeptide of the present disclosure. Preferably, the affinity variant exhibits one or more amino acid modifications in the amino acid sequence relative to the parent single-domain antibody from which it is derived, wherein the affinity variant has altered binding affinity for an antigen of interest as compared to the parent antibody.

### Heavy-chain antibody

In another aspect of the present disclosure, the present disclosure further provides a heavy-chain antibody comprising the heavy chain variable region of the VHH antibody of the present disclosure.

In some embodiments, the single-domain antibody or the VHH (e.g., a camelid-derived VHH or a humanized form thereof) of the present disclosure may be linked to a constant region of a human antibody or a portion thereof (e.g., an Fc region) to produce a heavy-chain antibody comprising a VHH-constant region, VHH-CH1-Fc, or VHH-Fc. In one embodiment, the heavy-chain antibody comprises the VHH antibody of the present disclosure and the Fc region located at the C-terminus thereof. In some embodiments, the VHH is linked to the Fc via a hinge region or a portion thereof, e.g., a hinge region from IgG (e.g., a hinge region from IgG1, IgG2, IgG3, or IgG4) or a portion thereof.

In some embodiments, the anti-GDF15 heavy-chain antibody of the present disclosure comprises the VHH as defined herein, and the heavy chain constant region or the Fc region of the heavy chain constant region as defined herein. In some embodiments, the Fc region comprises an antibody hinge region or a portion thereof, e.g., a hinge region from IgG or a portion thereof. The hinge region may comprise a mutation(s). In some embodiments, the hinge region may be EPKSS or EPKSC.

In one embodiment, the heavy-chain antibody comprises an Fc region or a portion thereof from an animal of the Camelidae family (e.g., an alpaca). In one embodiment, the heavy-chain antibody is produced and isolated by immunizing an animal of the Camelidae family, e.g., an alpaca. A variety of methods known in the art can be used for immunizing the animal of the Camelidae family and isolating the VHH antibody or the heavy-chain antibody produced against the antigen of interest.

In some embodiments, the heavy-chain antibody comprises a constant region from a human or non-human primate (e.g., cynomolgus monkey) antibody, e.g., a constant region from human IgG1, human IgG2, human IgG3, or human IgG4, as defined herein.

In some embodiments, the heavy-chain antibody comprises an Fc portion from a human or non-human primate (e.g., cynomolgus monkey). In yet another embodiment, the heavy-chain antibody comprises a human IgG Fc region, e.g., a human IgG1, human IgG2, human IgG3, or human IgG4 Fc region, preferably a human IgG1 or human IgG4 Fc region, such as a human IgG1 Fc region, for example, as defined herein.

In one embodiment, the heavy-chain antibody according to the present disclosure may be dimerized, via the Fc region, with another polypeptide chain comprising an Fc region (e.g., another identical or different heavy-chain antibody). Thus, in one embodiment, the present disclosure further provides a homo- or heteromultimeric protein comprising the heavy-chain antibody of the present disclosure. In one preferred embodiment, the protein preferably comprises a heavy-chain antibody formed by pairing two identical heavy-chain antibody chains.

The Fc region of the present disclosure may be mutated to obtain desired properties. Mutations to the Fc region, as defined herein, are known in the art.

In some embodiments, the anti-GDF15 antibody or the antigen-binding fragment thereof of the present disclosure comprises a heavy chain comprising or consisting of a heavy chain variable region and an Fc region.

In some embodiments, the anti-GDF15 antibody or the antigen-binding fragment thereof of the present disclosure comprises or consists of a heavy chain comprising or consisting of a heavy chain variable region of the VHH of the present disclosure and an Fc region (e.g., the Fc region comprises a partial hinge region or a hinge region), wherein the heavy chain
(i) comprises or consists of an amino acid sequence having at least 85%, 90%, 91%, 92%, 93%, 94%, 95%, 96%, 97%, 98%, or 99% identity to an amino acid sequence selected from SEQ ID NOs: 32 and 37; or
(ii) comprises or consists of an amino acid sequence selected from SEQ ID NOs: 32 and 37; or
(iii) comprises an amino acid sequence having one or more (preferably no more than 10, and more preferably no more than 5, 4, 3, 2, or 1) amino acid modifications (preferably amino acid substitutions, and more preferably conservative amino acid substitutions) compared to an amino acid sequence selected from SEQ ID NOs: 32 and 37, wherein preferably, the amino acid modifications do not occur in the CDRs.

### III. Coding Nucleic Acid and Host Cell Comprising Same

In one aspect, the present disclosure provides a nucleic acid encoding any of the above anti-GDF15 antibodies or antigen-binding fragments thereof, or any one of the above chains thereof.

For example, the nucleic acid of the present disclosure includes a nucleic acid encoding an amino acid sequence selected from any one of SEQ ID NOs: 7, 8, 14, 15, 19, 23-37, and 49, or a nucleic acid encoding an amino acid sequence having at least 85%, 90%, 91%, 92%, 93%, 94%, 95%, 96%, 97%, 98%, or 99% identity to the amino acid sequence selected from any one of SEQ ID NOs: 7, 8, 14, 15, 19, 23-37, and 49.

As will be appreciated by those skilled in the art, each antibody or polypeptide amino acid sequence may be encoded by a variety of nucleic acid sequences because of codon degeneracy. Nucleic acid sequences encoding the molecule of the present disclosure may be generated using methods well known in the art, e.g., *de novo* solid-phase DNA synthesis, or PCR amplification.

In one aspect, the present disclosure provides a nucleic acid encoding any of the above antibodies or any of the above antibody chains. When expressed in a suitable expression vector, a polypeptide encoded by the nucleic acid is capable of exhibiting binding capacity to human and/or monkey (e.g., cynomolgus monkey) GDF15 antigen.

In one embodiment, the nucleic acids encoding the chains of the antibody or the antigen-binding fragment thereof of the present disclosure may be in the same vector or in different vectors. In another embodiment, the nucleic acids encoding the chains of the antibody or the antigen-binding fragment thereof of the present disclosure may be introduced into the same or different host cells for expression. Thus, in some embodiments, the method for producing the antibody or the antigen-binding fragment thereof of the present disclosure comprises a step of: culturing a host cell comprising the nucleic acids encoding the chains of the molecule under a condition suitable for expressing the chains, thereby producing the antibody or the antigen-binding fragment thereof of the present disclosure.

In one embodiment, provided is a vector comprising the nucleic acid. In one embodiment, the vector is an expression vector, e.g., a eukaryotic expression vector. The vector includes, but is not limited to, a virus, a plasmid, a cosmid, a λ phage, or a yeast artificial chromosome (YAC). In one embodiment, the expression vector is, for example, a pcDNA vector, such as pcDNA3.1.

In one embodiment, provided is a host cell comprising the nucleic acid or the vector, e.g., for cloning or expressing a vector encoding the anti-GDF15 antibody or the antigen-binding fragment thereof. In one embodiment, the host cell is eukaryotic. In another embodiment, the host cell is selected from a yeast cell, a mammalian cell (e.g., a CHO cell (e.g., CHO-S, such as ExpiCHO-S) or a 293 cell (e.g., 293F or HEK293 cell)), and other cells suitable for preparing an antibody or a fragment thereof. In one embodiment, the host cell is prokaryotic, e.g., a bacterium, such as *Escherichia coli*.

In some embodiments, eukaryotic microorganisms, such as filamentous fungi or yeast, are suitable cloning or expression hosts for the vector encoding the antibody. For example, fungus and yeast strains in which a glycosylation pathway has been "humanized" result in the production of antibodies with a partially or fully human glycosylation pattern. Host cells suitable for expressing glycosylated antibodies are also derived from multicellular organisms (invertebrates and vertebrates). Vertebrate cells may also be used as hosts. For example, mammalian cell lines engineered to be suitable for suspension growth may be used. Other examples of useful mammalian host cell lines are the monkey kidney CV1 line transformed with SV40 (COS-7), human embryonic kidney lines (HEK293, 293F, or 293T cells), and the like. Other useful mammalian host cell lines include Chinese hamster ovary (CHO) cells, including DHFR-CHO cells, CHO-S cells, ExpiCHO, and the like; and myeloma cell lines such as Y0, NS0, and Sp2/0. Mammalian host cell lines suitable for producing antibodies are known in the art.

### IV. Production and Purification of Anti-GDF15 Antibody or Antigen-Binding Fragment Thereof of the Present Disclosure

In one embodiment, provided is a method for preparing the anti-GDF15 antibody or the antigen-binding fragment thereof of the present disclosure, wherein the method comprises: culturing a host cell comprising a nucleic acid encoding the anti-GDF15 antibody or the antigen-binding fragment thereof (e.g., any one and/or more polypeptide chains) or an expression vector comprising the nucleic acid, as provided above, under a condition suitable for expressing the anti-GDF15 antibody or the antigen-binding fragment thereof or the chains thereof, and optionally recovering the anti-GDF15 antibody or the antigen-binding fragment thereof from the host cell (or a host cell culture medium).

Polynucleotides encoding the polypeptide chains of the anti-GDF15 antibody or the antigen-binding fragment thereof of the present disclosure may be inserted into one or more vectors for further cloning and/or expression in a host cell. Methods well known to those skilled in the art can be used to construct expression vectors. Once an expression vector comprising one or more nucleic acid molecules of the present disclosure has been prepared for expression, the expression vector may be transfected or introduced into a suitable host cell. Various techniques can be used to achieve this purpose, for example, protoplast fusion, calcium phosphate precipitation, electroporation, retroviral transduction, viral transfection, biolistics, liposome-based transfection, or other conventional techniques.

The anti-GDF15 antibody or the antigen-binding fragment thereof prepared as described herein may be purified by known existing techniques such as high-performance liquid chromatography, ion exchange chromatography, gel electrophoresis, affinity chromatography, and size exclusion chromatography. The actual conditions used for purifying a particular protein also depend on factors such as net charge, hydrophobicity, and hydrophilicity, and these will be apparent to those skilled in the art.

The purity of the anti-GDF15 antibody or the antigen-binding fragment thereof of the present disclosure may be determined by any one of a variety of well-known analytical methods including size exclusion chromatography, gel electrophoresis, high-performance liquid chromatography, and the like.

### V. Assays for Anti-GDF15 Antibody or Antigen-Binding Fragment Thereof

The anti-GDF15 antibody or the antigen-binding fragment thereof provided herein can be identified, screened, or characterized for physical/chemical properties and/or biological activities thereof through a variety of assays known in the art.

In one aspect, the anti-GDF15 antibody or the antigen-binding fragment thereof of the present disclosure is assayed for its binding activity to a target (e.g., an antigen, such as a free antigen or an antigen expressed on a cell), for example, by known methods such as biolayer interferometry, ELISA, and flow cytometry. The binding to VEGF and/or GDF15 (or VEGF and/or GDF15 expressed on a cell) can be determined using methods known in the art, and exemplary methods are disclosed herein, such as the method shown in Example 7. In some embodiments, the binding is measured using radioimmunoassay (RIA), biolayer interferometry (BLI), electrochemiluminescence (ECL), surface plasmon resonance (SPR), or flow cytometry (FACS).

The present disclosure further provides an assay for identifying the biological activity of the anti-GDF15 antibody or the antigen-binding fragment thereof. The biological activity is selected from the properties of the anti-GDF15 antibody or the antigen-binding fragment thereof of the present disclosure.

For example, the cross-reactivity of the molecule of the present disclosure with human TGFβ family proteins can be determined by methods known in the art, such as ELISA, or by the exemplary methods disclosed in the examples herein, such as the method shown in Example 8.

For example, the activity of the molecule of the present disclosure in blocking GDF15 activation can be determined by methods known in the art, such as the method shown in Example 9.

For example, the activity of the molecule of the present disclosure in inhibiting ERK phosphorylation levels can be determined by methods known in the art, such as the method shown in Example 10.

For example, the reversal or treatment of cachexia, such as tumor-induced cachexia or cachexia induced by a chemotherapeutic agent such as cisplatin, by the molecule of the present disclosure can be determined by methods known in the art, such as an *in vivo* efficacy experiment conducted on a tumor model, e.g., the method shown in Example 11 or 12.

For example, the pharmacokinetic analysis of the molecule of the present disclosure can be determined by methods known in the art, such as a pharmacokinetic experiment, e.g., the method shown in Example 13.

Cells for use in any of the above *in vitro* assays are primary cells or cell lines, including cells that naturally express or overexpress VEGF (e.g., human or monkey (e.g., cynomolgus monkey)) or GDF15 (e.g., human or monkey (e.g., cynomolgus monkey) GDF15), for example, cells overexpressing GDF15, e.g., 293 cells, CHO cells, or HT-1080 cells.

It will be appreciated that any of the assays described above can be performed using a combination of the antibody of the present disclosure and an additional active agent.

### VI. Immunoconjugate, Pharmaceutical Composition, Pharmaceutical Combination Product, and Kit of Anti-GDF15 Antibody or Antigen-Binding Fragment Thereof of the Present Disclosure

In some embodiments, the present disclosure provides an immunoconjugate comprising any of the anti-GDF15 antibodies or the antigen-binding fragments thereof described herein. Preferably, the immunoconjugate comprises one or more additional therapeutic agents (e.g., cytotoxins or small molecule compounds) or markers. In some embodiments, the immunoconjugate is an antibody-drug conjugate (ADC).

In some embodiments, the present disclosure provides a composition or a medicament or a formulation comprising any of the anti-GDF15 antibodies or the antigen-binding fragments thereof or the immunoconjugates described herein, wherein preferably, the composition is a pharmaceutical composition.

In one embodiment, the composition, e.g., the pharmaceutical composition, comprises a combination of the anti-GDF15 antibody or the antigen-binding fragment thereof or the immunoconjugate of the present disclosure, and one or more additional therapeutic agents.

The composition or the medicament or the formulation of the present disclosure may further comprise a suitable pharmaceutical supplementary material, such as a pharmaceutical carrier and a pharmaceutical excipient, including a buffer, known in the art. As used herein, the "pharmaceutical carrier" includes any and all solvents, dispersion media, isotonic agents, absorption delaying agents, and the like that are physiologically compatible.

For the use and purposes of pharmaceutical supplementary materials, see Handbook of Pharmaceutical Excipients, 8th ed., R. C. Rowe, P. J. Seskey, and S. C. Owen, Pharmaceutical Press, London, Chicago.

The composition or the medicament or the formulation of the present disclosure may be in a variety of forms. These forms include, for example, liquid, semi-solid, and solid dosage forms, such as liquid solutions (e.g., injectable solutions or eye drops), pulvis or suspensions, liposomal formulations, and suppositories. The preferred form depends on the intended mode of administration and therapeutic use.

The medicament or the formulation comprising the anti-GDF15 antibody or the antigen-binding fragment thereof described herein may be prepared by mixing the anti-GDF15 antibody or the antigen-binding fragment thereof or the immunoconjugate of the present disclosure having a desired purity with one or more optional pharmaceutical supplementary materials, e.g., in the form of a lyophilized formulation or an aqueous solution.

The composition or the medicament or the formulation of the present disclosure may further comprise more than one active ingredient required by a particular indication treated, preferably those having complementary activity without adversely affecting one another. For example, it may be desirable to further provide an additional therapeutic agent.

The present disclosure further provides a pharmaceutical combination or a pharmaceutical combination product comprising the anti-GDF15 antibody or the antigen-binding fragment thereof or the immunoconjugate of the present disclosure, and one or more additional therapeutic agents.

The present disclosure further provides a kit of parts, comprising the pharmaceutical combination, wherein, for example, the kit of parts comprises, in the same package:
- a first container containing a pharmaceutical composition comprising the anti-GDF15 antibody or the antigen-binding fragment thereof or the immunoconjugate of the present disclosure; and
- a second container containing a pharmaceutical composition comprising an additional therapeutic agent.

In some embodiments, the additional therapeutic agent is, for example, a chemotherapeutic agent, a cytokine, a cytotoxic agent, an additional antibody, a small molecule drug, or an immunomodulatory agent (e.g., an immunosuppressant).

### VII. Use of Anti-GDF15 Antibody or Antigen-Binding Fragment Thereof and Method for Using Same

In one aspect, the present disclosure relates to the anti-GDF15 antibody or the antigen-binding fragment thereof (as well as the immunoconjugate, the composition, the pharmaceutical composition, the formulation, the combination product, etc. comprising same) of the present disclosure for use in a therapy.

In some embodiments, the present disclosure relates to the anti-GDF15 antibody or the antigen-binding fragment thereof (as well as the immunoconjugate, the composition, the pharmaceutical composition, the formulation, the combination product, etc. comprising same) of the present disclosure for use as a medicament.

In another aspect, the present disclosure provides a method for preventing or treating a disease or condition in an individual, comprising administering to the individual the anti-GDF15 antibody or the antigen-binding fragment thereof, or the immunoconjugate, the composition, the medicament, or the formulation comprising same of the present disclosure.

In some embodiments, the condition is cachexia.

The term "cachexia" as used herein includes metabolic disorders and comorbidities that occur in association with (or are induced by) various diseases (e.g., chronic diseases), or metabolic disorders or comorbidities induced by certain therapies. In some embodiments, the disease is selected from a tumor, chronic heart failure, congestive heart failure, sarcopenia, chronic obstructive pulmonary disease (COPD), sarcopenia, and chronic kidney disease (CKD). In some embodiments, the tumor is a cancer. In some embodiments, the therapy is, for example, chemotherapy or chemotherapy in combination with immuno-oncology therapy. In some embodiments, the chemotherapy comprises administering a chemotherapeutic agent, e.g., cisplatin, to the patient.

In some embodiments, the disease is a tumor. In some embodiments, the disease is a cancer. In some embodiments, the cancer may be at an early, intermediate, or advanced stage, or may be a metastatic cancer. In some embodiments, the tumor may be a solid tumor or a hematological tumor, e.g., a malignant solid tumor or hematological tumor. In some embodiments, the cancer is fibrosarcoma, prostate cancer, pancreatic cancer, endometrial cancer, or colorectal cancer.

In some embodiments, a sample from the individual with cachexia has GDF15, for example, has or exhibits elevated levels of GDF15 protein and/or nucleic acids, e.g., compared to the GDF15 protein and/or nucleic acid levels in the same tissue of a healthy individual. In some embodiments, the sample is a body fluid, such as blood, serum, or plasma.

In some embodiments, the cancer is a GDF15-positive cancer. In some embodiments, the cancer is characterized by the presence of, or elevated levels of GDF15 protein and/or nucleic acids in a patient with the cancer (e.g., in the patient's cancer tissue or cells) (e.g., compared to the GDF15 protein and/or nucleic acid levels in the same tissue of a healthy individual, or compared to the GDF15 protein and/or nucleic acid levels in the adjacent healthy tissue of the patient). For example, the tumor cells of the cancer have or exhibit elevated levels of GDF15 protein and/or nucleic acids (e.g., compared to the GDF15 protein and/or nucleic acid levels in the cells of the same tissue of a healthy individual, or compared to the GDF15 protein and/or nucleic acid levels in the healthy cells of the same tissue or in the cells of the adjacent healthy tissue of the patient).

The anti-GDF15 antibody or the antigen-binding fragment thereof of the present disclosure (as well as the immunoconjugate, the composition, the pharmaceutical composition, the formulation, the combination product, etc. comprising same) may be administered by any suitable method, including parenteral administration, intrapulmonary administration, intranasal administration, and, if required by locoregional treatment, intralesional administration. Parenteral injection or infusion includes intramuscular, intravenous, intra-arterial, intraperitoneal, or infusion administration. Depending to some extent on whether the medication is short-term or long-term, the administration may be performed via any suitable route, e.g., by injection, such as intravenous injection. Various administration schedules are encompassed herein, including, but not limited to, single administration or multiple administrations at multiple time points, bolus injection, and pulse infusion.

For the prevention or treatment of a disease or condition, the appropriate dose of the anti-GDF15 antibody or the antigen-binding fragment thereof (as well as the immunoconjugate, the composition, the pharmaceutical composition, the formulation, the combination product, etc. comprising same) of the present disclosure (when used alone or in combination with one or more additional therapeutic agents) will depend on the type of the disease to be treated, the type of the antibody, the severity and progression of the disease, purpose of administration (prophylactic or therapeutic), previous therapies, the patient's clinical history and response to the antibody, and the discretion of an attending physician. The antibody is suitably administered to the patient through a single treatment or through a series of treatments.

In other aspects, the present disclosure provides the anti-GDF15 antibody or the antigen-binding fragment thereof, or the immunoconjugate, the composition, or the combination product comprising same of the present disclosure for the use described herein, e.g., for use in preventing or treating the related disease or condition mentioned herein.

In other aspects, the present disclosure provides use of the anti-GDF15 antibody or the antigen-binding fragment thereof, or the immunoconjugate, the composition, or the combination product comprising same of the present disclosure in the manufacture or preparation of a medicament for the use described herein, e.g., for use in preventing or treating the related disease or disorder mentioned herein.

In some embodiments, the anti-GDF15 antibody or the antigen-binding fragment thereof (as well as the immunoconjugate, the composition, the pharmaceutical composition, the formulation, etc. comprising same) may also be administered in combination with one or more additional therapies such as therapeutic modalities and/or additional therapeutic agents, for the use described herein, e.g., for use in preventing and/or treating the related disease or disorder mentioned herein.

In some embodiments, the therapeutic modality is, for example, surgery or radiotherapy.

In some embodiments, the additional therapeutic agent is, for example, a drug effective in a cancer, such as a chemotherapeutic agent, a cytokine, a cytotoxic agent, an additional antibody, a small molecule drug, or an immunomodulatory agent (e.g., an immunosuppressant).

### VIII. Diagnosis and Detection

In one aspect, the present disclosure further relates to a method for diagnosis and detection (e.g., for diagnostic or non-diagnostic purposes) using the anti-GDF15 antibody or the antigen-binding fragment thereof, and a composition comprising same for diagnosis and detection.

In certain embodiments, the anti-GDF15 antibody provided herein may be used to detect the presence of GDF15 in a biological sample.

The term "detection" as used herein includes quantitative and qualitative detections, and exemplary detection methods may involve immunohistochemistry, immunocytochemistry, flow cytometry (e.g., FACS), magnetic beads complexed with antibody molecules, ELISA, and PCR techniques (e.g., RT-PCR). In certain embodiments, the biological sample is a body fluid, such as blood, serum, or plasma.

In certain embodiments, the method comprises contacting a biological sample with the anti-GDF15 antibody or the antigen-binding fragment thereof described herein under a condition that allows the binding thereof to GDF15, and detecting whether a complex is formed by the anti-GDF15 antibody or the antigen-binding fragment thereof and GDF15. The formation of the complex indicates the presence of GDF15. The method may be an *in vitro* or *in vivo* method.

In certain embodiments, provided is a labeled anti-GDF15 antibody or antigen-binding fragment thereof. The label includes, but is not limited to, a label or moiety that is detected directly (such as a fluorescent label, a chromophoric label, an electron-dense label, a chemiluminescent label, and a radioactive label), and a moiety that is detected indirectly (e.g., through an enzymatic reaction or a molecular interaction), such as an enzyme or a ligand. In some embodiments, the label is, for example, a marker such as biotin or hFc.

In some embodiments provided herein, the sample is obtained prior to treatment with the anti-GDF15 antibody or the antigen-binding fragment thereof of the present disclosure. In some embodiments, the sample is obtained prior to other therapies. In some embodiments, the sample is obtained during treatment with other therapies, or after treatment with other therapies.

In some embodiments, GDF15 is detected prior to a treatment, for example, prior to an initial treatment or prior to a certain treatment after a treatment interval.

In some specific embodiments, the present disclosure relates to:
1. An anti-GDF15 antibody or an antigen-binding fragment thereof, comprising
   (i). three complementarity determining regions HCDR1, HCDR2, and HCDR3 contained in the VH set forth in SEQ ID NO: 7, and three complementarity determining regions LCDR1, LCDR2, and LCDR3 contained in the VL set forth in SEQ ID NO: 8;
   (ii). three complementarity determining regions HCDR1, HCDR2, and HCDR3 contained in the VH set forth in SEQ ID NO: 14, and three complementarity determining regions LCDR1, LCDR2, and LCDR3 contained in the VL set forth in SEQ ID NO: 15;
   (iii).three complementarity determining regions HCDR1, HCDR2, and HCDR3 contained in the VH set forth in SEQ ID NO: 23, and three complementarity determining regions LCDR1, LCDR2, and LCDR3 contained in the VL set forth in SEQ ID NO: 24; or
   (iv). three complementarity determining regions HCDR1, HCDR2, and HCDR3 contained in the VH set forth in SEQ ID NO: 25, and three complementarity determining regions LCDR1, LCDR2, and LCDR3 contained in the VL set forth in SEQ ID NO: 26.
2. An anti-GDF15 antibody or an antigen-binding fragment thereof, comprising an HCDR1, an HCDR2, and an HCDR3, and an LCDR1, an LCDR2, and an LCDR3, wherein
   (i). the HCDR1 comprises or consists of the amino acid sequence of SEQ ID NO: 1; the HCDR2 comprises or consists of the amino acid sequence of SEQ ID NO: 2; the HCDR3 comprises or consists of the amino acid sequence of SEQ ID NO: 3; the LCDR1 comprises or consists of the amino acid sequence of SEQ ID NO: 4; the LCDR2 comprises or consists of the amino acid sequence of SEQ ID NO: 5; and/or the LCDR3 comprises or consists of the amino acid sequence of SEQ ID NO: 6;
   (ii). the HCDR1 comprises or consists of the amino acid sequence of SEQ ID NO: 1; the HCDR2 comprises or consists of the amino acid sequence of SEQ ID NO: 9; the HCDR3 comprises or consists of the amino acid sequence of SEQ ID NO: 10; the LCDR1 comprises or consists of the amino acid sequence of SEQ ID NO: 11; the LCDR2 comprises or consists of the amino acid sequence of SEQ ID NO: 12; and/or the LCDR3 comprises or consists of the amino acid sequence of SEQ ID NO: 13; or
   (iii).the HCDR1 comprises or consists of the amino acid sequence of SEQ ID NO: 1; the HCDR2 comprises or consists of the amino acid sequence of SEQ ID NO: 20; the HCDR3 comprises or consists of the amino acid sequence of SEQ ID NO: 3; the LCDR1 comprises or consists of the amino acid sequence of SEQ ID NO: 21; the LCDR2 comprises or consists of the amino acid sequence of SEQ ID NO: 22; and/or the LCDR3 comprises or consists of the amino acid sequence of SEQ ID NO: 6.
3. The antibody or the antigen-binding fragment thereof according to embodiment 1 or 2, comprising a heavy chain variable region VH, wherein the heavy chain variable region
   (i) comprises or consists of an amino acid sequence having at least 90%, 91%, 92%, 93%, 94%, 95%, 96%, 97%, 98%, or 99% identity to the amino acid sequence of SEQ ID NO: 7, 14, 23, or 25; or
   (ii) comprises or consists of the amino acid sequence of SEQ ID NO: 7, 14, 23, or 25.
4. The antibody or the antigen-binding fragment thereof according to any one of embodiments 1-3, comprising a light chain variable region VL, wherein the light chain variable region
   (i) comprises or consists of an amino acid sequence having at least 90%, 91%, 92%, 93%, 94%, 95%, 96%, 97%, 98%, or 99% identity to the amino acid sequence of SEQ ID NO: 8, 15, 24, or 26; or
   (ii) comprises or consists of the amino acid sequence of SEQ ID NO: 8, 15, 24, or 26.
5. The antibody or the antigen-binding fragment thereof according to embodiment 1 or 2, comprising a heavy chain variable region VH and a light chain variable region VL, wherein
   (i). the VH comprises or consists of the amino acid sequence set forth in SEQ ID NO: 7 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 8 or an amino acid sequence having at least 90%, 91%, 92%, 93%, 94%, 95%, 96%, 97%, 98%, or 99% identity thereto;
   (ii). the VH comprises or consists of the amino acid sequence set forth in SEQ ID NO: 14 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 15 or an amino acid sequence having at least 90%, 91%, 92%, 93%, 94%, 95%, 96%, 97%, 98%, or 99% identity thereto;
   (iii).the VH comprises or consists of the amino acid sequence set forth in SEQ ID NO: 23 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 24 or an amino acid sequence having at least 90%, 91%, 92%, 93%, 94%, 95%, 96%, 97%, 98%, or 99% identity thereto; or
   (iv). the VH comprises or consists of the amino acid sequence set forth in SEQ ID NO: 25 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 26 or an amino acid sequence having at least 90%, 91%, 92%, 93%, 94%, 95%, 96%, 97%, 98%, or 99% identity thereto.
6. The antibody or the antigen-binding fragment thereof according to embodiment 1 or 2, comprising a heavy chain variable region VH and a light chain variable region VL, wherein
   (i). the VH comprises or consists of the amino acid sequence set forth in SEQ ID NO: 7, and the VL comprises or consists of the amino acid sequence set forth in SEQ ID NO: 8;
   (ii). the VH comprises or consists of the amino acid sequence set forth in SEQ ID NO: 14, and the VL comprises or consists of the amino acid sequence set forth in SEQ ID NO: 15;
   (iii).the VH comprises or consists of the amino acid sequence set forth in SEQ ID NO: 23, and the VL comprises or consists of the amino acid sequence set forth in SEQ ID NO: 24; or
   (iv). the VH comprises or consists of the amino acid sequence set forth in SEQ ID NO: 25, and the VL comprises or consists of the amino acid sequence set forth in SEQ ID NO: 26.
7. The antibody or the antigen-binding fragment thereof according to any one of embodiments 1-6, further comprising a heavy chain constant region HC, wherein, for example, the heavy chain constant region HC of the antibody is a heavy chain constant region of IgG1, IgG2, IgG3, or IgG4, preferably a heavy chain constant region of IgG1; optionally, the heavy chain constant region has an Fc region amino acid modification(s), e.g., a modification(s) that reduces or eliminates an effector function, a modification(s) that increases the affinity for FcRn, a modification(s) that prolongs the half-life, etc.; preferably, the heavy chain constant region has one or more of L234A/L235A mutations, a G237A mutation, and M252Y/S254T/T256E mutations; more preferably, the heavy chain constant region has L234A/L235A mutations, a G237A mutation, and M252Y/S254T/T256E mutations.
8. The antibody or the antigen-binding fragment thereof according to embodiment 7, wherein the heavy chain constant region
   (i) comprises or consists of an amino acid sequence having at least 85%, 90%, 91%, 92%, 93%, 94%, 95%, 96%, 97%, 98%, or 99% identity to the amino acid sequence of SEQ ID NO: 44 or 48; or
   (ii) comprises or consists of the amino acid sequence of SEQ ID NO: 44 or 48.
9. The antibody or the antigen-binding fragment thereof according to any one of embodiments 1-8, comprising a light chain constant region, wherein, for example, the light chain constant region is a lambda or kappa light chain constant region.
10. The antibody or the antigen-binding fragment thereof according to embodiment 9, wherein the light chain constant region
   (i) comprises or consists of an amino acid sequence having at least 85%, 90%, 91%, 92%, 93%, 94%, 95%, 96%, 97%, 98%, or 99% identity to the amino acid sequence of SEQ ID NO: 45; or
   (ii) comprises or consists of the amino acid sequence of SEQ ID NO: 45.
11. The antibody or the antigen-binding fragment thereof according to any one of embodiments 1-10, wherein the antibody comprises a heavy chain; the heavy chain
   (i) comprises or consists of an amino acid sequence having at least 90%, 91%, 92%, 93%, 94%, 95%, 96%, 97%, 98%, or 99% identity to the amino acid sequence of SEQ ID NO: 28, 30, 33, 35, or 49; or
   (ii) comprises or consists of the amino acid sequence of SEQ ID NO: 28, 30, 33, 35, or 49.
12. The antibody or the antigen-binding fragment thereof according to any one of embodiments 1-11, wherein the antibody comprises a light chain; the light chain
   (i) comprises or consists of an amino acid sequence having at least 90%, 91%, 92%, 93%, 94%, 95%, 96%, 97%, 98%, or 99% identity to the amino acid sequence of SEQ ID NO: 29, 31, 34, or 36; or
   (ii) comprises or consists of the amino acid sequence of SEQ ID NO: 29, 31, 34, or 36.
13. The antibody or the antigen-binding fragment thereof according to any one of embodiments 1-12, comprising a heavy chain and a light chain, wherein
   (i). the heavy chain comprises or consists of the amino acid sequence set forth in SEQ ID NO: 28 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 29 or an amino acid sequence having at least 90%, 91%, 92%, 93%, 94%, 95%, 96%, 97%, 98%, or 99% identity thereto;
   (ii). the heavy chain comprises or consists of the amino acid sequence set forth in SEQ ID NO: 30 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 31 or an amino acid sequence having at least 90%, 91%, 92%, 93%, 94%, 95%, 96%, 97%, 98%, or 99% identity thereto;
   (iii).the heavy chain comprises or consists of the amino acid sequence set forth in SEQ ID NO: 33 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 34 or an amino acid sequence having at least 90%, 91%, 92%, 93%, 94%, 95%, 96%, 97%, 98%, or 99% identity thereto;
   (iv). the heavy chain comprises or consists of the amino acid sequence set forth in SEQ ID NO: 35 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 36 or an amino acid sequence having at least 90%, 91%, 92%, 93%, 94%, 95%, 96%, 97%, 98%, or 99% identity thereto;
   (v). the heavy chain comprises or consists of the amino acid sequence set forth in SEQ ID NO: 49 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 34 or an amino acid sequence having at least 90%, 91%, 92%, 93%, 94%, 95%, 96%, 97%, 98%, or 99% identity thereto.
14. The antibody or the antigen-binding fragment thereof according to any one of embodiments 1-13, comprising a heavy chain and a light chain, wherein
   (i). the heavy chain comprises or consists of the amino acid sequence set forth in SEQ ID NO: 28, and the light chain comprises or consists of the amino acid sequence set forth in SEQ ID NO: 29;
   (ii). the heavy chain comprises or consists of the amino acid sequence set forth in SEQ ID NO: 30, and the light chain comprises or consists of the amino acid sequence set forth in SEQ ID NO: 31;
   (iii).the heavy chain comprises or consists of the amino acid sequence set forth in SEQ ID NO: 33, and the light chain comprises or consists of the amino acid sequence set forth in SEQ ID NO: 34;
   (iv). the heavy chain comprises or consists of the amino acid sequence set forth in SEQ ID NO: 35, and the light chain comprises or consists of the amino acid sequence set forth in SEQ ID NO: 36; or
   (v). the heavy chain comprises or consists of the amino acid sequence set forth in SEQ ID NO: 49, and the light chain comprises or consists of the amino acid sequence set forth in SEQ ID NO: 34.
15. The antibody or the antigen-binding fragment thereof according to any one of embodiments 1-14, wherein the antibody is a chimeric antibody or a humanized antibody.
16. The antibody or the antigen-binding fragment thereof according to any one of embodiments 1-15, wherein the antibody is a monoclonal antibody.
17. The antibody or the antigen-binding fragment thereof according to any one of embodiments 1-16, wherein the antigen-binding fragment is an antibody fragment selected from: a Fab, a Fab', a Fab'-SH, an Fv, a single-chain antibody, a (Fab')₂, a single-domain antibody such as a heavy chain variable region VHH, a domain antibody (dAb), a heavy-chain antibody, and a linear antibody.
18. The antibody or the antigen-binding fragment thereof according to embodiment 17, wherein the single-chain antibody is an scFv.
19. A VHH antibody specifically binding to GDF15, comprising three complementarity determining regions (CDRs) contained in a VHH set forth in any one of SEQ ID NOs: 19 and 27, wherein preferably, the sequences of the CDRs are defined according to IMGT.
20. The VHH antibody according to embodiment 19, comprising complementarity determining regions (CDRs) VHH CDR1, VHH CDR2, and VHH CDR3, wherein
   the VHH CDR1 comprises or consists of the amino acid sequence set forth in SEQ ID NO: 16, the VHH CDR2 comprises or consists of the amino acid sequence set forth in SEQ ID NO: 17, and the VHH CDR3 comprises or consists of the amino acid sequence set forth in SEQ ID NO: 18.
21. The VHH antibody according to embodiment 19, comprising or consisting of a heavy chain variable region, wherein the heavy chain variable region
   (i) comprises or consists of an amino acid sequence having at least 90%, 91%, 92%, 93%, 94%, 95%, 96%, 97%, 98%, or 99% identity to an amino acid sequence selected from any one of SEQ ID NOs: 19 and 27; or
   (ii) comprises or consists of an amino acid sequence selected from any one of SEQ ID NOs: 19 and 27.
22. A heavy-chain antibody specifically binding to GDF15, comprising the VHH antibody according to any one of embodiments 19-21.
23. The heavy-chain antibody according to embodiment 22, comprising the VHH antibody according to any one of embodiments 19-21 linked to an antibody constant region or Fc region, wherein preferably, the antibody constant region or Fc region is from human IgG1, human IgG2, human IgG3, or human IgG4, and preferably, the Fc region
   (i) comprises an amino acid sequence having at least 85%, 90%, 91%, 92%, 93%, 94%, 95%, 96%, 97%, 98%, or 99% identity to the amino acid sequence set forth in SEQ ID NO: 46; or
   (ii) comprises or consists of the amino acid sequence set forth in SEQ ID NO: 46.
24. The heavy-chain antibody according to embodiment 22, comprising the VHH antibody according to any one of embodiments 1-3 linked to an antibody Fc region, wherein the Fc region is an Fc region from human IgG1, IgG2, IgG3, or IgG4, optionally, the Fc region comprises L234A/L235A mutations and a G237A mutation, and preferably, the Fc region
   (i) comprises an amino acid sequence having at least 85%, 90%, 91%, 92%, 93%, 94%, 95%, 96%, 97%, 98%, or 99% identity to the amino acid sequence set forth in SEQ ID NO: 43; or
   (ii) comprises or consists of the amino acid sequence set forth in SEQ ID NO: 43.
25. The heavy-chain antibody according to embodiment 22, wherein the heavy-chain antibody
   (i) comprises or consists of an amino acid sequence having at least 85%, 90%, 91%, 92%, 93%, 94%, 95%, 96%, 97%, 98%, or 99% identity to an amino acid sequence selected from SEQ ID NOs: 32 and 37; or
   (ii) comprises or consists of an amino acid sequence selected from SEQ ID NOs: 32 and 37; or
   (iii) comprises an amino acid sequence having one or more (preferably no more than 10, and more preferably no more than 5, 4, 3, 2, or 1) amino acid modifications (preferably amino acid substitutions, and more preferably conservative amino acid substitutions) compared to an amino acid sequence selected from SEQ ID NOs: 32 and 37, wherein preferably, the amino acid modifications do not occur in the CDRs.
26. The VHH antibody according to any one of embodiments 19-21 or the heavy-chain antibody according to any one of embodiments 22-25, wherein the antibody is a chimeric antibody or a humanized antibody.
27. A multispecific antibody, e.g., a bispecific antibody, comprising the antibody or the antigen-binding fragment thereof according to any one of embodiments 1-18, the VHH antibody according to any one of embodiments 19-21, or the heavy-chain antibody according to any one of embodiments 22-26.
28. A nucleic acid molecule, wherein the nucleic acid molecule encodes any one of the chains in the antibody or the antigen-binding fragment thereof according to any one of embodiments 1-18, the VHH antibody according to any one of embodiments 19-21, the heavy-chain antibody according to any one of embodiments 22-26, or the multispecific antibody according to embodiment 27, or consists of the nucleic acid sequence.
29. An expression vector, comprising the nucleic acid molecule according to embodiment 28, wherein preferably, the expression vector is pCDNA, e.g., pCDNA3.1.
30. A host cell, comprising the nucleic acid molecule according to embodiment 28 or the expression vector according to embodiment 29, wherein preferably, the host cell is prokaryotic or eukaryotic, e.g., a 293 cell or a CHO cell.
31. A method for preparing the antibody or the antigen-binding fragment thereof according to any one of embodiments 1-18, the VHH antibody according to any one of embodiments 19-21, the heavy-chain antibody according to any one of embodiments 22-26, or the multispecific antibody according to embodiment 27, comprising: culturing a host cell comprising the nucleic acid molecule according to embodiment 28 or the expression vector according to embodiment 29 or the host cell according to embodiment 30 under a condition suitable for expressing the chain(s) of the antibody, and optionally recovering the antibody from the host cell (or a host cell culture medium).
32. An antibody or an antigen-binding fragment thereof or a multispecific binding molecule prepared by the method according to embodiment 31.
33. An immunoconjugate, comprising the antibody or the antigen-binding fragment thereof according to any one of embodiments 1-18, the VHH antibody according to any one of embodiments 19-21, the heavy-chain antibody according to any one of embodiments 22-26, or the multispecific antibody according to embodiment 27, wherein, for example, the immunoconjugate is an ADC.
34. A pharmaceutical composition or a medicament or a formulation, comprising the antibody or the antigen-binding fragment thereof according to any one of embodiments 1-18, the VHH antibody according to any one of embodiments 19-21, the heavy-chain antibody according to any one of embodiments 22-26, the multispecific antibody according to embodiment 27, or the immunoconjugate according to embodiment 33, and optionally a pharmaceutical supplementary material.
35. A pharmaceutical combination product, comprising the antibody or the antigen-binding fragment thereof according to any one of embodiments 1-18, the VHH antibody according to any one of embodiments 19-21, the heavy-chain antibody according to any one of embodiments 22-26, the multispecific antibody according to embodiment 27, or the immunoconjugate according to embodiment 33, and one or more additional therapeutic agents (e.g., a chemotherapeutic agent, a cytokine, a cytotoxic agent, an additional antibody, a small molecule drug, or an immunomodulatory agent).
36. A method for preventing or treating cachexia in a subject, comprising administering to the individual an effective amount of the antibody or the antigen-binding fragment thereof according to any one of embodiments 1-18, the VHH antibody according to any one of embodiments 19-21, the heavy-chain antibody according to any one of embodiments 22-26, the multispecific antibody according to embodiment 27, the immunoconjugate according to embodiment 33, the pharmaceutical composition or the formulation according to embodiment 34, or the pharmaceutical combination product according to embodiment 35.
37. The method according to embodiment 36, wherein the cachexia occurs in association with a disease selected from a tumor such as a cancer, chronic heart failure, congestive heart failure, sarcopenia, chronic obstructive pulmonary disease (COPD), sarcopenia, and chronic kidney disease (CKD); for example, the tumor is a solid tumor or a hematological tumor, e.g., a malignant solid tumor or hematological tumor, such as fibrosarcoma, prostate cancer, pancreatic cancer, endometrial cancer, or colorectal cancer.
38. The method according to embodiment 36, wherein the cachexia is induced by chemotherapy or chemotherapy in combination with immuno-oncology therapy; for example, the chemotherapy comprises administering a chemotherapeutic agent, e.g., cisplatin.
39. The method according to any one of embodiments 36-38, wherein a sample from the individual has GDF15, for example, has or exhibits elevated levels of GDF15 protein and/or nucleic acids, e.g., compared to GDF15 protein and/or nucleic acid levels in the same tissue of a healthy individual; for example, the sample is a body fluid, such as blood, serum, or plasma.
40. The method according to any one of embodiments 36-39, further comprising administering in combination with an additional therapy such as a therapeutic modality (e.g., surgery or radiotherapy) and/or an additional therapeutic agent (e.g., a chemotherapeutic agent, a cytokine, a cytotoxic agent, an additional antibody, a small molecule drug, or an immunomodulatory agent (e.g., an immune checkpoint molecule inhibitor or agonist)).
41. A method for detecting the presence of GDF15 in a biological sample, comprising
   (i) contacting the biological sample with the antibody or the antigen-binding fragment thereof according to any one of embodiments 1-18, the VHH antibody according to any one of embodiments 19-21, the heavy-chain antibody according to any one of embodiments 22-26, the multispecific antibody according to embodiment 27, or the immunoconjugate according to embodiment 33 under a condition that allows the binding thereof to GDF15, and
   (ii) detecting whether a complex is formed by the antibody or the multispecific binding molecule and GDF15,
   wherein the formation of the complex indicates the presence of GDF15.

### Examples

### Example 1: Obtaining Murine Antibodies Through Mouse Immunization and Hybridoma Generation

### 1.1 Animal immunization

Five BALB/c mice (Nanjing Probio Biotech) were immunized with Human GDF15 protein (Kactus Biosystems, Cat. No. GDF-HE115) once every two weeks. Starting from the second immunization, the mice were subjected to venous blood collection 7 days after each immunization. The mouse immune sera were subjected to enzyme-linked immunosorbent assay (ELISA) using Human GDF15 protein (Kactus Biosystems, Cat. No. GDF-HE115), Cynomolgus GDF15 protein (Kactus Biosystems, Cat. No. GDF-CE115), and Mouse GDF15 protein (Kactus Biosystems, Cat. No. GDF-ME115). Two mice were selected for animal fusion. Four days after the final booster immunization, mouse spleens were taken, and lymphocytes isolated from the spleens were used for fusion to generate hybridomas.

### 1.2 Hybridoma screening

Hybridoma culture supernatants were subjected to a primary screening test by ELISA. In this assay, 30 clones that showed binding to the Human GDF15 protein, Cynomolgus GDF15 protein, and Mouse GDF15 protein were selected for subcloning.

### 1.3 Subcloning

The selected hybridoma cell lines (parental clones) were subcloned to ensure the monoclonality. Subcloning was performed by replating the parental clones using a single-step cloning system. The 30 subclones were transferred to a 96-well culture plate. The subclones were screened by the ELISA method for antibodies that simultaneously bind to the Human GDF15 protein, Cynomolgus GDF15 protein, and Mouse GDF15 protein.

### 1.4 Hybridoma sequencing

The hybridomas selected in the previous step were expanded in culture, and a portion of the cells were cryopreserved for sequencing. The variable region amino acid sequences of the antibodies obtained are shown in Table 1 below, where the CDRs are determined using Kabat.

| Antibody name | Chi-19C4C7 | Chi-21H6D2 |
|---|---|---|
| HCDR1 | SEQ ID NO:1 | SEQ ID NO:1 |
| HCDR2 | SEQ ID NO:2 | SEQ ID NO:9 |
| HCDR3 | SEQ ID NO:3 | SEQ ID NO:10 |
| LCDR1 | SEQ ID NO:4 | SEQ ID NO:11 |
| LCDR2 | SEQ ID NO:5 | SEQ ID NO:12 |
| LCDR3 | SEQ ID NO:6 | SEQ ID NO:13 |
| VH | SEQ ID NO:7 | SEQ ID NO:14 |
| VL | SEQ ID NO:8 | SEQ ID NO:15 |

### Example 2: Production of Anti-GDF15 Antibodies Through Alpaca Immunization

### 2.1 Animal immunization

Two alpacas (Chengdu NBbiolab, Co., Ltd.) were immunized with Human GDF15 protein (Kactus Biosystems, Cat. No. GDF-HE115). The alpacas were immunized with four injections administered at 21-day intervals (Table 1). The alpacas were subjected to venous blood collection on day 10 after the second immunization and day 10 after the third booster immunization. The alpaca immune sera were subjected to ELISA using the recombinant human GDF15 protein. One alpaca was selected for blood collection and library construction.

**Table 2. Alpaca immunization procedure**

| Step | Route of administration | Dose |
|---|---|---|
| Primary Immunization | Subcutaneous injection | 0.5 mg protein/alpaca |
| 1st Boost | Subcutaneous injection | 0.25 mg protein/alpaca |
| 2nd Boost | Subcutaneous injection | 0.25 mg protein/alpaca |
| 3nd Boost | Subcutaneous injection | 0.25 mg protein/alpaca |
| Final Boost | Subcutaneous injection | 0.25 mg protein/alpaca |

### 2.2 Alpaca immune library construction and screening

Total RNA was extracted from alpaca peripheral blood using the Trizol method, and reverse transcription was performed using a PrimeScript II 1st Strand cDNA Synthesis Kit (Takara, Cat. No. 6210A) to obtain cDNA. The cDNA was subjected to nested PCR amplification, and 750 bp products containing nanobodies were recovered. A second round of PCR was then performed to amplify and purify the VHHs. The VHHs were ligated into a phage vector using the enzymatic cleavage ligation technique, followed by electroporation, transformation, and plating. The next day, the library capacity was determined, and the constructed library was found to have a library capacity of 2 × 10e8 CFU, with an insertion rate of 100%. Two rounds of solid-phase screening were performed using the recombinant human GDF15 protein, and the positive clones obtained were sequenced and identified. Partial amino acid sequences obtained are shown in Table 3, where the CDRs were determined using IMGT.

**Table 3. Amino acid sequences of camelid-derived VHH (anti-GDF15 antibody)**

| Antibody name | Chi-VHH43 |
|---|---|
| HCDR1 | SEQ ID NO:16 |
| HCDR2 | SEQ ID NO:17 |
| HCDR3 | SEQ ID NO:18 |
| VHH | SEQ ID NO:19 |
| VHH-IgG Fc mut | SEQ ID NO:32 |

### Example 3: Synthesis and Expression of Anti-GDF15 Chimeric Antibodies

### 3.1 Synthesis of anti-GDF15 chimeric antibodies

The variable region amino acid sequences of 2 murine antibodies from Table 1 and 1 camelid-derived antibody from Table 3 were sent to TONGYONG Biotech Co., Ltd. (Anhui) for codon optimization and gene synthesis. The genes encoding the VH and VL regions of the antibodies were inserted sequentially into an expression vector containing a human IgG1 heavy chain constant region (SEQ ID NO: 44, with L234A, L235A, and G237A) and a kappa light chain constant region (SEQ ID NO: 45) to obtain murine chimeric antibodies.

The sequence of the control antibody Ponsegromab was derived from the patent CN112912395A.

The amino acid sequence of the heavy chain of the control antibody Ponsegromab is as follows: (SEQ ID NO: 38).

The amino acid sequence of the light chain of the control antibody Ponsegromab is as follows: (SEQ ID NO: 39).

### 3.2 Expression and purification of anti-GDF15 chimeric antibodies

Plasmids encoding the heavy and light chains of the anti-GDF15 antibodies were co-transfected into ExpiCHO-S cells to express the murine chimeric antibodies as described in Table 4 and the control antibody Ponsegromab, and then corresponding purification was performed. The plasmids encoding the heavy and light chains of the anti-GDF15 antibodies described in Table 4 were co-transfected into ExpiCHO-S cells using an ExpiCHO^{™} expression system (ThermoFisher, Cat# A29133) according to the manufacturer's product manual instructions to express the anti-GDF15 chimeric antibodies, and the control antibody Ponsegromab was similarly expressed. The cells were cultured for 10-12 days after transfection. When the cell viability decreased to 60%-70%, the supernatants were collected, and the antibodies expressed in the supernatants were purified using a MabSelect Sure protein A affinity chromatography system (GE Healthcare). The purified antibodies were concentrated and subjected to sterile filtration. The purity of the antibody proteins was detected by SDS-PAGE and size exclusion chromatography. The results indicated that the purity of the antibodies was greater than 90%, and thus the antibodies could be used in the next step.

**Table 4. Murine chimeric antibody names and combinations of corresponding heavy and light chains**

| Chimeric antibody name | Heavy chain | Light chain |
|---|---|---|
| Chi-19C4C7 | 19C4C7-HC (SEQ ID NO:28) | 19C4C7-LC (SEQ ID NO:29) |
| Chi-21H6D2 | 21H6D2-HC (SEQ ID NO:30) | 21H6D2-LC (SEQ ID NO:31) |

**Table 5. Camelid-derived chimeric antibody name and corresponding heavy chain**

| Chimeric antibody name | Heavy chain |
|---|---|
| Chi-VHH43 | VHH43-HC (SEQ ID NO:32) |

### Example 4: Humanization of Murine Anti-GDF15 Antibodies and Construction of Humanized Anti-GDF15 Antibodies with YTE Mutations in Fc Region

4.1. The murine antibodies 19C4C7 and 21H6D2 were humanized using CDR grafting technology. The heavy and light chain variable region sequences of 19C4C7 were separately searched and aligned in the IMGT database, thereby obtaining a human germline gene sequence IGHV1-69*02 with high homology to the 19C4C7 heavy chain variable region for use as a humanized framework of the heavy chain variable region, and selecting a human germline gene sequence IGKV3-11*01 with high homology to the light chain variable region for use as a humanized framework of the light chain variable region. The CDRs of the 19C4C7 heavy and light chain variable regions were grafted into the corresponding humanized frameworks to form humanized 19C4C7 antibody variable regions. To maintain the affinity of the antibody 19C4C7, the humanized antibody variable regions obtained needed to be back-mutated. The heavy and light chain variable region sequences of 21H6D2 were separately searched and aligned in the IMGT database, thereby obtaining a human germline gene sequence IGHV1-3*01 with high homology to the 21H6D2 heavy chain variable region for use as a humanized framework of the heavy chain variable region, and selecting a human germline gene sequence IGKV6-21*01 with high homology to the light chain variable region for use as a humanized framework of the light chain variable region. The CDRs of the 21H6D2 heavy and light chain variable regions were grafted into the corresponding humanized frameworks to form humanized 21H6D2 antibody variable regions. To maintain the affinity of the antibody 21H6D2, the humanized antibody variable regions obtained needed to be back-mutated.

Thus, the sequences of the humanized heavy and light chain variable regions for 19C4C7 and 21H6D2 were obtained. The amino acid sequences of the humanized variable regions for the murine antibodies 19C4C7 and 21H6D2 are listed in Table 6. The variable region amino acid sequences were sent to TONGYONG Biotech Co., Ltd. (Anhui) for codon optimization and gene synthesis. The genes encoding the VH and VL regions of the antibodies were inserted sequentially into an expression vector containing a coding gene of a human IgG1 heavy chain constant region (SEQ ID NO: 44, with L234A, L235A and G237A) and a coding gene of a kappa light chain constant region (SEQ ID NO: 45) to obtain plasmids expressing the full-length heavy chains of the humanized anti-GDF15 antibodies and plasmids encoding the full-length light chains. The names, variable region sequences, and heavy and light chain sequences of the antibodies are listed in Table 6.

### 4.2 Construction of humanized anti-GDF15 antibody with YTE mutations in Fc region

Using the constructed sequence containing the human IgG1 heavy chain constant region (SEQ ID NO: 44, with L234A, L235A, and G237A) as a template, site-directed mutagenesis was performed. Mutations M252Y, S254T, and T256E (according to EU numbering) were introduced using the overlapping PCR technique. Then, the genes encoding the VH and VL regions of the antibody Hu19C4C7-YTE were inserted sequentially into an expression vector containing a coding gene of a human IgG1 heavy chain constant region (SEQ ID NO: 48, with L234A, L235A, G237A, M252Y, S254T, and T256E) and a coding gene of a kappa light chain constant region (SEQ ID NO: 45) to obtain a plasmid expressing the full-length heavy chain of the humanized anti-GDF15 antibody and a plasmid encoding the full-length light chain.

**Table 6. Humanized antibody names and SEQ ID NOs of corresponding sequences**

| Antibody name | Hu19C4C7 | Hu21H6D2 | hu19C4C7-YTE |
|---|---|---|---|
| HCDR1 | SEQ ID NO:1 | SEQ ID NO:1 | SEQ ID NO:1 |
| HCDR2 | SEQ ID NO:20 | SEQ ID NO:9 | SEQ ID NO:20 |
| HCDR3 | SEQ ID NO:3 | SEQ ID NO:10 | SEQ ID NO:3 |
| LCDR1 | SEQ ID NO:21 | SEQ ID NO:11 | SEQ ID NO:21 |
| LCDR2 | SEQ ID NO:22 | SEQ ID NO:12 | SEQ ID NO:22 |
| LCDR3 | SEQ ID NO:6 | SEQ ID NO:13 | SEQ ID NO:6 |
| VH | SEQ ID NO:23 | SEQ ID NO:25 | SEQ ID NO:23 |
| VL | SEQ ID NO:24 | SEQ ID NO:26 | SEQ ID NO:24 |
| Heavy chain | SEQ ID NO:33 | SEQ ID NO:35 | SEQ ID NO:49 |
| Light chain | SEQ ID NO:34 | SEQ ID NO:36 | SEQ ID NO:34 |

### Example 5: Humanization of Camelid-Derived Anti-GDF15 Antibody

The camelid-derived antibody VHH43 was humanized using CDR grafting technology. The heavy and light chain variable region sequences of VHH43 were searched and aligned in the IMGT database, thereby obtaining a human germline gene sequence IGHV3-23*04 with high homology to the VHH43 heavy chain variable region for use as a humanized framework of the heavy chain variable region. The CDRs of the VHH43 heavy chain variable region were grafted into the corresponding humanized framework to form a humanized VHH43 antibody variable region. To maintain the affinity of the antibody VHH43-3, the humanized antibody variable regions obtained needed to be back-mutated.

Thus, the sequences of the humanized heavy chain variable region for VHH43 were obtained. The amino acid sequences of the humanized variable region for the camelid-derived antibody VHH43 are listed in Table 7. The variable region amino acid sequences were sent to TONGYONG Biotech Co., Ltd. (Anhui) for codon optimization and gene synthesis. The gene encoding the VHH region of the antibody was inserted into an expression vector containing a coding gene of human IgG1-Fc (with L234A, L235A, and G237A) (SEQ ID NO: 52) to obtain a plasmid expressing the full-length heavy chain of the humanized anti-GDF15 antibody. The name, variable region sequence, and heavy and light chain sequences of the antibody are listed in Table 7, where the HCDRs were determined using IMGT.

**Table 7. Humanized antibody name and SEQ ID NOs of corresponding sequences**

| Antibody name | HuVHH43-3 |
|---|---|
| HCDR1 | SEQ ID NO:16 |
| HCDR2 | SEQ ID NO:17 |
| HCDR3 | SEQ ID NO:18 |
| VHH | SEQ ID NO:27 |
| VHH-IgG Fc mut | SEQ ID NO:37 |

### Example 6: Expression and Purification of Humanized Anti-GDF15 Antibodies

The plasmids encoding the heavy and light chains of the humanized antibodies shown in Table 6 and the heavy chain of the humanized antibody shown in Table 7 were transfected into ExpiCHO-S cells using the method in Example 3.2 to express the humanized anti-GDF15 antibodies, and the corresponding purification was performed as described in Example 3.2. The purified antibodies were concentrated and subjected to sterile filtration. The purity of the antibodies was detected by SDS-PAGE and size exclusion chromatography (SEC).

### Example 7: Binding Affinity of Humanized Anti-GDF15 Antibodies for Human, Cynomolgus Monkey, and Mouse GDF15 Proteins

To evaluate the binding affinity of the humanized anti-GDF15 antibodies for human, cynomolgus monkey, and mouse GDF15, detection was performed using Fortebio.

A running buffer (PBS, 0.1% BSA, 0.02% Tween20, pH 7.0) was added to a pre-wetted plate, and an AHC sensor (Fortebio, Cat. No.: 18-5060) was pre-equilibrated. Before loading, the baseline was equilibrated with the running buffer for 300 s. Coupling of anti-GDF15 antibodies to AHC sensor: The humanized anti-GDF15 antibodies were diluted to 5 µg/mL with the running buffer, and immobilization was performed for 200 s. The baseline was equilibrated with the running buffer for 200 s. Human GDF15 protein (Kactus Biosystems, Cat. No.: GDF-HE115), cynomolgus monkey GDF15 protein (Kactus Biosystems, Cat. No.: GDF-CE115), and mouse GDF15 protein (Beijing ACROBiosystems, Cat. No.: GD5-M5149) were diluted to 200 nM, 100 nM, 50 nM, 25 nM, 12.5 nM, 6.25 nM, and 3.13 nM with the running buffer. Binding was performed for 200 s, and dissociation was performed for 600 s. Regeneration was performed with a regeneration solution for 30 s. The data obtained were fitted to a 1:1 binding model using Octet software, with Local fitting applied for calculation.

Table 10 shows the KD values for the binding of humanized anti-GDF15 antibodies to human, cynomolgus monkey, and mouse GDF15. The humanized anti-GDF15 antibodies showed strong binding to human and cynomolgus monkey GDF15, while also binding to mouse GDF15.

**Table 10. Binding affinity of humanized anti-GDF15 antibodies to human, cynomolgus monkey, and mouse GDF15**

| Sample | Antigen | K_{D}(M) | Kₒₙ(M⁻¹S⁻¹) | K_{off}(S⁻¹) |
|---|---|---|---|---|
| Hu19C4C7 | Human GDF15 | 2.64E-11 | 8.87E+05 | 2.35E-05 |
| | Cynomolgus monkey GDF15 | 3.12E-10 | 2.65E+05 | 8.24E-05 |
| | Mouse GDF15 | 1.49E-08 | 1.62E+05 | 2.41E-03 |
| Hu21H6D2 | Human GDF15 | 5.15E-11 | 9.46E+05 | 4.87E-05 |
| | Cynomolgus monkey GDF15 | 2.31E-10 | 2.65E+05 | 6.13E-05 |
| | Mouse GDF15 | 1.06E-08 | 1.10E+05 | 1.16E-03 |
| HuVHH43-3 | Human GDF15 | <1.0E-12 | 5.73E+05 | <1.0E-07 |
| | Cynomolgus monkey GDF15 | <1.0E-12 | 2.05E+05 | <1.0E-07 |
| | Mouse GDF15 | 1.25E-09 | 5.25E+04 | 6.58E-05 |

### Example 8: Humanized Anti-GDF15 Antibodies and Humanized Anti-GDF15 Antibody with YTE Mutations in Fc Region Do Not Cross-React with Human TGFβ Family Proteins

8.1 To evaluate the binding specificity of the humanized anti-GDF15 antibodies to GDF15, the binding activity of the humanized anti-GDF15 antibodies to human GDF1, GDF2, GDF3, AMH, BMP4, GDF11, GDF8, GDNF, TGFβ1, GFRα1, Activin A, Activin B, and BMP2 was determined using the ELISA method.

A 96-well plate was coated with human GDF1 protein (Wuhan Fine Biotech, Cat. No.: P0298), human GDF2 protein (Kactus Biosystems, Cat. No.: BMP-HM209), human GDF3 protein (Wuhan Fine Biotech, Cat. No.: P0300), human AMH protein (Novoprotein, Cat. No.: DRA15), human BMP4 protein (Kactus Biosystems, Cat. No.: BMP-HE004), human GDF11 protein (Wuhan ABclonal Technology, Cat. No.: RP00378), human GDF8 protein (Beijing ACROBiosystems, Cat. No.: GD8-H5243), human GDNF protein (Kactus Biosystems, Cat. No.: GDF-HE001), human TGFβ1 protein (Beijing ACROBiosystems, Cat. No.: TG1-H4212), human GFRα1 protein (Kactus Biosystems, Cat. No.: GFR-HM11A), human Activin A protein (Kactus Biosystems, Cat. No.: ACV-HM001), human Activin B protein (Novoprotein, Cat. No.: C905), human BMP2 protein (Kactus Biosystems, Cat. No.: BMP-HE002), and human GDF15 protein (Kactus Biosystems, Cat. No.: GDF-HE115) and then incubated at 4 °C overnight. Non-specific binding sites were blocked by incubation with PBS containing 1% BSA at 37 °C for 1 h. After the blocking was completed, the plate was washed three times with PBST (PBS containing 0.05% Tween 20). The humanized anti-GDF15 antibodies and an IgG control (Sino Biological, Cat. No.: HG1K) were diluted to 4 µg/mL with a binding buffer (PBS containing 0.05% Tween 20 and 0.5% BSA) and incubated with the proteins on the plate at 37 °C for 1 h. After the incubation was completed, the plate was washed three times with PBST. A peroxidase-labeled goat anti-human Fc secondary antibody (Jackson Immuno Research, Cat# 109-035-098) was diluted in a 1/20000 ratio with a binding buffer and added to the plate. The plate was incubated at 37 °C for 1 h, then washed again, and subjected to color development with TMB. Then, the color development was stopped with 1 M H₂SO₄. The absorbance (OD values) at 450 nm-620 nm was measured using a microplate reader (MD, SpectraMax_i3x).

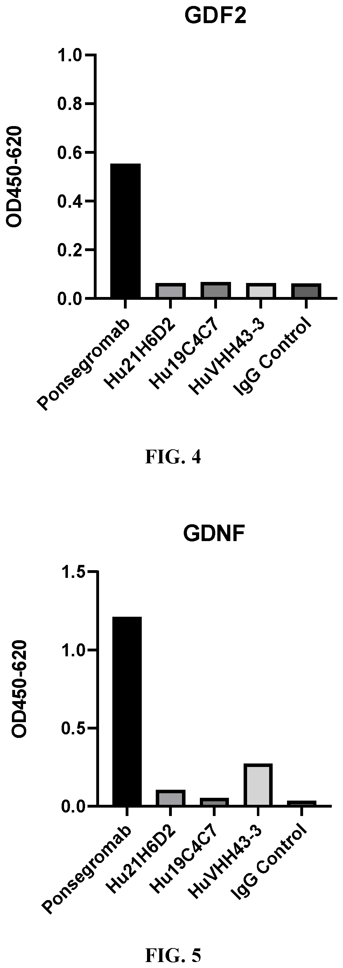

The OD values for the binding of the humanized anti-GDF15 antibodies to the human TGFβ family proteins are shown in Table 11-1. The humanized anti-GDF15 antibodies did not bind to the human GDF1, GDF2, GDF3, AMH, BMP4, GDF11, GDF8, GDNF, TGFβ1, GFRα1, Activin A, Activin B, or BMP2 proteins, demonstrating excellent specificity. FIGs. 4 and 5 show that the control antibody Ponsegromab non-specifically bound to GDNF and GDF-2. Considering that GDF-2 is associated with chondroskeletal development and GDNF promotes neuronal survival, the binding of Ponsegromab to GDNF and GDF-2 may cause safety problems.

**Table 11-1. OD values for binding of humanized anti-GDF15 antibodies to human TGFβ family proteins**

| Name | Ponsegromab | Hu21H6D2 | Hu19C4C7 | HuVHH43-3 | IgG Control |
|---|---|---|---|---|---|
| GDF15 | 3.3331 | 3.1698 | 3.2659 | 3.2139 | 0.0386 |
| GDF1 | 0.0818 | 0.0412 | 0.0433 | 0.0504 | 0.0416 |
| GDF2 | 0.5553 | 0.0648 | 0.0678 | 0.0644 | 0.0622 |
| GDF3 | 0.0676 | 0.0410 | 0.0418 | 0.0406 | 0.0400 |
| AMH | 0.0754 | 0.0537 | 0.0464 | 0.0485 | 0.0465 |
| BMP4 | 0.0639 | 0.0411 | 0.0432 | 0.0539 | 0.0439 |
| GDF11 | 0.0375 | 0.0426 | 0.0368 | 0.0400 | 0.0380 |
| GDF8 | 0.0755 | 0.0426 | 0.0423 | 0.0421 | 0.0351 |
| GDNF | 1.2133 | 0.1054 | 0.0531 | 0.2732 | 0.0360 |
| TGFβ1 | 0.1289 | 0.0426 | 0.0437 | 0.0550 | 0.0376 |
| GFRα1 | 0.1239 | 0.0655 | 0.0468 | 0.2702 | 0.1248 |
| BMP2 | 0.2653 | 0.0683 | 0.0457 | 0.0538 | 0.0463 |
| ActivinA | 0.0698 | 0.0632 | 0.0577 | 0.0493 | 0.0524 |
| ActivinB | 0.1874 | 0.0899 | 0.1356 | 0.0620 | 0.1675 |

8.2 To evaluate the binding specificity of the anti-GDF15 antibody with YTE mutations in the Fc region to GDF15, the binding activity of the anti-GDF15 antibody with YTE mutations in the Fc region to human GDF1, GDF2, GDF3, AMH, BMP4, GDF11, GDF8, GDNF, TGFβ1, GFRα1, Activin A, Activin B, and BMP2 was determined using the ELISA method.

The experimental procedures were the same as those in Example 8.1, and the results are shown in Table 11-2.

The results show that both the anti-GDF15 antibodies hu19C4C7 and hu19C4C7-YTE did not bind to human GDF1, GDF2, GDF3, AMH, BMP4, GDF11, GDF8, GDNF, TGFβ1, GFRα1, Activin A, Activin B, or BMP2 proteins, whereas the control antibody showed relatively strong binding to GDNF and GDF2. This indicates that the specificity of hu19C4C7 and hu19C4C7-YTE was superior to that of the control antibody.

**Table 11-2. OD values for binding of anti-GDF15 antibodies to human TGFβ family proteins**

| Name | Ponsegromab | hu19C4C7-YTE | hu19C4C7 | IgG Control |
|---|---|---|---|---|
| GDF15 | 3.3331 | 2.3867 | 2.4744 | 0.043 |
| GDF1 | 0.0818 | 0.14405 | 0.20125 | 0.10125 |
| GDF2 | 0.5553 | 0.0643 | 0.0616 | 0.04435 |
| GDF3 | 0.0676 | 0.09075 | 0.08625 | 0.05565 |
| AMH | 0.0754 | 0.0409 | 0.08875 | 0.0569 |
| BMP4 | 0.0639 | 0.05945 | 0.05875 | 0.0464 |
| GDF11 | 0.0375 | 0.0709 | 0.0735 | 0.06585 |
| GDF8 | 0.0755 | 0.0508 | 0.05045 | 0.04845 |
| GDNF | 1.2133 | 0.29645 | 0.2408 | 0.04935 |
| TGFβ1 | 0.1289 | 0.1141 | 0.1026 | 0.111 |
| GFRα1 | 0.1239 | 0.1321 | 0.1098 | 0.0807 |
| BMP2 | 0.2265 | 0.09725 | 0.07635 | 0.055 |
| ActivinA | 0.0863 | 0.05815 | 0.05945 | 0.04745 |
| ActivinB | 0.1562 | 0.11415 | 0.1203 | 0.082 |

### Example 9: Reporter Gene Activity of Humanized Anti-GDF15 Antibodies and Humanized Anti-GDF15 Antibody with YTE Mutations in Fc Region

9.1 HEK293 cells (ATCC, Cat. No.: CRL-1573) were maintained in a DMEM culture medium containing 10% FBS in a humidified incubator with 5% CO₂ at 37 °C. GFRAL (Gene ID: NM_207410.2), RET9 (Gene ID: NM_020975.6), and SRE-Luc2P (Sequence ID: AAV52871.1) plasmids were co-transfected into HEK293 cells via lentivirus, and puromycin (Gibco, Cat. No.: A1113802), hygromycin (ThermoFisher, Cat. No.: 10687010), and bleomycin (Beyotime, Cat. No.: ST1450-1mL) were used for screening to obtain polyclonal cells expressing human GFRAL, RET9, and SRE-Luc2P (HEK293/GFRAL/RET9/SRE-Luc2P cells). HEK293/GFRAL/RET9/SRE-Luc2P cells were resuspended in a culture medium (99.375% DMEM + 0.625% FBS) containing 12.5 nM GDF15 (ACRO, Cat. No. GD5-H5149) and then seeded into a 384-well plate (20000 cells (40 µL)/well). The antibodies were diluted in a 5× gradient (starting from a final concentration of 30 nM, 2-fold dilution, 8 points) using a plain DMEM culture medium, and then the antibody dilutions were added at 10 µL/well. The culture plate was incubated in an incubator with 5% CO₂ at 37 °C for 5 h. After the incubation, a One-Glo^{TM} reagent (Promega, Cat. No.: E6130) was added to the assay plate at 25 µL/well, and luminescence was measured using a luminescence microplate reader (Tecan F200 Pro).

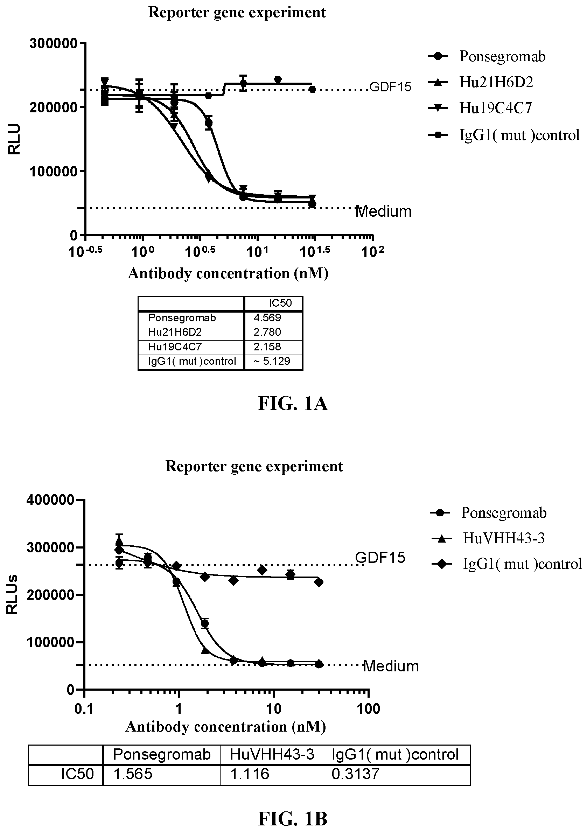

The results are shown in FIGs. 1A and 1B. The results indicate that GDF15 protein could activate the SRE-Luc2P reporter gene activity; Hu21H6D2, Hu19C4C7, and HuVHH43-3 could block this process, inhibiting the reporter gene activity, and their inhibitory effects showed a dose-dependent trend; moreover, Hu21H6D2, Hu19C4C7, and HuVHH43-3 exhibited superior EC₅₀ values compared to Ponsegromab, demonstrating stronger *in vitro* efficacy.

9.2 Reporter gene activity assay of humanized anti-GDF15 antibody with YTE mutations in Fc region HEK293 cells (ATCC, Cat. No.: CRL-1573) were maintained in a DMEM culture medium containing 10% FBS in a humidified incubator with 5% CO₂ at 37 °C. GFRAL (Gene ID: NM_207410.2), RET9 (Gene ID: NM_020975.6), and SRE-Luc2P (Sequence ID: AAV52871.1) plasmids were co-transfected into HEK293 cells via lentivirus, and puromycin (Gibco, Cat. No.: A1113802), hygromycin (ThermoFisher, Cat. No.: 10687010), and bleomycin (Beyotime, Cat. No.: ST1450-1mL) were used for screening to obtain polyclonal cells expressing human GFRAL, RET9, and SRE-Luc2P. Finally, a monoclonal cell line simultaneously expressing human GFRAL, RET9, and SRE-Luc2P was obtained by the limiting dilution method. HEK293/GFRAL/RET9/SRE-Luc2P cells were resuspended in a culture medium (99.375% DMEM + 0.625% FBS) containing 10 nM GDF-15 (ACRO, Cat. No. GD5-H5149) and then seeded into a 384-well plate (20000 cells (40 µL)/well). The antibodies were diluted in a 5× gradient (starting from a final concentration of 100 nM, 2-fold dilution, 12 points) using a plain DMEM culture medium, and then the antibody dilutions were added at 10 µL/well. The culture plate was incubated in an incubator with 5% CO₂ at 37 °C for 5 h. After the incubation, a Bio-Lite Luciferase Assay System reagent (Vazyme, Cat. No.: DD1201) was added to the assay plate at 25 µL/well, and luminescence was measured using a luminescence microplate reader (Tecan F200 Pro).

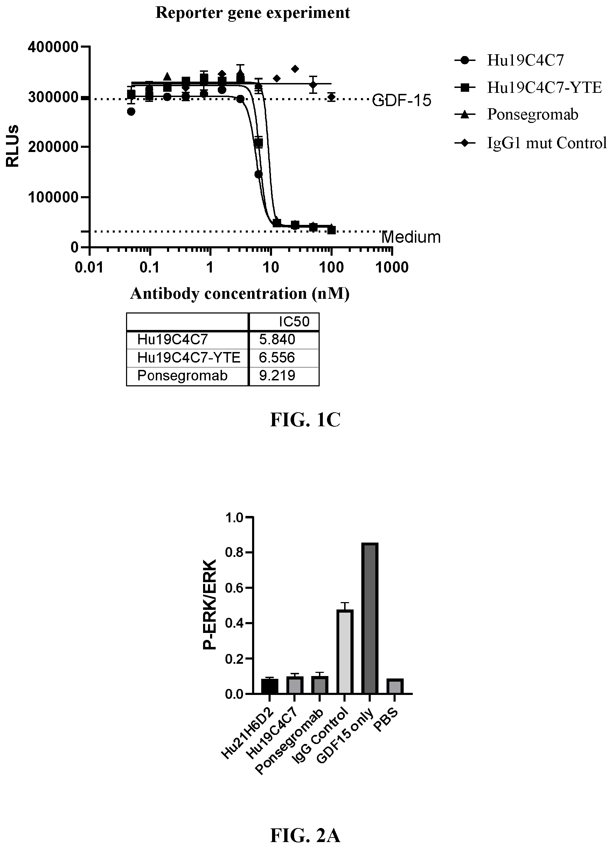

The results are shown in FIG. 1C. The results indicate that GDF-15 protein could activate the SRE-Luc2P reporter gene activity; hu19C4C7 and 19C4C7-YTE could block this process, inhibiting the reporter gene activity, and their inhibitory effects showed a dose-dependent trend and were superior to that of the control antibody Ponsegromab.

### Example 10: Inhibition of ERK Phosphorylation Levels by Humanized Anti-GDF15 Antibodies

To evaluate the inhibition of downstream ERK phosphorylation levels of GDF15/GFRAL/RET by the anti-GDF15 antibodies, 12.5 nM GDF15 and 10 µL of anti-human GDF15 antibody were co-incubated at 37 °C for 30 min. The mixture was then added to HEK293/GFRAL/RET9/SRE-Luc2P cells (2 × 10⁶ cells/well), and the resulting mixture was incubated at 37 °C for 10 min. The supernatant was removed. A lysis buffer (Solarbio, Cat. No.: R0010) was added at 200 µL/well, and the cells were lysed at 4 °C for 10 min. The levels of ERK and p-ERK were detected by Western blot.

After the cell lysis, the total protein concentration was determined using a BCA protein concentration assay kit (Beyotime, Cat. No.: P0010S). Then, 80 µL of the cell lysate and 20 µL of a 5× loading buffer were mixed well. The mixture was placed in a metal bath at 100 °C for 5 min, then mixed well again, and centrifuged. The sample was loaded at 20 µg/well, and a marker (Thermo Scientific, Cat. No.: 26619) was added. Electrophoresis was then performed. After the electrophoresis was completed, a PVDF membrane (Merck, Cat. No.: ISEQ00010) was cut to the desired size according to the size of the gel. The membrane was first immersed in methanol for 30 s for membrane activation, and then transferred to an equilibration solution (Nanjing GenScript, Cat. No.: L00734C) for membrane transfer. After the membrane transfer was completed, the membrane was washed once with PBST, and then blocked in a blocking buffer (PBS containing 5% BSA and 0.05% Tween 20) on a shaker at room temperature for 1 h. After the blocking was completed, ERK1/2 antibody (ABclonal Technology, Cat. No.: A4782) and a p-ERK1/2 antibody (ABclonal Technology, Cat. No.: AP0974) were prepared at a 1:1000 dilution. The blocked membrane was placed in the prepared solutions and incubated at 4 °C overnight. The incubated membrane was then washed 3 times with PBST (5 min each time). A secondary antibody (Cell Signaling, Cat. No.: 7074S) was prepared at a 1:2000 dilution. The washed membrane was placed in the prepared solution and incubated on a shaker at room temperature for 1 h, followed by 3 washes with PBST (5 min each time). Luminescence solutions A and B were diluted and mixed at a 1:1 ratio. One corner of the membrane was lifted with forceps, and excess liquid on the membrane was blotted by touching the corner with filter paper. A developing solution was then dropped onto the membrane, and images were captured using a chemiluminescence imaging system for grayscale analysis.

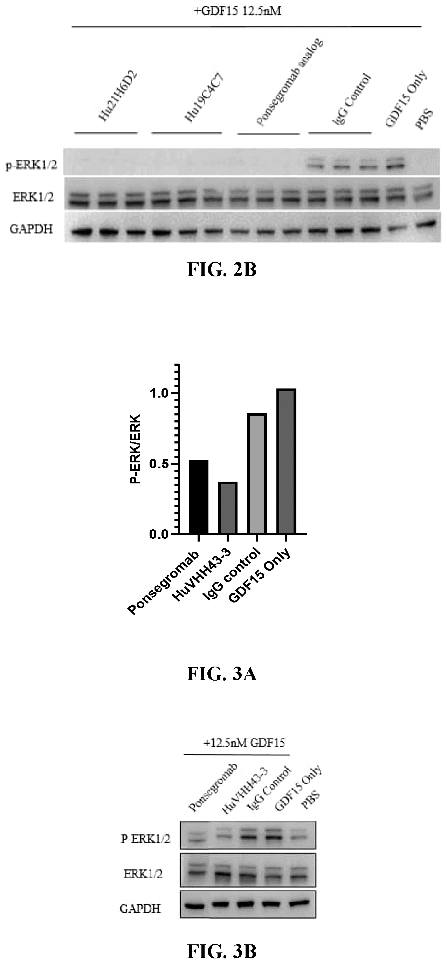

FIGs. 2A and 2B and FIGs. 3A and 3B show the grayscale value analysis results of p-ERK/ERK for the humanized anti-GDF15 antibodies as determined by Western blot. The results show that the humanized anti-GDF15 antibodies could all inhibit ERK phosphorylation, and the inhibition of ERK1/2 phosphorylation levels by Hu21H6D2 and Hu19C4C7 was comparable to that by Ponsegromab, while HuVHH43-3 was superior to Ponsegromab.

### Example 11: Reversal of HT-1080-Induced Tumor Cachexia by Humanized Anti-GDF15 Antibodies

The ability of the humanized anti-GDF15 antibodies to reverse tumor cachexia was investigated using a CB17-SCID mouse HT-1080 tumor cachexia model.

Eighty CB17-SCID mice were purchased from Beijing Vital River Laboratory Animal Technology Co., Ltd.

Human fibrosarcoma HT-1080 cells were purchased from Cell Bank of Chinese Academy of Sciences. The cells were cultured in an incubator with 5% CO₂ at 37 °C, and the culture medium used was a DMEM culture medium containing 10% inactivated fetal bovine serum.

Eight mice were randomly selected and not inoculated with tumor cells (NTB). The remaining 72 CB17-SCID mice were subcutaneously inoculated on the right dorsal side with 2 × 10⁶ HT-1080 cells/0.1 mL serum-free DMEM/0.1 mL Matrigel matrix per mouse.

Experiment 1: The tumor volume in tumor-bearing mice was measured using a vernier caliper. Based on the conversion that a tumor volume of 1 cm³ corresponds to a tumor weight of approximately 1 g, the tumor weight was subtracted to give the net body weight (actual body weight - tumor weight). When the net body weight decreased by about 8% as compared to that on day 0 post-inoculation, suitable mice were selected and randomly assigned to 5 experimental groups, with 6 mice in each group. On days 0 and 3 after grouping, a blank control (PBS), Ponsegromab (10 mg/kg), Hu19C4C7 (10 mg/kg), Hu21H6D2 (10 mg/kg), and HuVHH43-3 (5.3 mg/kg) were intraperitoneally administered to the mice, respectively. Every other day after grouping, all mice were weighed, and the maximum length of the major axis (L) and the maximum length of the minor axis (W) of tumors in tumor-bearing mice were measured. The tumor volume was calculated using the following formula: V = L × W²/2.

Mouse serum was collected at 72 hours after the first administration (prior to the second administration) and at the experimental endpoint to detect free GDF15 levels and free drug levels.

Experiment 2: A similar experiment was conducted as described above. Eight mice were randomly selected and not inoculated with tumor cells (G1 NTB). The remaining 53 CB17-SCID mice were subcutaneously inoculated on the right dorsal side with 2 × 10⁶ HT-1080 cells/0.1 mL serum-free DMEM/0.1 mL Matrigel matrix per mouse.

The tumor volume in tumor-bearing mice was measured using a vernier caliper. Based on the conversion that a tumor volume of 1 cm³ corresponds to a tumor weight of approximately 1 g, the tumor weight was subtracted to give the net body weight (actual body weight - tumor weight). When the net body weight decreased by about 3% as compared to that on day 0 post-inoculation, suitable mice were selected and randomly assigned to 7 experimental groups, with 5 mice in each group. On days 0 and 3 after grouping, a blank control (PBS) (G2), Ponsegromab (1 mg/kg) (G3), Ponsegromab (3 mg/kg) (G4), Hu19C4C7 (1 mg/kg) (G5), Hu19C4C7 (3 mg/kg) (G6), Hu21H6D2 (1 mg/kg) (G7), and Hu21H6D2 (3 mg/kg) (G8) were intraperitoneally administered to the mice, respectively. On days 0, 2, 5, and 7 after grouping, all mice were weighed, and the maximum length of the major axis (L) and the maximum length of the minor axis (W) of tumors in tumor-bearing mice were measured. The tumor volume was calculated using the following formula: V = L × W²/2. Every 2-3 days after grouping, the total food intake in each group of mice was monitored, and the daily food intake per mouse was calculated accordingly. The monitoring was performed 3 times during the experimental period. At the experimental endpoint (day 7 after grouping), all mice were euthanized, and bilateral ovarian fat was collected and weighed.

Free drug detection method: Human GDF15 protein (Kactus Biosystems, Cat. No.: GDF-HE115) was diluted to 0.5 µg/mL with PBS and added to a 96-well plate at 50 µL/well for coating, and the plate was incubated at 4 °C overnight. Non-specific binding sites were blocked by incubation with PBS containing 1% BSA at 37 °C for 1 h. After the blocking was completed, the plate was washed three times with PBST (PBS containing 0.05% Tween 20). Ponsegromab, Hu19C4C7, Hu21H6D2, and HuVHH43-3 were diluted to 20 ng/mL with a diluent containing 1% blank mouse serum, followed by two-fold serial dilution to give final concentrations of 20 ng/mL, 10 ng/mL, 5 ng/mL, 2.5 ng/mL, 1.25 ng/mL, 0.63 ng/mL, and 0.31 ng/mL. The antibody dilutions, together with diluted mouse serum samples, were added to the plate, and the plate was incubated at 37 °C for 1 h. After the incubation was completed, the plate was washed three times with PBST. A peroxidase-labeled goat anti-human Fc secondary antibody (Jackson Immuno Research, Cat# 109-035-098) was diluted in a 1/20000 ratio with a binding buffer and added to the plate. The plate was incubated at 37 °C for 1 h, then washed again, and subjected to color development with TMB. Then, the color development was stopped with 1 M H₂SO₄. The absorbance (OD values) at 450 nm-620 nm was measured using a microplate reader.

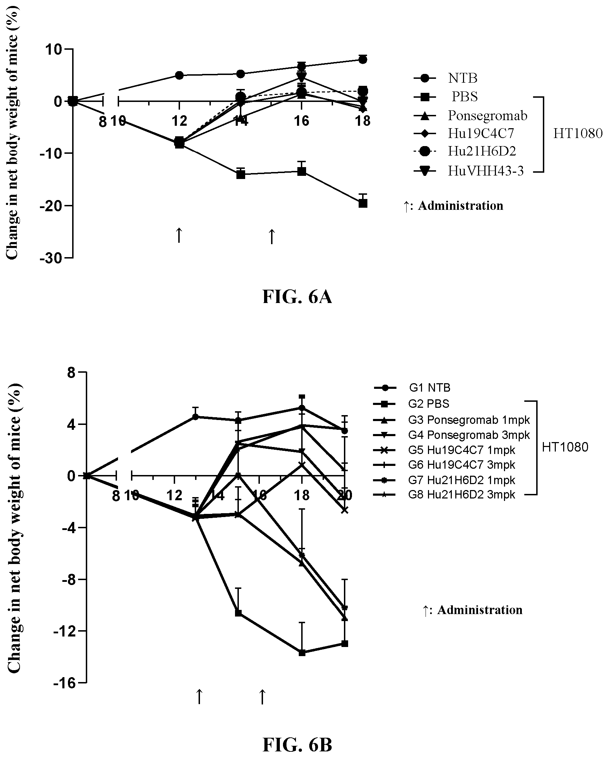

FIG. 6A shows the changes in net body weight of mice on day 6 after administration in Experiment 1. Treatment with the humanized GDF15 antibodies significantly ameliorated cachexia-induced body weight loss in mice compared to treatment with PBS. The body weights of the mice in the Hu19C4C7, Hu21H6D2, HuVHH43-3, and Ponsegromab administration groups recovered from -19.6% in the PBS group to -1.5%, +2.0%, -1.0%, and -0.1%, respectively. Hu21H6D2 and HuVHH43-3 were superior to Ponsegromab, while Hu19C4C7 was comparable to Ponsegromab.

As shown in FIG. 6B, on day 7 after grouping, the body weight in the PBS group decreased to -13.0%. All administration groups showed a dose-dependent inhibition of body weight loss in mice. At 1 mg/kg, the body weights of the mice in the Ponsegromab, Hu19C4C7, and Hu21H6D2 groups recovered to -11.0%, -2.6%, and -10.2%, respectively; at 3 mg/kg, the body weights of the mice in the Ponsegromab, Hu19C4C7, and Hu21H6D2 groups recovered to -1.8%, 0.4%, and 3.6%, respectively. Compared to the control antibody Ponsegromab, Hu19C4C7 and Hu21H6D2 were able to reverse tumor-induced body weight loss more potently.

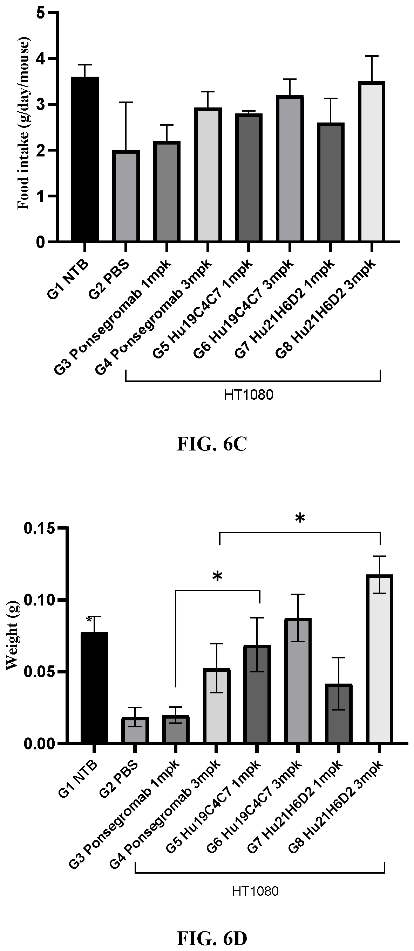

As shown in FIG. 6C, all administration groups exhibited a dose-dependent increase in food intake in mice.

As shown in FIG. 6D, on day 7 after grouping, the ovarian fat weight in the PBS group was significantly reduced, while all administration groups exhibited an increase in ovarian fat weight at different doses. Compared to the control antibody Ponsegromab, Hu19C4C7 at a dose of 1 mg/kg and Hu21H6D2 at a dose of 3 mg/kg could more significantly increase ovarian fat weight.

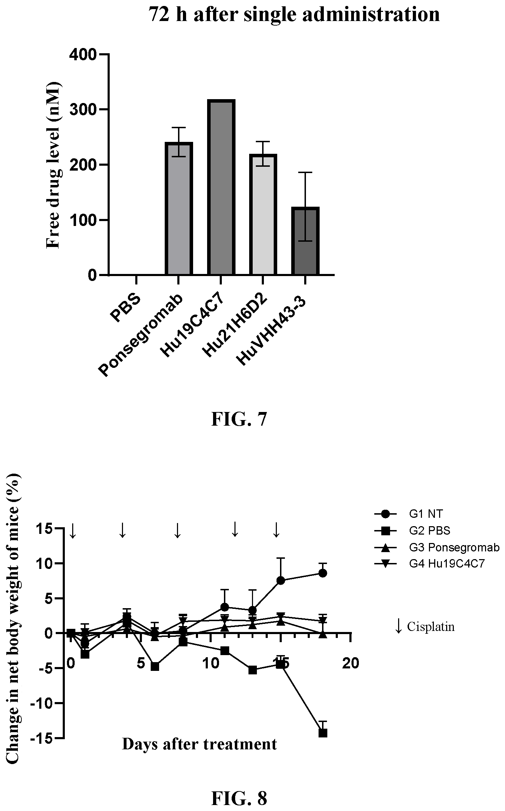

FIG. 7 shows the free drug concentrations 72 h after a single administration, with Hu19C4C7, Hu21H6D2, and HuVHH43-3 exhibiting good drug exposure levels.

### Example 12: Reversal of Cisplatin-Induced Cachexia by Humanized Anti-GDF15 Antibodies Eighteen B-hGDF15 mice were purchased from Biocytogen Pharmaceuticals (Beijing) Co., Ltd.

Three mice were randomly selected and left untreated (G1 NT). The remaining 15 mice were randomly divided into 3 groups, with 5 mice in each group. On days 0, 4, 8, 12, and 15, cisplatin was intraperitoneally administered to the mice at 5 mg/kg. On days 0, 4, 7, 11, and 14 after grouping, a blank control (PBS) (G2), Ponsegromab (10 mg/kg) (G3), and Hu19C4C7 (10 mg/kg) (G4) were intraperitoneally administered to the mice. On days 0, 1, 4, 6, 8, 11, 13, 15, and 18, the body weight of the mice was monitored. At the endpoint, blood was collected to detect free drug levels (as shown in Example 11).

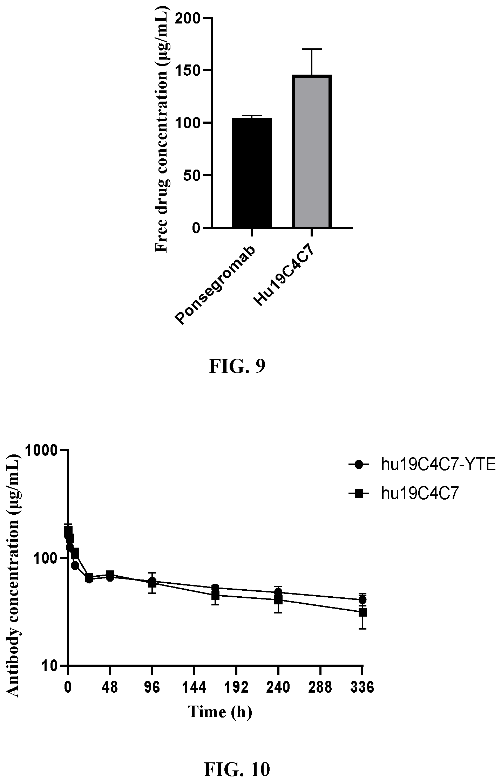

FIG. 8 shows the changes in body weight of mice. On day 18 after administration, all administration groups showed significant amelioration in cachexia compared to the PBS group at the experimental endpoint. The body weights of the mice in the Hu19C4C7 and Ponsegromab administration groups recovered from -14.2% in the PBS group to +1.7% and -0.1%, respectively, and Hu19C4C7 was superior to Ponsegromab. FIG. 9 shows the free drug levels at the endpoint, with the level of Hu19C4C7 being higher than that of Ponsegromab.

### Example 13: Pharmacokinetic Study of Humanized Anti-GDF15 Antibody in Rats

The pharmacokinetic characteristics of the humanized anti-GDF15 antibody Hu19C4C7 in rats (PharmaLegacy) were evaluated. The procedures involving the care and use of animals in this study were reviewed and approved. For each antibody, 3 naive rats were used. In this study, the animals were intravenously injected with the Hu19C4C7 antibody at a dose of 10 mg/kg, and blood samples were collected at various time points from 0 h to 336 h (day 0 to day 14). All samples were processed into serum and stored frozen at -70 °C to -86 °C until analysis. The concentration of the Hu19C4C7 antibody in the serum was determined.

The pharmacokinetic results of the Hu19C4C7 antibody were analyzed by capture with human GDF15 protein and detection with the peroxidase-labeled goat anti-human Fc secondary antibody (as shown in Example 11). Pharmacokinetic parameters were analyzed using WinNonlin 8.3 and are shown in Table 12. The humanized anti-GDF15 antibody Hu19C4C7 exhibited very excellent pharmacokinetic properties, with a half-life of up to 12.6 days in rats.

**Table 12. Pharmacokinetic parameters of Hu19C4C7 antibody in rats**

| PK parameter | Unit | Hu19C4C7 |
|---|---|---|
| T1/2 | h | 302.7 |
| Cmax | µg/ml | 241.3 |
| AUClast | h*µg/ml | 22490.4 |
| CL | ml/h/kg | 0.25 |
| Vz | ml/kg | 107.8 |

### Example 14: Assay for Affinity of Anti-GDF15 Antibody with YTE Mutations in Fc Region for Human FcRn

The binding affinity of hu19C4C7 and hu19C4C7-YTE antibodies for human FcRn protein (ACRO Biosystems, Cat. No.: FCM-H82W4) was determined using ForteBio Octet RED96e.

Briefly, an SA sensor (ForteBio, Cat. No.: 18-5019) was pre-equilibrated in a loading buffer (1× PBS, Cytiva, Cat. No.: SH30256.01, containing 0.05% Tween 20, pH 7.0) at room temperature for 10 min. In a 96-well plate, the FcRn kinetic assay was performed according to the following steps:
a) the baseline was equilibrated with the loading buffer for 180 s;
b) the human FcRn protein diluted with the loading buffer to a final concentration of 1 µg/mL was added, and the immobilization was stopped until the amount reached 0.3 nm;
c) the baseline was equilibrated with a blocking buffer (1× PBS, Cytiva, Cat. No.: SH30256.01, containing 0.05% Tween 20 and 0.1% BSA, pH 7.0) for 180 s;
d) the hu19C4C7 and hu19C4C7-YTE antibodies diluted to the following concentrations with a running buffer (1× PBS, Cytiva, Cat. No.: SH30256.01, containing 0.05% Tween 20 and 0.1% BSA, pH 6.0/7.4) were added to the respective wells: 200 nM, 100 nM, 50 nM, 25 nM, 12.5 nM, 6.25 nM, and 3.13 nM, followed by binding for 60 s (pH 6.0) and dissociation for 60 s (pH 6.0/7.4); and
e) regeneration was performed with a regeneration solution (1× PBS, Cytiva, Cat. No.: SH30256.01, pH 7.7) for 30 s, and the baseline was equilibrated with the blocking buffer for 60 s; the experimental data were fitted and calculated using a 1:1 binding model in the Fortebio Data Analysis software.

The binding affinity of the hu19C4C7 and hu19C4C7-YTE antibodies for human FcRn protein is summarized in Table 13.

**Table 13. Binding affinity of hu19C4C7 and hu19C4C7-YTE antibodies for human FcRn protein**

| Antibody | Antigen | KD (M) | Kon(1/Ms) | Kdis(1/s) |
|---|---|---|---|---|
| hu19C4C7 (binding at pH 6.0, dissociation at pH 6.0) | Human FcRn | 7.41E-09 | 1.97E+06 | 1.46E-02 |
| hu19C4C7-YTE (binding at pH 6.0, dissociation at pH 6.0) | | 2.89E-09 | 8.89E+05 | 2.57E-03 |

Table 13 indicates that, under the conditions of binding for 60 s (pH 6.0) and dissociation for 60 s (pH 6.0) in the running buffer, the hu19C4C7-YTE antibody of the present disclosure exhibited increased affinity for human FcRn compared to hu19C4C7.

### Example 15: Pharmacokinetic Study of Anti-GDF15 Antibodies in Humanized FcRn Mice

Humanized FcRn mice (Biocytogen) were intravenously injected with the anti-GDF15 antibodies at a dose of 10 mg/kg. Blood samples were collected at various time points from 0 h to 336 h. All samples were centrifuged to process into serum and stored frozen at -80 °C until analysis. The concentration of each anti-GDF15 antibody present in the serum was determined using ELISA. The ELISA procedures were as follows: A 96-well plate was coated with 0.5 µg/mL human GDF15 protein (KACTUS, Cat# GDF-HE115) at 50 µL/well and incubated at 4 °C overnight. The plate was incubated with a blocking buffer (a PBS solution containing 1% BSA) at 37 °C for blocking for 1 h. After blocking, the plate was washed once with a PBST solution (a PBS solution containing 0.05% Tween 20). The anti-GDF15 antibodies (hu19C4C7 and hu19C4C7-YTE) were diluted in a gradient with a diluent containing 2% blank mouse serum (a PBS solution containing 0.05% Tween 20 and 0.5% BSA) to serve as standards. The serum samples were diluted in different ratios with blank mouse serum and the diluent, and added to the plate. The plate was incubated at 37 °C for 1 h. After the incubation was completed, the plate was washed twice with the PBST solution. A secondary antibody (horseradish peroxidase (HRP)-labeled goat anti-human Fc secondary antibody, Jackson Immuno Research, Cat# 109-035-098) was diluted with the diluent and added to the plate. The plate was incubated at 37 °C for 1 h. After the incubation was completed, the plate was washed again, and subjected to color development with TMB (Thermo Scientific, Cat# 34029). Then, the color development was stopped with 1 M H₂SO₄. The absorbance values at OD450 nm-OD620 nm were then measured using a microplate reader. Pharmacokinetic parameters were calculated by WinNonlin 8.3 software, and the analysis results are shown in Table 14 and FIG. 10.

**Table 14. Pharmacokinetic parameters of anti-GDF15 antibodies in humanized FcRn mice**

| Parameter | Unit | hu19C4C7-YTE | hu19C4C7 |
|---|---|---|---|
| Cmax | µg/ml | 162.1 | 182.2 |
| CL | ml/h/kg | 0.2 | 0.3 |
| T1/2 | h | 457.3 | 302.3 |
| Vz | ml/kg | 140.3 | 133.4 |
| AUClast | h*µg/ml | 18662.3 | 17849.6 |

| | | | |
|---|---|---|---|
| Cmax: Maximum observed concentration value Cl: Clearance T1/2: Terminal elimination half-life Vz: Apparent volume of distribution AUClast: Area under the serum concentration-time curve from t = 0 to time t | | | |

The pharmacokinetic study of anti-GDF15 antibodies in humanized FcRn mice indicates that hu19C4C7-YTE had a superior half-life compared to hu19C4C7.

### SEQUENCE LISTING:

| SEQ ID NO | Chi-19C4C7 | |
|---|---|---|
| 1 | Chi-19C4C7>HCDR1 | GYIMN |
| 2 | Chi-19C4C7>HCDR2 | DIDPYYGGPNYNQKFKA |
| 3 | Chi-19C4C7>HCDR3 | RDYNYGSFAY |
| 4 | Chi-19C4C7>LCDR1 | GASSSVNYMH |
| 5 | Chi-19C4C7>LCDR2 | ATSNLAS |
| 6 | Chi-19C4C7>LCDR3 | QQWSDNPPT |
| 7 | Chi-19C4C7>VH | EVQLQQSGPELERPGASVKISCKASGYSFTGYIMNWVK QSDGKSLEWIGDIDPYYGGPNYNQKFKAKATLTVDKSS STAYMQLKSLTSEDSAVYYCSRRDYNYGSFAYWGQGT LVTVSA |
| 8 | Chi-19C4C7>VL | QIVLSQSPAILSASPGEKVTMTCGASSSVNYMHWYQHQ PGSSPKPWIYATSNLASGVPARFSGSGSGTSYSLTISRVE AEDAATYYCQQWSDNPPTFGGGTKLEIK |
| | Chi-21H6D2 | |
| 1 | Chi-21H6D2>HCDR1 | SEQ ID NO:1 |
| 9 | Chi-21H6D2>HCDR2 | DIDPYYGDAKYNQKFKG |
| 10 | Chi-21H6D2>HCDR3 | RDYDHGSFAY |
| 11 | Chi-21H6D2>LCDR1 | SASSSVNYMH |
| 12 | Chi-21H6D2>LCDR2 | DTSKLAS |
| 13 | Chi-21H6D2>LCDR3 | QQWSSNPPT |
| 14 | Chi-21H6D2>VH | EVQLQQSGPELEKPGASVKISCKASGYSFTGYIMNWVK QSNGKSLEWIGDIDPYYGDAKYNQKFKGKATLTVDKS SSTGYMHLKSLTSEDSTVYYCARRDYDHGSFAYWGQG TLVTVST |
| 15 | Chi-21H6D2>VL | QIVLTQSPAIMSASPGEKVTMTCSASSSVNYMHWYQQ KSGTSPKRWIYDTSKLASGVPARFSGSGSGTSYSLTIST MEAEDAATYYCQQWSSNPPTFGAGTKLELK |
| | Chi-VHH43 (defined by IMGT) | |
| 16 | Chi-VHH43>HCDR1 | GHTYG |
| 17 | Chi-VHH43>HCDR2 | ISRSGSST |
| 18 | Chi-VHH43>HCDR3 | GASTSALVPTTTGMYAY |
| 19 | Chi-VHH43>VH | QLQLVESGGGLVQAGGSLRLSCAASGHTYGMGWFRQ VPGKAREIVAAISRSGSSTYYADSVRGRFTISRDNAKNT LYLQMNSLKPEDAAVYICGASTSALVPTTTGMYAYWG QGTRVTVSS |
| | hu19C4C7 | |
| 1 | hu19C4C7>HCDR1 | SEQ ID NO:1 |
| 20 | hu19C4C7>HCDR2 | DIDPYYGGPNYNQKFQG |
| 3 | hu19C4C7>HCDR3 | SEQ ID NO:3 |
| 21 | hu19C4C7>LCDR1 | RASSSVNYMH |
| 22 | hu19C4C7>LCDR2 | ATSNLAT |
| 6 | hu19C4C7>LCDR3 | SEQ ID NO:6 |
| 23 | hu19C4C7>VH | QVQLVQSGAEVKKPGSSVKVSCKASGYSFTGYIMNWV RQAPGQSLEWMGDIDPYYGGPNYNQKFQGRVTITVDK STSTAYMELSSLRSEDTAVYYCSRRDYNYGSFAYWGQ GTLVTVSS |
| 24 | hu19C4C7>VL | QIVLTQSPATLSLSPGERATLSCRASSSVNYMHWYQQK PGQAPRPLIYATSNLATGIPARFSGSGSGTDYTLTISSLEP EDFAVYYCQQWSDNPPTFGQGTKLEIK |
| | hu21H6D2 | |
| 1 | hu21H6D2>HCDR1 | SEQ ID NO:1 |
| 9 | hu21H6D2>HCDR2 | SEQ ID NO:9 |
| 10 | hu21H6D2>HCDR3 | SEQ ID NO:10 |
| 11 | hu21H6D2>LCDR1 | SEQ ID NO:11 |
| 12 | hu21H6D2>LCDR2 | SEQ ID NO:12 |
| 13 | hu21H6D2>LCDR3 | SEQ ID NO:13 |
| 25 | hu21H6D2>VH | QVQLVQSGAEVKKPGASVKVSCKASGYTFTGYIMNWV RQAPGQRLEWMGDIDPYYGDAKYNQKFKGKVTITVDK SASTAYMELSSLRSEDTAVYYCARRDYDHGSFAYWGQ GTLVTVSS |
| 26 | hu21H6D2>VL | EIVLTQSPDFQSVTPKEKVTITCSASSSVNYMHWYQQK PDQSPKRLIYDTSKLASGVPSRFSGSGSGTDYTLTINSLE AEDAATYYCQQWSSNPPTFGQGTKLEIK |
| | | |
| | huVHH43 | |
| 16 | huVHH43>HCDR1 | SEQ ID NO:16 |
| 17 | huVHH43>HCDR2 | SEQ ID NO:17 |
| 18 | huVHH43>HCDR3 | SEQ ID NO:18 |
| 27 | huVHH43>VH | QLQLVESGGGLVQPGGSLRLSCAASGHTYGMGWFRQA PGKAREIVSAISRSGSSTYYADSVKGRFTISRDNSKNTL YLQMNSLRAEDTAVYICGASTSALVPTTTGMYAYWGQ GTRVTVSS |
| | | |
| | Chi-19C4C7-IgG1-mut(L234A,L235A,G23 7A) | |
| 28 | Chi-19C4C7-IgG1-mut>HC | EVQLQQSGPELERPGASVKISCKASGYSFTGYIMNWVK QSDGKSLEWIGDIDPYYGGPNYNQKFKAKATLTVDKSS STAYMQLKSLTSEDSAVYYCSRRDYNYGSFAYWGQGT LVTVSAASTKGPSVFPLAPSSKSTSGGTAALGCLVKDYF PEPVTVSWNSGALTSGVHTFPAVLQSSGLYSLSSVVTVP SSSLGTQTYICNVNHKPSNTKVDKKVEPKSCDKTHTCP PCPAPEAAGAPSVFLFPPKPKDTLMISRTPEVTCVVVDV SHEDPEVKFNWYVDGVEVHNAKTKPREEQYNSTYRVV SVLTVLHQDWLNGKEYKCKVSNKALPAPIEKTISKAKG QPREPQVYTLPPSREEMTKNQVSLTCLVKGFYPSDIAVE WESNGQPENNYKTTPPVLDSDGSFFLYSKLTVDKSRW QQGNVFSCSVMHEALHNHYTQKSLSLSPGK |
| 29 | Chi-19C4C7-IgG1-mut>LC | QIVLSQSPAILSASPGEKVTMTCGASSSVNYMHWYQHQ PGSSPKPWIYATSNLASGVPARFSGSGSGTSYSLTISRVE AEDAATYYCQQWSDNPPTFGGGTKLEIKRTVAAPSVFI FPPSDEQLKSGTASVVCLLNNFYPREAKVQWKVDNAL QSGNSQESVTEQDSKDSTYSLSSTLTLSKADYEKHKVY ACEVTHQGLSSPVTKSFNRGEC |
| | Chi-21H6D2-IgG1-mut(L234A,L235A,G23 7A) | |
| 30 | Chi-21H6D2-IgG1-mut>HC | EVQLQQSGPELEKPGASVKISCKASGYSFTGYIMNWVK QSNGKSLEWIGDIDPYYGDAKYNQKFKGKATLTVDKS SSTGYMHLKSLTSEDSTVYYCARRDYDHGSFAYWGQG TLVTVSTASTKGPSVFPLAPSSKSTSGGTAALGCLVKDY |
| | | FPEPVTVSWNSGALTSGVHTFPAVLQSSGLYSLSSVVTV PSSSLGTQTYICNVNHKPSNTKVDKKVEPKSCDKTHTC PPCPAPEAAGAPSVFLFPPKPKDTLMISRTPEVTCVVVD VSHEDPEVKFNWYVDGVEVHNAKTKPREEQYNSTYRV VSVLTVLHQDWLNGKEYKCKVSNKALPAPIEKTISKAK GQPREPQVYTLPPSREEMTKNQVSLTCLVKGFYPSDIA VEWESNGQPENNYKTTPPVLDSDGSFFLYSKLTVDKSR WQQGNVFSCSVMHEALHNHYTQKSLSLSPGK |
| 31 | Chi-21H6D2-IgG1-mut>LC | QIVLTQSPAIMSASPGEKVTMTCSASSSVNYMHWYQQ KSGTSPKRWIYDTSKLASGVPARFSGSGSGTSYSLTIST MEAEDAATYYCQQWSSNPPTFGAGTKLELKRTVAAPS VFIFPPSDEQLKSGTASVVCLLNNFYPREAKVQWKVDN ALQSGNSQESVTEQDSKDSTYSLSSTLTLSKADYEKHK VYACEVTHQGLSSPVTKSFNRGEC |
| | Chi-VHH43-IgG1-mut(L234A,L235A,G23 7A) | |
| 32 | Chi-VHH43-IgG1-mut>HC | QLQLVESGGGLVQAGGSLRLSCAASGHTYGMGWFRQ VPGKAREIVAAISRSGSSTYYADSVRGRFTISRDNAKNT LYLQMNSLKPEDAAVYICGASTSALVPTTTGMYAYWG QGTRVTVSSEPKSSDKTHTCPPCPAPEAAGAPSVFLFPP KPKDTLMISRTPEVTCVVVDVSHEDPEVKFNWYVDGV EVHNAKTKPREEQYNSTYRVVSVLTVLHQDWLNGKEY KCKVSNKALPAPIEKTISKAKGQPREPQVYTLPPSREEM TKNQVSLTCLVKGFYPSDIAVEWESNGQPENNYKTTPP VLDSDGSFFLYSKLTVDKSRWQQGNVFSCSVMHEALH NHYTQKSLSLSPGK |
| | hu19C4C7-IgG1-mut(L234A,L235A,G23 7A) | |
| 33 | hu19C4C7-IgG1-mut>HC | QVQLVQSGAEVKKPGSSVKVSCKASGYSFTGYIMNWV RQAPGQSLEWMGDIDPYYGGPNYNQKFQGRVTITVDK STSTAYMELSSLRSEDTAVYYCSRRDYNYGSFAYWGQ GTLVTVSSASTKGPSVFPLAPSSKSTSGGTAALGCLVKD YFPEPVTVSWNSGALTSGVHTFPAVLQSSGLYSLSSVVT VPSSSLGTQTYICNVNHKPSNTKVDKKVEPKSCDKTHT CPPCPAPEAAGAPSVFLFPPKPKDTLMISRTPEVTCVVV DVSHEDPEVKFNWYVDGVEVHNAKTKPREEQYNSTYR VVSVLTVLHQDWLNGKEYKCKVSNKALPAPIEKTISKA |
| | | KGQPREPQVYTLPPSREEMTKNQVSLTCLVKGFYPSDI AVEWESNGQPENNYKTTPPVLDSDGSFFLYSKLTVDKS RWQQGNVFSCSVMHEALHNHYTQKSLSLSPGK |
| 34 | hu19C4C7-IgG1-mut>LC hu19C4C7-YTE-IgG1-mut>LC | QIVLTQSPATLSLSPGERATLSCRASSSVNYMHWYQQK PGQAPRPLIYATSNLATGIPARFSGSGSGTDYTLTISSLEP EDFAVYYCQQWSDNPPTFGQGTKLEIKRTVAAPSVFIFP PSDEQLKSGTASVVCLLNNFYPREAKVQWKVDNALQS GNSQESVTEQDSKDSTYSLSSTLTLSKADYEKHKVYAC EVTHQGLSSPVTKSFNRGEC |
| | hu21H6D2-IgG1-mut(L234A,L235A,G23 7A) | |
| 35 | hu21H6D2-IgG1-mut>HC | QVQLVQSGAEVKKPGASVKVSCKASGYTFTGYIMNWV RQAPGQRLEWMGDIDPYYGDAKYNQKFKGKVTITVDK SASTAYMELSSLRSEDTAVYYCARRDYDHGSFAYWGQ GTLVTVSSASTKGPSVFPLAPSSKSTSGGTAALGCLVKD YFPEPVTVSWNSGALTSGVHTFPAVLQSSGLYSLSSVVT VPSSSLGTQTYICNVNHKPSNTKVDKKVEPKSCDKTHT CPPCPAPEAAGAPSVFLFPPKPKDTLMISRTPEVTCVVV DVSHEDPEVKFNWYVDGVEVHNAKTKPREEQYNSTYR VVSVLTVLHQDWLNGKEYKCKVSNKALPAPIEKTISKA KGQPREPQVYTLPPSREEMTKNQVSLTCLVKGFYPSDI AVEWESNGQPENNYKTTPPVLDSDGSFFLYSKLTVDKS RWQQGNVFSCSVMHEALHNHYTQKSLSLSPGK |
| 36 | hu21H6D2-IgG1-mut>LC | EIVLTQSPDFQSVTPKEKVTITCSASSSVNYMHWYQQK PDQSPKRLIYDTSKLASGVPSRFSGSGSGTDYTLTINSLE AEDAATYYCQQWSSNPPTFGQGTKLEIKRTVAAPSVFIF PPSDEQLKSGTASVVCLLNNFYPREAKVQWKVDNALQ SGNSQESVTEQDSKDSTYSLSSTLTLSKADYEKHKVYA CEVTHQGLSSPVTKSFNRGEC |
| | huVHH43-IgG1-mut(L234A,L235A,G23 7A) | |
| 37 | huVHH43-IgG1-mut>HC | QLQLVESGGGLVQPGGSLRLSCAASGHTYGMGWFRQA PGKAREIVSAISRSGSSTYYADSVKGRFTISRDNSKNTL YLQMNSLRAEDTAVYICGASTSALVPTTTGMYAYWGQ GTRVTVSSEPKSSDKTHTCPPCPAPEAAGAPSVFLFPPK PKDTLMISRTPEVTCVVVDVSHEDPEVKFNWYVDGVE |
| | | VHNAKTKPREEQYNSTYRVVSVLTVLHQDWLNGKEY KCKVSNKALPAPIEKTISKAKGQPREPQVYTLPPSREEM TKNQVSLTCLVKGFYPSDIAVEWESNGQPENNYKTTPP VLDSDGSFFLYSKLTVDKSRWQQGNVFSCSVMHEALH NHYTQKSLSLSPGK |
| | | |
| | Ponsegromab | |
| 38 | Ponsegromab>HC | QVQLVQSGAEVKKPGSSVKVSCKASGYTFSSYNIDWV RQAPGQGLEWMGGINPIFGTAFYNQKFQGRVTITADES TSTAYMELSSLRSEDTAVYYCAREAITTVGAMDHWGQ GTLVTVSSASTKGPSVFPLAPSSKSTSGGTAALGCLVKD YFPEPVTVSWNSGALTSGVHTFPAVLQSSGLYSLSSVVT VPSSSLGTQTYICNVNHKPSNTKVDKKVEPKSCDKTHT CPPCPAPEAAGAPSVFLFPPKPKDTLMISRTPEVTCVVV DVSHEDPEVKFNWYVDGVEVHNAKTKPREEQYNSTYR VVSVLTVLHQDWLNGKEYKCKVSNKALPAPIEKTISKA KGQPREPQVYTLPPSREEMTKNQVSLTCLVKGFYPSDI AVEWESNGQPENNYKTTPPVLDSDGSFFLYSKLTVDKS RWQQGNVFSCSVMHEALHNHYTQKSLSLSPGK |
| 39 | Ponsegromab>LC | EIVLTQSPATLSLSPGERATLSCRTSQSVHNYLAWYQQK PGQAPRLLIYDASTRADGIPARFSGSGSGTDFTLTISSLE PEDFAVYYCQQFWSWPWTFGQGTKVEIKRTVAAPSVFI FPPSDEQLKSGTASVVCLLNNFYPREAKVQWKVDNAL QSGNSQESVTEQDSKDSTYSLSSTLTLSKADYEKHKVY ACEVTHQGLSSPVTKSFNRGEC |
| 40 | Human-GDF15-his | HHHHHHARNGDHCPLGPGRCCRLHTVRASLEDLGWA DWVLSPREVQVTMCIGACPSQFRAANMHAQIKTSLHR LKPDTVPAPCCVPASYNPMVLIQKTDTGVSLQTYDDLL AKDCHCI |
| 41 | Cynomolgus-GDF15 | HHHHHHRRARARNGDRCPLGPGRCCRLHTVHASLEDL GWADWVLSPREVQVTMCIGACPSQFREANMHAQIKM NLHRLKPDTVPAPCCVPASYNPMVLIQKTDTGVSLQTY DDLLAKDCHCV |
| 42 | Mouse-GDF15 | HHHHHHSAHAHPRDSCPLGPGRCCHLETVQATLEDLG WSDWVLSPRQLQLSMCVGECPHLYRSANTHAQIKARL HGLQPDKVPAPCCVPSSYTPVVLMHRTDSGVSLQTYD DLVARGCHCA |
| 43 | IgG1 mut Fc region (L234A, L235A, G237A) | DKTHTCPPCPAPEAAGAPSVFLFPPKPKDTLMISRTPEV TCVVVDVSHEDPEVKFNWYVDGVEVHNAKTKPREEQ YNSTYRVVSVLTVLHQDWLNGKEYKCKVSNKALPAPI EKTISKAKGQPREPQVYTLPPSREEMTKNQVSLTCLVK GFYPSDIAVEWESNGQPENNYKTTPPVLDSDGSFFLYSK LTVDKSRWQQGNVFSCSVMHEALHNHYTQKSLSLSPG K |
| 44 | IgG1-mut heavy chain constant region (L234A, L235A, G237A) | ASTKGPSVFPLAPSSKSTSGGTAALGCLVKDYFPEPVTV SWNSGALTSGVHTFPAVLQSSGLYSLSSVVTVPSSSLGT QTYICNVNHKPSNTKVDKKVEPKSCDKTHTCPPCPAPE AAGAPSVFLFPPKPKDTLMISRTPEVTCVVVDVSHEDPE VKFNWYVDGVEVHNAKTKPREEQYNSTYRVVSVLTVL HQDWLNGKEYKCKVSNKALPAPIEKTISKAKGQPREPQ VYTLPPSREEMTKNQVSLTCLVKGFYPSDIAVEWESNG QPENNYKTTPPVLDSDGSFFLYSKLTVDKSRWQQGNVF SCSVMHEALHNHYTQKSLSLSPGK |
| 45 | Kappa light chain constant region | |
| 46 | IgG1-Fc region | DKTHTCPPCPAPELLGGPSVFLFPPKPKDTLMISRTPEVT CVVVDVSHEDPEVKFNWYVDGVEVHNAKTKPREEQY NSTYRVVSVLTVLHQDWLNGKEYKCKVSNKALPAPIE KTISKAKGQPREPQVYTLPPSRDELTKNQVSLTCLVKGF YPSDIAVEWESNGQPENNYKTTPPVLDSDGSFFLYSKLT VDKSRWQQGNVFSCSVMHEALHNHYTQKSLSLSPGK |
| 47 | IgG1 mut Fc region L234A, L235A, G237A; M252Y/S254T/T256E | DKTHTCPPCPAPEAAGAPSVFLFPPKPKDTLYITREPEVT CVVVDVSHEDPEVKFNWYVDGVEVHNAKTKPREEQY NSTYRVVSVLTVLHQDWLNGKEYKCKVSNKALPAPIE KTISKAKGQPREPQVYTLPPSREEMTKNQVSLTCLVKG FYPSDIAVEWESNGQPENNYKTTPPVLDSDGSFFLYSKL TVDKSRWQQGNVFSCSVMHEALHNHYTQKSLSLSPGK |
| 48 | IgG1-mut heavy chain constant region L234A, L235A, G237A; M252Y/S254T/T256E | ASTKGPSVFPLAPSSKSTSGGTAALGCLVKDYFPEPVTV SWNSGALTSGVHTFPAVLQSSGLYSLSSVVTVPSSSLGT QTYICNVNHKPSNTKVDKKVEPKSCDKTHTCPPCPAPE AAGAPSVFLFPPKPKDTLYITREPEVTCVVVDVSHEDPE VKFNWYVDGVEVHNAKTKPREEQYNSTYRVVSVLTVL HQDWLNGKEYKCKVSNKALPAPIEKTISKAKGQPREPQ VYTLPPSREEMTKNQVSLTCLVKGFYPSDIAVEWESNG QPENNYKTTPPVLDSDGSFFLYSKLTVDKSRWQQGNVF |
| | | SCSVMHEALHNHYTQKSLSLSPGK |
| 49 | hu19C4C7-YTE-IgG1-mut>HC | QVQLVQSGAEVKKPGSSVKVSCKASGYSFTGYIMNWV RQAPGQSLEWMGDIDPYYGGPNYNQKFQGRVTITVDK STSTAYMELSSLRSEDTAVYYCSRRDYNYGSFAYWGQ GTLVTVSSASTKGPSVFPLAPSSKSTSGGTAALGCLVKD YFPEPVTVSWNSGALTSGVHTFPAVLQSSGLYSLSSVVT VPSSSLGTQTYICNVNHKPSNTKVDKKVEPKSCDKTHT CPPCPAPEAAGAPSVFLFPPKPKDTLYITREPEVTCVVV DVSHEDPEVKFNWYVDGVEVHNAKTKPREEQYNSTYR VVSVLTVLHQDWLNGKEYKCKVSNKALPAPIEKTISKA KGQPREPQVYTLPPSREEMTKNQVSLTCLVKGFYPSDI AVEWESNGQPENNYKTTPPVLDSDGSFFLYSKLTVDKS RWQQGNVFSCSVMHEALHNHYTQKSLSLSPGK |
| 50 | Hinge region | EPKSC |
| 51 | Hinge region | EPKSS |
| 52 | IgG1 mut Fc region (with hinge region) | EPKSSDKTHTCPPCPAPEAAGAPSVFLFPPKPKDTLMISR TPEVTCVVVDVSHEDPEVKFNWYVDGVEVHNAKTKPR EEQYNSTYRVVSVLTVLHQDWLNGKEYKCKVSNKALP APIEKTISKAKGQPREPQVYTLPPSREEMTKNQVSLTCL VKGFYPSDIAVEWESNGQPENNYKTTPPVLDSDGSFFL YSKLTVDKSRWQQGNVFSCSVMHEALHNHYTQKSLSL SPGK |

## Claims

1. An anti-GDF15 antibody or an antigen-binding fragment thereof, comprising
(i). three complementarity determining regions HCDR1, HCDR2, and HCDR3 contained in the VH set forth in SEQ ID NO: 7, and three complementarity determining regions LCDR1, LCDR2, and LCDR3 contained in the VL set forth in SEQ ID NO: 8;
(ii). three complementarity determining regions HCDR1, HCDR2, and HCDR3 contained in the VH set forth in SEQ ID NO: 14, and three complementarity determining regions LCDR1, LCDR2, and LCDR3 contained in the VL set forth in SEQ ID NO: 15;
(iii).three complementarity determining regions HCDR1, HCDR2, and HCDR3 contained in the VH set forth in SEQ ID NO: 23, and three complementarity determining regions LCDR1, LCDR2, and LCDR3 contained in the VL set forth in SEQ ID NO: 24; or
(iv). three complementarity determining regions HCDR1, HCDR2, and HCDR3 contained in the VH set forth in SEQ ID NO: 25, and three complementarity determining regions LCDR1, LCDR2, and LCDR3 contained in the VL set forth in SEQ ID NO: 26.

2. An anti-GDF15 antibody or an antigen-binding fragment thereof, comprising an HCDR1, an HCDR2, and an HCDR3, and an LCDR1, an LCDR2, and an LCDR3, wherein
(i). the HCDR1 comprises or consists of the amino acid sequence of SEQ ID NO: 1; the HCDR2 comprises or consists of the amino acid sequence of SEQ ID NO: 2; the HCDR3 comprises or consists of the amino acid sequence of SEQ ID NO: 3; the LCDR1 comprises or consists of the amino acid sequence of SEQ ID NO: 4; the LCDR2 comprises or consists of the amino acid sequence of SEQ ID NO: 5; and/or the LCDR3 comprises or consists of the amino acid sequence of SEQ ID NO: 6;
(ii). the HCDR1 comprises or consists of the amino acid sequence of SEQ ID NO: 1; the HCDR2 comprises or consists of the amino acid sequence of SEQ ID NO: 9; the HCDR3 comprises or consists of the amino acid sequence of SEQ ID NO: 10; the LCDR1 comprises or consists of the amino acid sequence of SEQ ID NO: 11; the LCDR2 comprises or consists of the amino acid sequence of SEQ ID NO: 12; and/or the LCDR3 comprises or consists of the amino acid sequence of SEQ ID NO: 13; or
(iii).the HCDR1 comprises or consists of the amino acid sequence of SEQ ID NO: 1; the HCDR2 comprises or consists of the amino acid sequence of SEQ ID NO: 20; the HCDR3 comprises or consists of the amino acid sequence of SEQ ID NO: 3; the LCDR1 comprises or consists of the amino acid sequence of SEQ ID NO: 21; the LCDR2 comprises or consists of the amino acid sequence of SEQ ID NO: 22; and/or the LCDR3 comprises or consists of the amino acid sequence of SEQ ID NO: 6.

3. The antibody or the antigen-binding fragment thereof according to claim 1 or 2, comprising a heavy chain variable region VH, wherein the heavy chain variable region
(i) comprises or consists of an amino acid sequence having at least 90%, 91%, 92%, 93%, 94%, 95%, 96%, 97%, 98%, or 99% identity to the amino acid sequence of SEQ ID NO: 7, 14, 23, or 25; or
(ii) comprises or consists of the amino acid sequence of SEQ ID NO: 7, 14, 23, or 25.

4. The antibody or the antigen-binding fragment thereof according to any one of claims 1-3, comprising a light chain variable region VL, wherein the light chain variable region
(i) comprises or consists of an amino acid sequence having at least 90%, 91%, 92%, 93%, 94%, 95%, 96%, 97%, 98%, or 99% identity to the amino acid sequence of SEQ ID NO: 8, 15, 24, or 26; or
(ii) comprises or consists of the amino acid sequence of SEQ ID NO: 8, 15, 24, or 26.

5. The antibody or the antigen-binding fragment thereof according to claim 1 or 2, comprising a heavy chain variable region VH and a light chain variable region VL, wherein
(i). the VH comprises or consists of the amino acid sequence set forth in SEQ ID NO: 7 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 8 or an amino acid sequence having at least 90%, 91%, 92%, 93%, 94%, 95%, 96%, 97%, 98%, or 99% identity thereto;
(ii). the VH comprises or consists of the amino acid sequence set forth in SEQ ID NO: 14 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 15 or an amino acid sequence having at least 90%, 91%, 92%, 93%, 94%, 95%, 96%, 97%, 98%, or 99% identity thereto;
(iii).the VH comprises or consists of the amino acid sequence set forth in SEQ ID NO: 23 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 24 or an amino acid sequence having at least 90%, 91%, 92%, 93%, 94%, 95%, 96%, 97%, 98%, or 99% identity thereto; or
(iv). the VH comprises or consists of the amino acid sequence set forth in SEQ ID NO: 25 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 26 or an amino acid sequence having at least 90%, 91%, 92%, 93%, 94%, 95%, 96%, 97%, 98%, or 99% identity thereto.

6. The antibody or the antigen-binding fragment thereof according to claim 1 or 2, comprising a heavy chain variable region VH and a light chain variable region VL, wherein
(i). the VH comprises or consists of the amino acid sequence set forth in SEQ ID NO: 7, and the VL comprises or consists of the amino acid sequence set forth in SEQ ID NO: 8;
(ii). the VH comprises or consists of the amino acid sequence set forth in SEQ ID NO: 14, and the VL comprises or consists of the amino acid sequence set forth in SEQ ID NO: 15;
(iii).the VH comprises or consists of the amino acid sequence set forth in SEQ ID NO: 23, and the VL comprises or consists of the amino acid sequence set forth in SEQ ID NO: 24; or
(iv). the VH comprises or consists of the amino acid sequence set forth in SEQ ID NO: 25, and the VL comprises or consists of the amino acid sequence set forth in SEQ ID NO: 26.

7. The antibody or the antigen-binding fragment thereof according to any one of claims 1-6, further comprising a heavy chain constant region HC, wherein, for example, the heavy chain constant region HC of the antibody is a heavy chain constant region of IgG1, IgG2, IgG3, or IgG4, preferably a heavy chain constant region of IgG1; optionally, the heavy chain constant region has an Fc region amino acid modification(s), e.g., a modification(s) that reduces or eliminates an ADCC effector function, a modification(s) that prolongs the half-life, a modification(s) that increases the affinity for FcRn, etc.; preferably, the heavy chain constant region has one or more of L234A/L235A mutations, a G237A mutation, and M252Y/S254T/T256E mutations; more preferably, the heavy chain constant region has L234A/L235A mutations, a G237A mutation, and M252Y/S254T/T256E mutations.

8. The antibody or the antigen-binding fragment thereof according to claim 7, wherein the heavy chain constant region
(i) comprises or consists of an amino acid sequence having at least 85%, 90%, 91%, 92%, 93%, 94%, 95%, 96%, 97%, 98%, or 99% identity to the amino acid sequence of SEQ ID NO: 44 or 48; or
(ii) comprises or consists of the amino acid sequence of SEQ ID NO: 44 or 48.

9. The antibody or the antigen-binding fragment thereof according to any one of claims 1-8, comprising a light chain constant region, wherein, for example, the light chain constant region is a lambda or kappa light chain constant region.

10. The antibody or the antigen-binding fragment thereof according to claim 9, wherein the light chain constant region
(i) comprises or consists of an amino acid sequence having at least 85%, 90%, 91%, 92%, 93%, 94%, 95%, 96%, 97%, 98%, or 99% identity to the amino acid sequence of SEQ ID NO: 45; or
(ii) comprises or consists of the amino acid sequence of SEQ ID NO: 45.

11. The antibody or the antigen-binding fragment thereof according to any one of claims 1-10, wherein the antibody comprises a heavy chain; the heavy chain
(i) comprises or consists of an amino acid sequence having at least 90%, 91%, 92%, 93%, 94%, 95%, 96%, 97%, 98%, or 99% identity to the amino acid sequence of SEQ ID NO: 28, 30, 33, 35, or 49; or
(ii) comprises or consists of the amino acid sequence of SEQ ID NO: 28, 30, 33, 35, or 49.

12. The antibody or the antigen-binding fragment thereof according to any one of claims 1-11, wherein the antibody comprises a light chain; the light chain
(i) comprises or consists of an amino acid sequence having at least 90%, 91%, 92%, 93%, 94%, 95%, 96%, 97%, 98%, or 99% identity to the amino acid sequence of SEQ ID NO: 29, 31, 34, or 36; or
(ii) comprises or consists of the amino acid sequence of SEQ ID NO: 29, 31, 34, or 36.

13. The antibody or the antigen-binding fragment thereof according to any one of claims 1-12, comprising a heavy chain and a light chain, wherein
(i). the heavy chain comprises or consists of the amino acid sequence set forth in SEQ ID NO: 28 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 29 or an amino acid sequence having at least 90%, 91%, 92%, 93%, 94%, 95%, 96%, 97%, 98%, or 99% identity thereto;
(ii). the heavy chain comprises or consists of the amino acid sequence set forth in SEQ ID NO: 30 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 31 or an amino acid sequence having at least 90%, 91%, 92%, 93%, 94%, 95%, 96%, 97%, 98%, or 99% identity thereto;
(iii).the heavy chain comprises or consists of the amino acid sequence set forth in SEQ ID NO: 33 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 34 or an amino acid sequence having at least 90%, 91%, 92%, 93%, 94%, 95%, 96%, 97%, 98%, or 99% identity thereto;
(iv). the heavy chain comprises or consists of the amino acid sequence set forth in SEQ ID NO: 35 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 36 or an amino acid sequence having at least 90%, 91%, 92%, 93%, 94%, 95%, 96%, 97%, 98%, or 99% identity thereto; or
(v). the heavy chain comprises or consists of the amino acid sequence set forth in SEQ ID NO: 49 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 34 or an amino acid sequence having at least 90%, 91%, 92%, 93%, 94%, 95%, 96%, 97%, 98%, or 99% identity thereto.

14. The antibody or the antigen-binding fragment thereof according to any one of claims 1-13, comprising a heavy chain and a light chain, wherein
(i). the heavy chain comprises or consists of the amino acid sequence set forth in SEQ ID NO: 28, and the light chain comprises or consists of the amino acid sequence set forth in SEQ ID NO: 29;
(ii). the heavy chain comprises or consists of the amino acid sequence set forth in SEQ ID NO: 30, and the light chain comprises or consists of the amino acid sequence set forth in SEQ ID NO: 31;
(iii).the heavy chain comprises or consists of the amino acid sequence set forth in SEQ ID NO: 33, and the light chain comprises or consists of the amino acid sequence set forth in SEQ ID NO: 34;
(iv). the heavy chain comprises or consists of the amino acid sequence set forth in SEQ ID NO: 35, and the light chain comprises or consists of the amino acid sequence set forth in SEQ ID NO: 36; or
(v). the heavy chain comprises or consists of the amino acid sequence set forth in SEQ ID NO: 49, and the light chain comprises or consists of the amino acid sequence set forth in SEQ ID NO: 34.

15. The antibody or the antigen-binding fragment thereof according to any one of claims 1-14, wherein the antibody is a chimeric antibody or a humanized antibody.

16. The antibody or the antigen-binding fragment thereof according to any one of claims 1-15, wherein the antibody is a monoclonal antibody.

17. The antibody or the antigen-binding fragment thereof according to any one of claims 1-16, wherein the antigen-binding fragment is an antibody fragment selected from: a Fab, a Fab', a Fab'-SH, an Fv, a single-chain antibody, a (Fab')₂, a single-domain antibody such as a heavy chain variable region VHH, a domain antibody (dAb), a heavy-chain antibody, and a linear antibody.

18. The antibody or the antigen-binding fragment thereof according to claim 17, wherein the single-chain antibody is an scFv.

19. A VHH antibody specifically binding to GDF15, comprising three complementarity determining regions (CDRs) contained in a VHH set forth in any one of SEQ ID NOs: 19 and 27, wherein preferably, the sequences of the CDRs are defined according to IMGT.

20. The VHH antibody according to claim 19, comprising complementarity determining regions (CDRs) VHH CDR1, VHH CDR2, and VHH CDR3, wherein
the VHH CDR1 comprises or consists of the amino acid sequence set forth in SEQ ID NO: 16, the VHH CDR2 comprises or consists of the amino acid sequence set forth in SEQ ID NO: 17, and the VHH CDR3 comprises or consists of the amino acid sequence set forth in SEQ ID NO: 18.

21. The VHH antibody according to claim 19, comprising or consisting of a heavy chain variable region, wherein the heavy chain variable region
(i) comprises or consists of an amino acid sequence having at least 90%, 91%, 92%, 93%, 94%, 95%, 96%, 97%, 98%, or 99% identity to an amino acid sequence selected from any one of SEQ ID NOs: 19 and 27; or
(ii) comprises or consists of an amino acid sequence selected from any one of SEQ ID NOs: 19 and 27.

22. A heavy-chain antibody specifically binding to GDF15, comprising the VHH antibody according to any one of claims 19-21.

23. The heavy-chain antibody according to claim 22, comprising the VHH antibody according to any one of claims 19-21 linked to an antibody constant region or Fc region, wherein preferably, the antibody constant region or Fc region is from human IgG1, human IgG2, human IgG3, or human IgG4, and preferably, the Fc region
(i) comprises an amino acid sequence having at least 85%, 90%, 91%, 92%, 93%, 94%, 95%, 96%, 97%, 98%, or 99% identity to the amino acid sequence set forth in SEQ ID NO: 46; or
(ii) comprises or consists of the amino acid sequence set forth in SEQ ID NO: 46.

24. The heavy-chain antibody according to claim 22, comprising the VHH antibody according to any one of claims 19-21 linked to an antibody Fc region, wherein the Fc region is an Fc region from human IgG1, IgG2, IgG3, or IgG4, optionally, the Fc region comprises L234A/L235A mutations and a G237A mutation, and preferably, the Fc region
(i) comprises an amino acid sequence having at least 85%, 90%, 91%, 92%, 93%, 94%, 95%, 96%, 97%, 98%, or 99% identity to the amino acid sequence set forth in SEQ ID NO: 43; or
(ii) comprises or consists of the amino acid sequence set forth in SEQ ID NO: 43.

25. The heavy-chain antibody according to claim 22, wherein the heavy-chain antibody
(i) comprises or consists of an amino acid sequence having at least 85%, 90%, 91%, 92%, 93%, 94%, 95%, 96%, 97%, 98%, or 99% identity to an amino acid sequence selected from SEQ ID NOs: 32 and 37; or
(ii) comprises or consists of an amino acid sequence selected from SEQ ID NOs: 32 and 37; or
(iii) comprises an amino acid sequence having one or more (preferably no more than 10, and more preferably no more than 5, 4, 3, 2, or 1) amino acid modifications (preferably amino acid substitutions, and more preferably conservative amino acid substitutions) compared to an amino acid sequence selected from SEQ ID NOs: 32 and 37, wherein preferably, the amino acid modifications do not occur in the CDRs.

26. The VHH antibody according to any one of claims 19-21 or the heavy-chain antibody according to any one of claims 22-25, wherein the antibody is a chimeric antibody or a humanized antibody.

27. A multispecific antibody, e.g., a bispecific antibody, comprising the antibody or the antigen-binding fragment thereof according to any one of claims 1-18, the VHH antibody according to any one of claims 19-21, or the heavy-chain antibody according to any one of claims 22-26.

28. A nucleic acid molecule, wherein the nucleic acid molecule encodes any one of the chains in the antibody or the antigen-binding fragment thereof according to any one of claims 1-18, the VHH antibody according to any one of claims 19-21, the heavy-chain antibody according to any one of claims 22-26, or the multispecific antibody according to claim 27, or consists of the nucleic acid sequence.

29. An expression vector, comprising the nucleic acid molecule according to claim 28, wherein preferably, the expression vector is pCDNA, e.g., pCDNA3.1.

30. A host cell, comprising the nucleic acid molecule according to claim 28 or the expression vector according to claim 29, wherein preferably, the host cell is prokaryotic or eukaryotic, e.g., a 293 cell or a CHO cell.

31. A method for preparing the antibody or the antigen-binding fragment thereof according to any one of claims 1-18, the VHH antibody according to any one of claims 19-21, the heavy-chain antibody according to any one of claims 22-26, or the multispecific antibody according to claim 27, comprising: culturing a host cell comprising the nucleic acid molecule according to claim 28 or the expression vector according to claim 29 or the host cell according to claim 30 under a condition suitable for expressing the chain(s) of the antibody, and optionally recovering the antibody from the host cell (or a host cell culture medium).

32. An antibody or an antigen-binding fragment thereof or a multispecific binding molecule prepared by the method according to claim 31.

33. An immunoconjugate, comprising the antibody or the antigen-binding fragment thereof according to any one of claims 1-18, the VHH antibody according to any one of claims 19-21, the heavy-chain antibody according to any one of claims 22-26, or the multispecific antibody according to claim 27, wherein, for example, the immunoconjugate is an ADC.

34. A pharmaceutical composition or a medicament or a formulation, comprising the antibody or the antigen-binding fragment thereof according to any one of claims 1-18, the VHH antibody according to any one of claims 19-21, the heavy-chain antibody according to any one of claims 22-26, the multispecific antibody according to claim 27, or the immunoconjugate according to claim 33, and optionally a pharmaceutical supplementary material.

35. A pharmaceutical combination product, comprising the antibody or the antigen-binding fragment thereof according to any one of claims 1-18, the VHH antibody according to any one of claims 19-21, the heavy-chain antibody according to any one of claims 22-26, the multispecific antibody according to claim 27, or the immunoconjugate according to claim 33, and one or more additional therapeutic agents (e.g., a chemotherapeutic agent, a cytokine, a cytotoxic agent, an additional antibody, a small molecule drug, or an immunomodulatory agent).

36. A method for preventing or treating cachexia in a subject, comprising administering to the individual an effective amount of the antibody or the antigen-binding fragment thereof according to any one of claims 1-18, the VHH antibody according to any one of claims 19-21, the heavy-chain antibody according to any one of claims 22-26, the multispecific antibody according to claim 27, the immunoconjugate according to claim 33, the pharmaceutical composition or the formulation according to claim 34, or the pharmaceutical combination product according to claim 35.

37. The method according to claim 36, wherein the cachexia occurs in association with a disease selected from a tumor such as a cancer, chronic heart failure, congestive heart failure, sarcopenia, chronic obstructive pulmonary disease (COPD), sarcopenia, and chronic kidney disease (CKD); for example, the tumor is a solid tumor or a hematological tumor, e.g., a malignant solid tumor or hematological tumor, such as fibrosarcoma, prostate cancer, pancreatic cancer, endometrial cancer, or colorectal cancer.

38. The method according to claim 36, wherein the cachexia is induced by chemotherapy or chemotherapy in combination with immuno-oncology therapy; for example, the chemotherapy comprises administering a chemotherapeutic agent, e.g., cisplatin.

39. The method according to any one of claims 36-38, wherein a sample from the individual has GDF15, for example, has or exhibits elevated levels of GDF15 protein and/or nucleic acids, e.g., compared to GDF15 protein and/or nucleic acid levels in the same tissue of a healthy individual; for example, the sample is a body fluid, such as blood, serum, or plasma.

40. The method according to any one of claims 36-39, further comprising administering in combination with an additional therapy such as a therapeutic modality (e.g., surgery or radiotherapy) and/or an additional therapeutic agent (e.g., a chemotherapeutic agent, a cytokine, a cytotoxic agent, an additional antibody, a small molecule drug, or an immunomodulatory agent (e.g., an immune checkpoint molecule inhibitor or agonist)).

41. A method for detecting the presence of GDF15 in a biological sample, comprising
(i) contacting the biological sample with the antibody or the antigen-binding fragment thereof according to any one of claims 1-18, the VHH antibody according to any one of claims 19-21, the heavy-chain antibody according to any one of claims 22-26, the multispecific antibody according to claim 27, or the immunoconjugate according to claim 33 under a condition that allows the binding thereof to GDF15, and
(ii) detecting whether a complex is formed by the antibody or the multispecific binding molecule and GDF15,
wherein the formation of the complex indicates the presence of GDF15.
